# EUROPÄISCHE PATENTSCHRIFT

(11) **EP 1 076 660 B1**
(45) Veröffentlichungstag und Bekanntmachung des Hinweises auf die Patenterteilung: **26.06.2002**
(21) Anmeldenummer: 99920734.3
(22) Anmeldetag: 22.04.1999
(51) Int. Cl.: C07D 417/04, C07D 413/04, A01N 43/78, A01N 43/76, C07D 231/16, C07F 9/6541, C07F 9/6558, C07D 417/14

(54) **SUBSTITUIERTE (4-BROMPYRAZOL-3-YL)BENZAZOLE**
SUBSTITUTED (4-BROMPYRAZOLE-3-YL)BENZAZOLES
(4-BROMPYRAZOL-3-YL)BENZAZOLES SUBSTITUES

(30) Priorität: 29.04.1998 DE 19819060
(43) Veröffentlichungstag der Anmeldung: 21.02.2001
(73) Patentinhaber: BASF AKTIENGESELLSCHAFT, 67056 Ludwigshafen (DE)
(72) Erfinder: ZAGAR, Cyrill, D-67061 Ludwigshafen (DE); HAMPRECHT, Gerhard, D-69469 Weinheim (DE); MENGES, Markus, D-64625 Bensheim (DE); MENKE, Olaf, D-67317 Altleiningen (DE); REINHARD, Robert, D-67061 Ludwigshafen (DE); SCHÄFER, Peter, D-67308 Ottersheim (DE); WESTPHALEN, Karl-Otto, D-67346 Speyer (DE); OTTEN, Martina, D-67069 Ludwigshafen (DE); WALTER, Helmut, D-67283 Obrigheim (DE)
(86) Internationale Anmeldenummer: EP9902699
(87) Internationale Veröffentlichungsnummer: WO9955702

(56) Entgegenhaltungen:
- WO-A-92/06962
- WO-A-96/15115
- WO-A-96/15116
- WO-A-98/27090

## Beschreibung

Die vorliegende Erfindung betrifft neue substituierte (4-Brompyrazol-3-yl)benzazole der Formel I 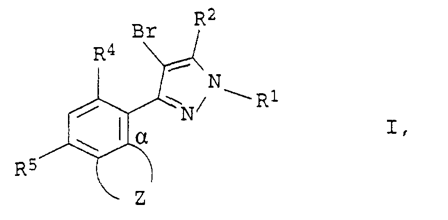 in der die Variablen folgende Bedeutungen haben:
- R¹: Wasserstoff, C₁-C₄-Alkyl oder C₁-C₄-Halogenalkyl;
- R²: Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
- R⁴: Wasserstoff oder Halogen;
- R⁵: Wasserstoff, Halogen, Cyano, C₁-C₄-Alkyl, C₁-C₄-Halogenalkyl, C₁-C₄-Alkoxy oder C₁-C₄-Halogenalkoxy;
- Z: eine Gruppe -N=C(XR⁶)-O- oder -N=C(XR⁶)-S-, die über den Stickstoff, Sauerstoff oder Schwefel an α gebunden sein kann;
- X: eine chemische Bindung, Sauerstoff, Schwefel, -S(O)-, -SO₂-, -NH- oder -N(R⁷)-;
- R⁶, R⁷: unabhängig voneinander
C₁-C₆-Alkyl, C₁-C₆-Halogenalkyl, Cyano-C₁-C₄-alkyl, Hydroxy-C₁-C₄-alkyl, C₃-C₆-Alkenyl, Cyano-C₃-C₆-alkenyl, C₃-C₆-Halogenalkenyl, C₃-C₆-Alkinyl, Cyano-C₃-C₆-alkinyl, C₃-C₆-Halogenalkinyl, C₁-C₄-Alkoxy-C₁-C₄-alkyl, C₁-C₄-Halogenalkoxy-C₁-C₄-alkyl, C₃-C₄-Alkenyloxy-C₁-C₄-alkyl, C₃-C₄-Alkinyloxy-C₁-C₄-alkyl, C₃-C₈-Cycloalkyloxy-C₁-C₄-alkyl, Amino-C₁-C₄-alkyl, C₁-C₄-Alkylamino-C₁-C₄-alkyl, Di(C₁-C₄-alkyl)amino-C₁-C₄-alkyl, C₁-C₄-Alkylthio-C₁-C₄-alkyl, C₁-C₄-Halogenalkylthio-C₁-C₄-alkyl, C₃-C₄-Alkenylthio-C₁-C₄-alkyl, C₃-C₄-Alkinylthio-C₁-C₄-alkyl, C₁-C₄-Alkylsulfinyl-C₁-C₄-alkyl, C₁-C₄-Halogenalkylsulfinyl-C₁-C₄-alkyl, C₃-C₄-Alkenylsulfinyl-C₁-C₄-alkyl, C₃-C₄-Alkinylsulfinyl-C₁-C₄-alkyl, C₁-C₄-Alkylsulfonyl-C₁-C₄-alkyl, C₁-C₄-Halogenalkylsulfonyl-C₁-C₄-alkyl, C₃-C₄-Alkenylsulfonyl-C₁-C₄-alkyl, C₃-C₄-Alkinylsulfonyl-C₁-C₄-alkyl, Hydroxycarbonyl-C₁-C₄-alkyl, (C₁-C₄-Alkoxy)carbonyl-C₁-C₄-alkyl, das eine Cyano- oder (C₁-C₄-Alkoxy)carbonylgruppe tragen kann,
(C₁-C₄-Alkylthio)carbonyl-C₁-C₄-alkyl, Aminocarbonyl-C₁-C₄-alkyl, C₁-C₄-Alkylaminocarbonyl-C₁-C₄-alkyl, Di(C₁-C₄-alkyl)-aminocarbonyl-C₁-C₄-alkyl, Di(C₁-C₄-alkyl)phosphonyl-C₁-C₄-alkyl, (C₁-C₄-Alkoxy)imino-C₁-C₄-alkyl, (C₃-C₄-Alkenyloxy)-imino-C₁-C₄-alkyl,
C₃-C₈-Cycloalkyl, C₃-C₈-Cycloalkyl-C₁-C₄-alkyl, Phenyl, Phenyl-C₁-C₄-alkyl, 3- bis 7-gliedriges Heterocyclyl oder Heterocyclyl-C₁-C₄-alkyl, wobei jeder Cycloalkyl- und jeder Heterocyclyl-Ring ein Carbonyl- oder Thiocarbonyl-Ringglied enthalten kann,
und wobei jeder Cycloalkyl-, Phenyl- und Heterocyclylring unsubstituiert sein oder ein bis vier Substituenten tragen kann, jeweils ausgewählt aus der Gruppe bestehend aus Cyano, Nitro, Amino, Hydroxy, Carboxy, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy und Di(C₁-C₄-alkyl)amino;
sofern X eine chemische Bindung, Sauerstoff, Schwefel, -NH- oder -N(R⁷)- ist, kann R⁶ auch (C₁-C₄-Alkyl)carbonyl, (C₁-C₄-Halogenalkyl)carbonyl, (C₁-C₄-Alkoxy)carbonyl, C₁-C₄-Alkylsulfonyl oder C₁-C₄-Halogenalkylsulfonyl bedeuten;
sofern X eine chemische Bindung ist, kann R⁶ außerdem Wasserstoff, Cyano, Mercapto, Amino, Halogen, -CH₂-CH(Halogen)-R⁸, -CH=CH-R⁸ oder -CH=C(Halogen)-R⁸ bedeuten, wobei
R⁸ für Hydroxycarbonyl, (C₁-C₄-Alkoxy)carbonyl, (C₁-C₄-Alkylthio)carbonyl, Aminocarbonyl, C₁-C₄-Alkylaminocarbonyl, Di(C₁-C₄-alkyl)aminocarbonyl oder Di(C₁-C₄-alkyl)phosphonyl steht,
oder R⁶ und R⁷ stehen zusammen für eine 1,3-Propylen-, Tetramethylen-, Pentamethylen- oder Ethylenoxyethylen-Kette, die jeweils unsubstituiert sein oder ein bis vier C₁-C₄-Alkylgruppen oder ein oder zwei (C₁-C₄-Alkoxy)carbonylgruppen tragen kann, sowie die landwirtschaftlich brauchbaren Salze dieser Verbindungen I.

Außerdem betrifft die Erfindung
- die Verwendung der Verbindungen I als Herbizide und/oder zur Desikkation/Defoliation von Pflanzen,
- herbizide Mittel und Mittel zur Desikkation und/oder Defoliation von Pflanzen, welche die Verbindungen I als wirksame Substanzen enthalten,
- Verfahren zur Herstellung der Verbindungen I und von herbiziden Mitteln und Mitteln zur Desikkation/Defoliation von Pflanzen unter Verwendung der Verbindungen I,
- Verfahren zur Bekämpfung von unerwünschtem Pflanzenwuchs und zur Desikkation/Defoliation von Pflanzen mit den Verbindungen I, sowie
- neue Zwischenprodukte der Formeln Va und Vb.

Die vorliegenden (4-Brompyrazol-3-yl)benzazole I fallen unter die allgemeine Formel von Wirkstoffen, die Gegenstand der älteren Anmeldung DE-A 19 652 240 sind.

Aufgabe der vorliegenden Erfindung war es, neue herbizid wirksame Pyrazol-Verbindungen bereitzustellen, mit denen sich unerwünschte Pflanzen besser als mit den bekannten gezielt bekämpfen lassen. Die Aufgabe erstreckte sich auch auf die Bereitstellung neuer desikkant/defoliant wirksamer Verbindungen.

Demgemäß wurden die vorliegenden substituierten (4-Brompyrazol-3-yl)benzazole der Formel I gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Desikkation/Defoliation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Bei Verbindungen I mit mindestens einem olefinischen Rest sind gegebenenfalls auch E-/Z-Isomere möglich. Gegenstand der Erfindung sind sowohl die reinen Enantiomeren oder Diastereomeren als auch deren Gemische.

Die bei der Definition der Substituenten R¹, R² und R⁵ bis R⁸ oder als Reste an Cycloalkyl-, Phenyl- oder heterocyclischen Ringen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Cyanoalkyl-, Oxyalkyl-, Aminoalkyl-, Oxycarbonylalkyl-, Aminocarbonylalkyl-, Phosphonylalkyl-, Oxyaminoalkyl-, Phenylalkyl-, Heterocyclylalkyl-, Alkenyl-, Halogenalkenyl-, Cyanoalkenyl-, Alkinyl-, Halogenalkinyl- und Cyanoalkinyl-Teile können geradkettig oder verzweigt sein. Halogenierte Substituenten tragen vorzugsweise ein bis fünf gleiche oder verschiedene Halogenatome. Die Bedeutung Halogen steht jeweils für Fluor, Chlor, Brom oder Iod.

Ferner stehen beispielsweise:
- C₁-C₄-Alkyl für: CH₃, C₂H₅, n-Propyl, CH(CH₃)₂, n-Butyl, 1-Methylpropyl, 2-Methylpropyl oder C(CH₃)₃;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂F, CHF₂, CF₃, CH₂Cl, CH(Cl)₂, C(Cl)₃, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, CH₂-C₂F₅, CF₂-C₂F₅, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Alkyl für: C₁-C₄-Alkyl wie vorstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise für CH₃, C₂H₅, CH₂-C₂H₅, CH(CH₃)₂, n-Butyl, C(CH₃)₃, n-Pentyl oder n-Hexyl;
- C₁-C₆-Halogenalkyl für: einen C₁-C₆-Alkylrest wie vorstehend genannt, der partiell oder vollständig durch Fluor, Chlor, Brom und/oder Iod substituiert ist, also z.B. einen der unter C₁-C₄-Halogenalkyl genannten Reste oder für 5-Fluor-1-pentyl, 5-Chlor-1-pentyl, 5-Brom-1-pentyl, 5-Iod-1-pentyl, 5,5,5-Trichlor-1-pentyl, n-C₅F₁₁, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl;
- Cyano-C₁-C₄-alkyl für: CH₂CN, 1-Cyanoethyl, 2-Cyanoethyl, 1-Cyanoprop-1-yl, 2-Cyanoprop-1-yl, 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 4-Cyanobut-2-yl, 1-(CH₂CN)eth-1-yl, 1-(CH₂CN)-1-(CH₃)-eth-1-yl oder 1-(CH₂CN)prop-1-yl;
- Hydroxy-C₁-C₄-alkyl für: CH₂OH, 1-Hydroxyethyl, 2-Hydroxyethyl, 1-Hydroxyprop-1-yl, 2-Hydroxyprop-1-yl, 3-Hydroxyprop-1-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 3-Hydroxybut-2-yl, 4-Hydroxybut-2-yl, 1-(CH₂OH)eth-1-yl, 1-(CH₂OH)-1-(CH₃)-eth-1-yl oder 1-(CH₂OH)prop-1-yl;
- Amino-C₁-C₄-alkyl für: CH₂NH₂, 1-Aminoethyl, 2-Aminoethyl, 1-Aminoprop-1-yl, 2-Aminoprop-1-yl, 3-Aminoprop-1-yl, 1-Aminobut-1-yl, 2-Aminobut-1-yl, 3-Aminobut-1-yl, 4-Aminobut-1-yl, 1-Aminobut-2-yl, 2-Aminobut-2-yl, 3-Aminobut-2-yl, 4-Aminobut-2-yl, 1-(CH₂NH₂)eth-1-yl, 1-(CH₂NH₂)-1-(CH₃)-eth-1-yl oder 1-(CH₂NH₂)prop-1-yl;
- Hydroxycarbonyl-C₁-C₄-alkyl für: CH₂COOH, 1-(COOH)ethyl, 2-(COOH)ethyl, 1-(COOH)prop-1-yl, 2-(COOH)prop-1-yl, 3-(COOH)-prop-1-yl, 1-(COOH)but-1-yl, 2-(COOH)but-1-yl, 3-(COOH)but-1-yl, 4-(COOH)but-1-yl, 1-(COOH)but-2-yl, 2-(COOH)but-2-yl, 3-(COOH)but-2-yl, 4-(COOH)but-2-yl, 1-(CH₂COOH)eth-1-yl, 1-(CH₂COOH)-1-(CH₃)-eth-1-yl oder 1-(CH₂COOH)prop-1-yl;
- Aminocarbonyl-C₁-C₄-alkyl für: CH₂CONH₂, 1-(CONH₂)ethyl, 2-(CONH₂)ethyl, 1-(CONH₂)prop-1-yl, 2-(CONH₂)prop-1-yl, 3-(CONH₂)prop-1-yl, 1-(CONH₂)but-1-yl, 2-(CONH₂)but-1-yl, 3-(CONH₂)but-1-yl, 4-(CONH₂)but-1-yl, 1-(CONH₂)but-2-yl, 2-(CONH₂)but-2-yl, 3- (CONH₂)but-2-yl, 4-(CONH₂)but-2-yl, 1-(CH₂CONH₂)eth-1-yl, 1-(CH₂CONH₂)-1-(CH₃)-eth-1-yl oder 1-(CH₂CONH₂)prop-1-yl;
- Phenyl-C₁-C₄-alkyl für: Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Benzyl)-eth-1-yl, 1-(Benzyl)-1-(methyl)-eth-1-yl oder 1-(Benzyl)-prop-1-yl, vorzugsweise für Benzyl oder 2-Phenylethyl;
- Heterocyclyl-C₁-C₄-alkyl für: Heterocyclylmethyl, 1-Heterocyclyl-ethyl, 2-Heterocyclyl-ethyl, 1-Heterocyclyl-prop-1-yl, 2-Heterocyclyl-prop-1-yl, 3-Heterocyclyl-prop-1-yl, 1-Heterocyclyl-but-1-yl, 2-Heterocyclyl-but-1-yl, 3-Heterocyclylbut-1-yl, 4-Heterocyclyl-but-1-yl, 1-Heterocyclyl-but-2-yl, 2-Heterocyclyl-but-2-yl, 3-Heterocyclyl-but-2-yl, 3-Heterocyclyl-but-2-yl, 4-Heterocyclyl-but-2-yl, 1-(Heterocyclylmethyl)-eth-1-yl, 1-(Heterocyclylmethyl)-1-(methyl)-eth-1-yl oder 1-(Heterocyclylmethyl)-prop-1-yl, vorzugsweise Heterocyclylmethyl oder 2-Heterocyclyl-ethyl;
- C₁-C₄-Alkoxy für: OCH₃, OC₂H₅, OCH₂-C₂H₅, OCH(CH₃)₂, n-Butoxy, OCH (CH₃)-C₂H₅, OCH₂-CH(CH₃)₂ oder C(CH₃)₃, vorzugsweise für OCH₃, OC₂H₅ oder OCH(CH₃)₂;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy, vorzugsweise für OCHF₂, OCF₃, Dichlorfluormethoxy, Chlordifluormethoxy oder 2,2,2-Trifluorethoxy;
- C₁-C₄-Alkylthio für: SCH₃, SC₂H₅, SCH₂-C₂H₅, SCH(CH₃)₂, n-Butylthio, 1-Methylpropylthio, SCH₂-CH(CH₃)₂ oder SC(CH₃)₃, vorzugsweise für SCH₃ oder SC₂H₅;
- C₁-C₄-Halogenalkylthio für: einen C₁-C₄-Alkylthiorest wie vorstehend genannt, der partiell oder vollständig durch Fluor, Chlor, Brom und/oder Iod substituiert ist, also z.B. SCH₂F, SCHF₂, SCF₃, SCH₂Cl, SCH(Cl)₂, SC(Cl)₃, Chlorfluormethylthio, Dichlorfluormethylthio, Chlordifluormethylthio, 2-Fluorethylthio, 2-Chlorethylthio, 2-Bromethylthio, 2-Iodethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, SC₂F₅, 2-Fluorpropylthio, 3-Fluorpropylthio, 2,2-Difluorpropylthio, 2,3-Difluorpropylthio, 2-Chlorpropylthio, 3-Chlorpropylthio, 2,3-Dichlorpropylthio, 2-Brompropylthio, 3-Brompropylthio, 3,3,3-Trifluorpropylthio, 3,3,3-Trichlorpropylthio, SCH₂-C₂F₅, SCF₂-C₂F₅, 1-(CH₂F)-2-fluorethylthio, 1-(CH₂Cl)-2-chlorethylthio, 1-(CH₂Br)-2-bromethylthio, 4-Fluorbutylthio, 4-Chlorbutylthio, 4-Brombutylthio oder SCF₂-CF₂-C₂F₅, vorzugsweise für SCHF₂, SCF₃, Dichlorfluormethylthio, Chlordifluormethylthio oder 2,2,2-Trifluorethylthio;
- C₁-C₄-Alkoxy-C₁-C₄-alkyl für: durch C₁-C₄-Alkoxy - wie vorstehend genannt - substituiertes C₁-C₄-Alkyl, also z.B. für CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)-propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise für CH₂-OCH₃, CH₂-OC₂H₅, 2-(OCH₃)ethyl oder 2-(OC₂H₅)ethyl;
- C₁-C₄-Halogenalkoxy-C₁-C₄-alkyl für: durch C₁-C₄-Halogenalkoxy wie vorstehend genannt substituiertes C₁-C₄-Alkyl, also z.B. für 2-(OCHF₂)ethyl, 2-(OCF₃)ethyl oder 2-(OC₂F₅)ethyl;
- C₁-C₄-Alkylthio-C₁-C₄-alkyl für: durch C₁-C₄-Alkylthio - wie vorstehend genannt - substituiertes C₁-C₄-Alkyl, also z.B. für CH₂-SCH₃, CH₂-SC₂H₅, n-Propylthiomethyl, CH₂-SCH(CH₃)₂, n-Butylthiomethyl, (1-Methylpropylthio)methyl, (2-Methylpropylthio)methyl, CH₂-SC(CH₃)₃, 2-(Methylthio)ethyl, 2-(Ethylthio)-ethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(Methylthio)propyl, 2-(Ethylthio)propyl, 2-(n-Propylthio)propyl, 2-(1-Methylethylthio)propyl, 2-(n-Butylthio)propyl, 2-(1-Methylpropylthio)propyl, 2-(2-Methylpropylthio)propyl, 2-(1,1-Dimethylethylthio)propyl, 3-(Methylthio)propyl, 3-(Ethylthio)propyl, 3-(n-Propylthio)propyl, 3-(1-Methylethylthio)propyl, 3-(n-Butylthio)propyl, 3-(1-Methylpropylthio)-propyl, 3-(2-Methylpropylthio)propyl, 3-(1,1-Dimethylethylthio)propyl, 2-(Methylthio)butyl, 2-(Ethylthio)butyl, 2-(n-Propylthio)butyl, 2-(1-Methylethylthio)butyl, 2-(n-Butylthio)butyl, 2-(1-Methylpropylthio)butyl, 2-(2-Methylpropylthio)butyl, 2-(1,1-Dimethylethylthio)butyl, 3-(Methylthio)butyl, 3-(Ethylthio)butyl, 3-(n-Propylthio)butyl, 3-(1-Methylethylthio)butyl, 3-(n-Butylthio)butyl, 3-(1-Methylpropylthio)butyl, 3-(2-Methylpropylthio)butyl, 3-(1,1-Dimethylethylthio)butyl, 4-(Methylthio)butyl, 4-(Ethylthio)butyl, 4-(n-Propylthio)butyl, 4-(1-Methylethylthio)butyl, 4-(n-Butylthio)-butyl, 4-(1-Methylpropylthio)butyl, 4-(2-Methylpropylthio)butyl oder 4-(1,1-Dimethylethylthio)butyl, vorzugsweise CH₂-SCH₃, CH₂-SC₂H₅, 2-(SCH₃)ethyl oder 2-(SC₂H₅)ethyl;
- C₁-C₄-Halogenalkylthio-C₁-C₄-alkyl für: durch C₁-C₄-Halogenalkylthio wie vorstehend genannt substituiertes C₁-C₄-Alkyl, also z.B. für 2-(SCHF₂)ethyl, 2-(SCF₃)ethyl oder 2-(SC₂F₅)ethyl;
- (C₁-C₄-Alkoxy)imino-C₁-C₄-alkyl für: durch (C₁-C₄-Alkoxy)imino wie =N-CH₃, =N-C₂H₅, =NCH₂-C₂H₅, =N-CH(CH₃)₂, =NCH₂-CH₂-C₂H₅, =NCH(CH₃)-C₂H₅, =NCH₂-CH(CH₃)₂ oder =N-C(CH₃)₃ substituiertes C₁-C₄-Alkyl, also z.B. für CH=N-CH₃, CH=N-C₂H₅, CH₂-CH=N-CH₃ oder CH₂-CH=N-C₂H₅;
- (C₁-C₄-Alkyl)carbonyl für: CO-CH₃, CO-C₂H₅, CO-CH₂-C₂H₅, CO-CH(CH₃)₂, n-Butylcarbonyl, CO-CH(CH₃)-C₂H₅, CO-CH₂-CH(CH₃)₂ oder CO-C(CH₃)₃, vorzugsweise für CO-CH₃ oder CO-C₂H₅;
- (C₁-C₄-Halogenalkyl)carbonyl für: einen (C₁-C₄-Alkyl)carbonylrest - wie vorstehend genannt - der partiell oder vollständig durch Fluor, Chlor, Brom und/oder Iod substituiert ist, also z.B. CO-CH₂F, CO-CHF₂, CO-CF₃, CO-CH₂Cl, CO-CH(Cl)₂, CO-C(Cl)₃, Chlorfluormethylcarbonyl, Dichlorfluormethylcarbonyl, Chlordifluormethylcarbonyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, CO-C₂F₅, 2-Fluorpropylcarbonyl, 3-Fluorpropylcarbonyl, 2,2-Difluorpropylcarbonyl, 2,3-Difluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, CO-CH₂-C₂F₅, CO-CF₂-C₂F₅, 1-(CH₂F)-2-fluorethylcarbonyl, 1-(CH₂Cl)-2-chlorethylcarbonyl, 1-(CH₂Br)-2-bromethylcarbonyl, 4-Fluorbutylcarbonyl, 4-Chlorbutylcarbonyl, 4-Brombutylcarbonyl oder Nonafluorbutylcarbonyl, vorzugsweise für CO-CF₃ CO-CH₂Cl oder 2,2,2-Trifluorethylcarbonyl;
- (C₁-C₄-Alkyl)carbonyloxy für: O-CO-CH₃, O-CO-C₂H₅, O-CO-CH₂-C₂H₅, O-CO-CH(CH₃)₂, O-CO-CH₂-CH₂-C₂H₅, O-CO-CH(CH₃)-C₂H₅, O-CO-CH₂-CH(CH₃)₂ oder O-CO-C(CH₃)₃, vorzugsweise für O-CO-CH₃ oder O-CO-C₂H₅;
- (C₁-C₄-Halogenalkyl)carbonyloxy für: einen (C₁-C₄-Alkyl)carbonylrest - wie vorstehend genannt - der partiell oder vollständig durch Fluor, Chlor, Brom und/oder Iod substituiert ist, also z.B. O-CO-CH₂F, O-CO-CHF₂, O-CO-CF₃, O-CO-CH₂Cl, O-CO-CH(Cl)₂, O-CO-C(Cl)₃, Chlorfluormethylcarbonyloxy, Dichlorfluormethylcarbonyloxy, Chlordifluormethylcarbonyloxy, 2-Fluorethylcarbonyloxy, 2-Chlorethylcarbonyloxy, 2-Bromethylcarbonyloxy, 2-Iodethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy, 2,2,2-Trichlorethylcarbonyloxy, O-CO-C₂F₅, 2-Fluorpropylcarbonyloxy, 3-Fluorpropylcarbonyloxy, 2,2-Difluorpropylcarbonyloxy, 2,3-Difluorpropylcarbonyloxy, 2-Chlorpropylcarbonyloxy, 3-Chlorpropylcarbonyloxy, 2,3-Dichlorpropylcarbonyloxy, 2-Brompropylcarbonyloxy, 3-Brompropylcarbonyloxy, 3,3,3-Trifluorpropylcarbonyloxy, 3,3,3-Trichlorpropylcarbonyloxy, O-CO-CH₂-C₂F₅, O-CO-CF₂-C₂F₅, 1-(CH₂F)-2-fluorethylcarbonyloxy, 1-(CH₂Cl)-2-chlorethylcarbonyloxy, 1-(CH₂Br)-2-bromethylcarbonyloxy, 4-Fluorbutylcarbonyloxy, 4-Chlorbutylcarbonyloxy, 4-Brombutylcarbonyloxy oder Nonafluorbutylcarbonyloxy, vorzugsweise für O-CO-CF₃, O-CO-CH₂Cl oder 2,2,2-Trifluorethylcarbonyloxy;
- (C₁-C₄-Alkoxy)carbonyl für: CO-OCH₃, CO-OC₂H₅, n-Propoxycarbonyl, CO-OCH(CH₃)₂, n-Butoxycarbonyl, CO-OCH(CH₃)-C₂H₅, CO-OCH₂-CH(CH₃)₂ oder CO-OC(CH₃)₃, vorzugsweise für CO-OCH₃ oder CO-OC₂H₅;
- (C₁-C₄-Alkoxy)carbonyl-C₁-C₄-alkyl für: durch (C₁-C₄-Alkoxy)-carbonyl - wie vorstehend genannt - substituiertes C₁-C₄-Alkyl, also z.B. für CH₂-CO-OCH₃, CH₂-CO-OC₂H₅, n-Propoxycarbonylmethyl, CH₂-CO-OCH(CH₃)₂, n-Butoxycarbonylmethyl, CH₂-CO-OCH(CH₃)-C₂H₅, CH₂-CO-OCH₂-CH(CH₃)₂, CH₂-CO-OC(CH₃)₃, 1-(Methoxycarbonyl)ethyl, 1-(Ethoxycarbonyl)ethyl, 1-(n-Propoxycarbonyl)ethyl, 1-(1-Methylethoxycarbonyl)ethyl, 1-(n-Butoxycarbonyl)ethyl, 2-(Methoxycarbonyl)ethyl, 2-(Ethoxycarbonyl)ethyl, 2-(n-Propoxycarbonyl)ethyl, 2-(1-Methylethoxycarbonyl)ethyl, 2-(n-Butoxycarbonyl)ethyl, 2-(1-Methylpropoxycarbonyl)ethyl, 2-(2-Methylpropoxycarbonyl)ethyl, 2-(1,1-Dimethylethoxycarbonyl)ethyl, 2-(Methoxycarbonyl)propyl, 2-(Ethoxycarbonyl)propyl, 2-(n-Propoxycarbonyl)propyl, 2-(1-Methylethoxycarbonyl)propyl, 2-(n-Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3-(Methoxycarbonyl)-propyl, 3-(Ethoxycarbonyl)propyl, 3-(n-Propoxycarbonyl)propyl, 3-(1-Methylethoxycarbonyl)propyl, 3-(n-Butoxycarbonyl)propyl, 3-(1-Methylpropoxycarbonyl)propyl, 3-(2-Methylpropoxycarbonyl)propyl, 3-(1,1-Dimethylethoxycarbonyl)propyl, 2-(Methoxycarbonyl)butyl, 2-(Ethoxycarbonyl)butyl, 2-(n-Propoxycarbonyl)butyl, 2-(1-Methylethoxycarbonyl)butyl, 2-(n-Butoxycarbonyl)butyl, 2-(1-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(n-Propoxycarbonyl)butyl, 3-(1-Methylethoxycarbonyl)butyl, 3-(n-Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)butyl, 4-(Ethoxycarbonyl)-butyl, 4-(n-Propoxycarbonyl)butyl, 4-(1-Methylethoxycarbonyl)-butyl, 4-(n-Butoxycarbonyl)butyl, 4-(1-Methylpropoxycarbonyl)-butyl, 4-(2-Methylpropoxycarbonyl)butyl oder 4-(1,1-Dimethylethoxycarbonyl)butyl, vorzugsweise für CH₂-CO-OCH₃, CH₂-CO-OC₂H₅, 1-(Methoxycarbonyl)ethyl oder 1-(Ethoxycarbonyl) ethyl;
- (C₁-C₄-Alkylthio)carbonyl für: CO-SCH₃, CO-SC₂H₅, CO-SCH₂-C₂H₅, CO-SCH(CH₃)₂, CO-SCH₂CH₂-C₂H₅, CO-SCH(CH₃)-C₂H₅, CO-SCH₂-CH(CH₃)₂ oder CO-SC(CH₃)₃, vorzugsweise für CO-SCH₃ oder CO-SC₂H₅;
- (C₁-C₄-Alkylthio)carbonyl-C₁-C₄-alkyl für: durch (C₁-C₄-Alkylthio)carbonyl - wie vorstehend genannt - substituiertes C₁-C₄-Alkyl, also z.B. für CH₂-CO-SCH₃, CH₂-CO-SC₂H₅, CH₂-CO-SCH₂-C₂H₅, CH₂-CO-SCH(CH₃)₂, CH₂-CO-SCH₂CH₂-C₂H₅, CH₂-CO-SCH(CH₃)-C₂H₅, CH₂-CO-SCH₂ -CH(CH₃)₂, CH₂-CO-SC (CH₃)₃, 1-(CO-SCH₃)ethyl, 1-(CO-SC₂H₅) ethyl, 1-(CO-SCH₂-C₂H₅)ethyl, 1- [CO-SCH(CH₃)₂]ethyl, 1-(CO-SCH₂CH₂-C₂H₅)ethyl, 1-[CO-SCH(CH₃)-C₂H₅]ethyl, 1-[CO-SCH₂-CH(CH₃)₂]ethyl, 1-[CO-SC(CH₃)₃]ethyl, 2-(CO-SCH₃)ethyl, 2-(CO-SC₂H₅)ethyl, 2- (CO-SCH₂-C₂H₅)ethyl, 2-[CO-SCHCH₃)₂]ethyl, 2- (CO-SCH₂CH₂-C₂H₅)ethyl, 2- [CO-SCH(CH₃)-C₂H₅]ethyl, 2-[CO-SCH₂-CH(CH₃)₂]ethyl, 2-[CO-SC(CH₃)₃]ethyl, 2-(CO-SCH₃)-propyl, 2-(CO-SC₂H₅)propyl, 2-(CO-SCH₂-C₂H₅)propyl, 2-[CO-SCH(CH₃)₂]propyl, 2- (CO-SCH₂CH₂-C₂H₅)propyl, 2- [CO-SCH(CH₃)-C₂H₅]propyl, 2-[CO-SCH₂-CH(CH₃)₂]propyl, 2-[CO-SC(CH₃)₃]propyl, 3-(CO-SCH₃)propyl, 3-(CO-SC₂H₅)propyl, 3-(CO-SCH₂-C₂H₅)propyl, 3-[CO-SCH(CH₃)₂]propyl, 3- (CO-SCH₂CH₂-C₂H₅)propyl, 3- [CO-SCH(CH₃)-C₂H₅]propyl, 3-[CO-SCH₂-CH(CH₃)₂]propyl, 3-[CO-SC(CH₃)₃]propyl, 2-(CO-SCH₃)butyl, 2- (CO-SC₂H₅)butyl, 2-(CO-SCH₂-C₂H₅)butyl, 2- [CO-SCH(CH₃)₂]butyl, 2-(CO-SCH₂CH₂-C₂H₅)butyl, 2- [CO-SCH(CH₃)-C₂H₅]butyl, 2- [CO-SCH₂-CH(CH₃)₂]butyl, 2-[CO-SC(CH₃)₃]butyl, 3-(CO-SCH₃)butyl, 3-(CO-SC₂H₅)butyl, 3-(CO-SCH₂-C₂H₅)butyl, 3-[CO-SCH(CH₃)₂]butyl, 3-(CO-SCH₂CH₂-C₂H₅)butyl, 3-[CO-SCH(CH₃)-C₂H₅] butyl, 3-[CO-SCH₂-CH(CH₃)₂]butyl, 3-[CO-SC(CH₃)₃]butyl, 4-(CO-SCH₃)-butyl, 4-(CO-SC₂H₅)butyl, 4-(CO-SCH₂-C₂H₅)butyl, 4-[CO-SCH(CH₃)₂]butyl, 4-(CO-SCH₂CH₂-C₂H₅)butyl, 4- [CO-SCH(CH₃)-C₂H₅]butyl, 4-[CO-SCH₂-CH(CH₃)₂]butyl oder 4-[CO-SC(CH₃)₃]butyl, vorzugsweise für CH₂-CO-SCH₃, CH₂-CO-SC₂H₅, 1-(CO-SCH₃)ethyl oder 1-(CO-SC₂H₅)ethyl;
- C₁-C₄-Alkylsulfinyl für: SO-CH₃, SO-C₂H₅, SO-CH₂-C₂H₅, SO-CH(CH₃)₂, n-Butylsulfinyl, SO-CH(CH₃)-C₂H₅, SO-CH₂-CH(CH₃)₂ oder SO-C(CH₃)₃, vorzugsweise für SO-CH₃ oder SO-C₂H₅;
- C₁-C₄-Halogenalkylsulfinyl für: einen C₁-C₄-Alkylsulfinylrest - wie vorstehend genannt - der partiell oder vollständig durch Fluor, Chlor, Brom und/oder Iod substituiert ist, also z.B. SO-CH₂F, SO-CHF₂, SO-CF₃, SO-CH₂Cl, SO-CH(Cl)₂, SO-C(Cl)₃, Chlorfluormethylsulfinyl, Dichlorfluormethylsulfinyl, Chlordifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2-Chlor-2-fluorethylsulfinyl, 2-Chlor-2,2-difluorethylsulfinyl, 2,2-Dichlor-2-fluorethylsulfinyl, 2,2,2-Trichlorethylsulfinyl, SO-C₂F₅, 2-Fluorpropylsulfinyl, -Fluorpropylsulfinyl, 2,2-Difluorpropylsulfinyl, 2,3-Difluorpropylsulfinyl, 2-Chlorpropylsulfinyl, 3-Chlorpropylsulfinyl, 2,3-Dichlorpropylsulfinyl, 2-Brompropylsulfinyl, 3-Brompropylsulfinyl, 3,3,3-Trifluorpropylsulfinyl, 3,3,3-Trichlorpropylsulfinyl, SO-CH₂-C₂F₅, SO-CF₂-C₂F₅, 1-(CH₂F)-2-fluorethylsulfinyl, 1-(CH₂Cl)-2-chlorethylsulfinyl, 1-(CH₂Br)-2-bromethylsulfinyl, 4-Fluorbutylsulfinyl, 4-Chlorbutylsulfinyl, 4-Brombutylsulfinyl oder Nonafluorbutylsulfinyl, vorzugsweise für SO-CF₃, SO-CH₂Cl oder 2,2,2-Trifluorethylsulfinyl;
- C₁-C₄-Alkylsulfinyl-C₁-C₄-alkyl für: durch C₁-C₄-Alkylsulfinyl wie vorstehend genannt substituiertes C₁-C₄-Alkyl, also z.B. für CH₂SOCH₃, CH₂SOC₂H₅, n-Propylsulfinylmethyl, CH₂SOCH(CH₃)₂, n-Butylsulfinylmethyl, (1-Methylpropylsulfinyl)methyl, (2-Methylpropylsulfinyl)methyl, (1,1-Dimethylethylsulfinyl)-methyl, 2-Methylsulfinylethyl, 2-Ethylsulfinylethyl, 2-(n-Propylsulfinyl)ethyl, 2-(1-Methylethylsulfinyl)ethyl, 2-(n-Butylsulfinyl)ethyl, 2-(1-Methylpropylsulfinyl)ethyl, 2-(2-Methylpropylsulfinyl)ethyl, 2-(1,1-Dimethylethylsulfinyl)ethyl, 2-(SOCH₃)propyl, 3-(SOCH₃)propyl, 2-(SOC₂H₅)-propyl, 3-(SOC₂H₅)propyl, 3-(Propylsulfinyl)propyl, 3-(Butylsulfinyl)propyl, 4-(SOCH₃)butyl, 4-(SOC₂H₅)butyl, 4-(n-Propylsulfinyl)butyl oder 4-(n-Butylsulfinyl)butyl, insbesondere für 2-(SOCH₃)ethyl;
- C₁-C₄-Halogenalkylsulfinyl-C₁-C₄-alkyl für: durch C₁-C₄-Halogenalkylsulfinyl wie vorstehend genannt substituiertes C₁-C₄-Alkyl, also z.B. für 2-(2,2,2-Trifluorethylsulfinyl)-ethyl;
- C₁-C₄-Alkylsulfonyl für: SO₂-CH₃, SO₂-C₂H₅, SO₂-CH₂-C₂H₅, SO₂-CH(CH₃)₂, n-Butylsulfonyl, SO₂-CH(CH₃)-C₂H₅, SO₂-CH₂-CH(CH₃)₂ oder SO₂-C(CH₃)₃, vorzugsweise für SO₂-CH₃ oder SO₂-C₂H₅;
- C₁-C₄-Halogenalkylsulfonyl für: einen C₁-C₄-Alkylsulfonylrestwie vorstehend genannt - der partiell oder vollständig durch Fluor, Chlor, Brom und/oder Iod substituiert ist, also z.B. SO₂-CH₂F, SO₂-CHF₂, SO₂-CF₃, SO₂-CH₂Cl, SO₂-CH(Cl)₂, SO₂-C(Cl)₃, Chlorfluormethylsulfonyl, Dichlorfluormethylsulfonyl, Chlordifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, SO₂-C₂F₅, 2-Fluorpropylsulfonyl, 3-Fluorpropylsulfonyl, 2,2-Difluorpropylsulfonyl, 2,3-Difluorpropylsulfonyl, 2-Chlorpropylsulfonyl, 3-Chlorpropylsulfonyl, 2,3-Dichlorpropylsulfonyl, 2-Brompropylsulfonyl, 3-Brompropylsulfonyl, 3,3,3-Trifluorpropylsulfonyl, 3,3,3-Trichlorpropylsulfonyl, SO₂-CH₂-C₂F₅, SO₂-CF₂-C₂F₅, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl, vorzugsweise für SO₂-CH₂Cl, SO₂-CF₃ oder 2,2,2-Trifluorethylsulfonyl;
- C₁-C₄-Alkylsulfonyl-C₁-C₄-alkyl für: durch C₁-C₄-Alkylsulfonyl wie vorstehend genannt substituiertes C₁-C₄-Alkyl, also z.B. für CH₂SO₂-CH₃, CH₂SO₂-C₂H₅, CH₂SO₂-CH₂-C₂H₅, CH₂SO₂-CH(CH₃)₂, CH₂SO₂-CH₂CH₂-C₂H₅, (1-Methylpropylsulfonyl)methyl, (2-Methylpropylsulfonyl)methyl, CH₂SO₂-C(CH₃)₃, CH(CH₃)SO₂-CH₃, CH(CH₃)SO₂-C₂H₅, CH₂CH₂SO₂-CH₃, CH₂CH₂SO₂-C₂H₅, CH₂CH₂SO₂-CH₂-C₂H₅, CH₂CH₂SO₂-CH(CH₃)₂, CH₂CH₂SO₂-CH₂CH₂-C₂H₅, 2-(1-Methylpropylsulfonyl) ethyl, 2- (2-Methylpropylsulfonyl) ethyl, CH₂CH₂SO₂-C(CH₃)₃, 2-(SO₂-CH₃)propyl, 2-(SO₂-C₂H₅)propyl, 2-(SO₂-CH₂-C₂H₅)propyl, 2-[SO₂-CH(CH₃)₂]propyl, 2-(SO₂-CH₂CH₂-C₂H₅)propyl, 2-(1-Methylpropylsulfonyl)propyl, 2-(2-Methylpropylsulfonyl)propyl, 2-[SO₂-C(CH₃)₃]propyl, 3-(SO₂-CH₃)propyl, 3-(SO₂-C₂H₅)propyl, 3- (SO₂-CH₂-C₂H₅)propyl, 3-[SO₂-CH(CH₃)₂] propyl, 3-(SO₂-CH₂CH₂-C₂H₅)propyl, 3-(1-Methylpropylsulfonyl)propyl, 3-(2-Methylpropylsulfonyl)propyl, 3- [SO₂-C(CH₃)₃]propyl, 2-(SO₂-CH₃)butyl, 2-(SO₂-C₂H₅)butyl, 2-(SO₂-CH₂-C₂H₅)butyl, 2-[SO₂-CH(CH₃)₂]butyl, 2-(SO₂-CH₂CH₂-C₂H₅)butyl, 2-(1-Methylpropylsulfonyl)butyl, 2- (2-Methylpropylsulfonyl)butyl, 2-[SO₂-C(CH₃)₃]butyl, 3-(SO₂-CH₃)butyl, 3-(SO₂-C₂H₅)butyl, 3-(SO₂-CH₂-C₂H₅)butyl, 3-[SO₂-CH(CH₃)₂]butyl, 3-(SO₂-CH₂CH₂-C₂H₅)butyl, 3-(1-Methylpropylsulfonyl)butyl, 3-(2-Methylpropylsulfonyl)butyl, 3-[SO₂-C(CH₃)₃]butyl, 4-(SO₂-CH₃)butyl, 4-(SO₂-C₂H₅)butyl, 4-(SO₂-CH₂-C₂H₅)butyl, 4-[SO₂-CH(CH₃)₂]butyl, 4-(SO₂-CH₂CH₂-C₂H₅)butyl, 4-(1-Methylpropylsulfonyl)butyl, 4-(2-Methylpropylsulfonyl)butyl oder 4-[SO₂-C(CH₃)₃]butyl, insbesondere für CH₂CH₂SO₂-CH₃ oder CH₂CH₂SO₂-C₂H₅;
- C₁-C₄-Halogenalkylsulfonyl-C₁-C₄-alkyl für: durch C₁-C₄-Halogenalkylsulfonyl wie vorstehend genannt substituiertes C₁-C₄-Alkyl, also z.B. für 2-(2,2,2-Trifluorethylsulfonyl)-ethyl;
- C₁-C₄-Alkylamino-C₁-C₄-alkyl für: durch C₁-C₄-Alkylamino wie -NH-CH₃, -NH-C₂H₅, -NH-CH₂-C₂H₅, -NH-CH(CH₃)₂, -NH-CH₂CH₂-C₂H₅, -NH-CH(CH₃)C₂H₅, -NH-CH₂-CH(CH₃)₂ und -NH-C(CH₃)₃, vorzugsweise -NH-CH₃ oder -NH-C₂H₅, substituiertes C₁-C₄-Alkyl, also beispielsweise für CH₂CH₂-NH-CH₃, CH₂CH₂-N(CH₃)₂, CH₂CH₂-NH-C₂H₅ oder CH₂CH₂-N(C₂H₅)₂;
- C₁-C₄-Alkylaminocarbonyl für: CO-NH-CH₃, CO-NH-C₂H₅, n-Propylamino, CO-NH-CH(CH₃)₂, CO-NH-CH₂CH₂-C₂H₅, CO-NH-CH(CH₃)-C₂H₅, CO-NH-CH₂-CH(CH₃)₂ oder CO-NH-C(CH₃)₃, vorzugsweise für CO-NH-CH₃ oder CO-NH-C₂H₅;
- C₁-C₄-Alkylaminocarbonyl-C₁-C₄-alkyl für: C₁-C₄-Alkylaminocarbonyl wie vorstehend genannt, vorzugsweise CO-NH-CH₃ oder CO-NH-C₂H₅, substituiertes C₁-C₄-Alkyl, als z.B. für CH₂-CO-NH-CH₃, CH₂-CO-NH-C₂H₅, CH₂-CO-NH-CH₂-C₂H₅, CH₂-CO-NH-CH(CH₃)₂, CH₂-CO-NH-CH₂CH₂-C₂H₅, CH₂-CO-NH-CH(CH₃)-C₂H₅, CH₂-CO-NH-CH₂-CH(CH₃)₂, CH₂-CO-NH-C(CH₃)₃, CH(CH₃)-CO-NH-CH₃, CH(CH₃)-CO-NH-C₂H₅, 2-(CO-NH-CH₃)ethyl, 2-(CO-NH-C₂H₅)ethyl, 2-(CO-NH-CH₂-C₂H₅)-ethyl, 2-[CH₂-CO-NH-CH(CH₃)₂] ethyl, 2-(CO-NH-CH₂CH₂-C₂H₅) ethyl, 2- [CO-NH-CH (CH₃)-C₂H₅] ethyl, 2-[CO-NH-CH₂-CH(CH₃)₂] ethyl, 2-[CO-NH-C(CH₃)₃] ethyl, 2-(CO-NH-CH₃)propyl, 2-(CO-NH-C₂H₅)propyl, 2-(CO-NH-CH₂-C₂H₅)propyl, 2- [CH₂-CO-NH-CH(CH₃)₂] propyl, 2- (CO-NH-CH₂CH₂-C₂H₅)propyl, 2-[CO-NH-CH(CH₃) -C₂H₅] propyl, 2-[CO-NH-CH₂-CH(CH₃)₂] propyl, 2-[CO-NH-C(CH₃)₃] propyl, 3-(CO-NH-CH₃) propyl, 3-(CO-NH-C₂H₅)propyl, 3-(CO-NH-CH₂-C₂H₅)propyl, 3- [CH₂-CO-NH-CH(CH₃)₂] propyl, 3- (CO-NH-CH₂CH₂-C₂H₅)propyl, 3-[CO-NH-CH(CH₃)-C₂H₅] propyl, 3- [CO-NH-CH₂-CH(CH₃)₂] propyl, 3-[CO-NH-C(CH₃)₃]propyl, 2-(CO-NH-CH₃)butyl, 2-(CO-NH-C₂H₅)-butyl, 2-(CO-NH-CH₂-C₂H₅)butyl, 2-[CH₂-CO-NH-CH(CH₃)₂]butyl, 2- (CO-NH-CH₂CH₂-C₂H₅)butyl, 2-[CO-NH-CH(CH₃)-C₂H₅]butyl, 2- [CO-NH-CH₂-CH(CH₃)₂]butyl, 2- [CO-NH-C (CH₃)₃]butyl, 3-(CO-NH-CH₃)butyl, 3-(CO-NH-C₂H₅)butyl, 3-(CO-NH-CH₂-C₂H₅)-butyl, 3-[CH₂-CO-NH-CH(CH₃)₂]butyl, 3- (CO-NH-CH₂CH₂-C₂H₅)butyl, 3-[CO-NH-CH(CH₃)-C₂H₅]butyl, 3-[CO-NH-CH₂-CH(CH₃)₂]butyl, 3-[CO-NH-C(CH₃)₃]butyl, 4-(CO-NH-CH₃)butyl, 4-(CO-NH-C₂H₅)butyl, 4- (CO-NH-CH₂-C₂H₅) butyl, 4- [CH₂-CO-NH-CH(CH₃)₂]butyl, 4-(CO-NH-CH₂CH₂-C₂H₅) butyl, 4- [CO-NH-CH(CH₃)-C₂H₅] butyl, 4-[CO-NH-CH₂-CH(CH₃)₂]butyl oder 4-[CO-NH-C(CH₃)₃]butyl, vorzugsweise für CH₂-CO-NH-CH₃, CH₂-CO-NH-C₂H₅, CH(CH₃)-CO-NH-CH₃ oder CH(CH₃)-CO-NH-C₂H₅;
- Di(C₁-C₄-alkyl)amino für: N(CH₃)₂, N(C₂H₅)₂, N,N-Dipropylamino, N[CH(CH₃)₂]₂, N(n-C₄H₉)₂, N,N-Di-(1-methylpropyl)amino, N,N-Di-(2-methylpropyl)amino, N[C(CH₃)₃]₂, N-Ethyl-N-methylamino, N-Methyl-N-propylamino, N-Methyl-N-(1-methylethyl)amino, N-Butyl-N-methylamino, N-Methyl-N-(1-methylpropyl) amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)-amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino, vorzugsweise für N(CH₃)₂ oder N(C₂H₅)₂;
- Di(C₁-C₄-alkyl)amino-C₁-C₄-alkyl für: durch Di(C₁-C₄-alkyl)-amino wie vorstehend genannt substituiertes C₁-C₄-Alkyl, also z.B. für CH₂N(CH₃)₂, CH₂N(C₂H₅)₂, N,N-Dipropylaminomethyl, N,N-Di[CH(CH₃)₂]aminomethyl, N,N-Dibutylaminomethyl, N,N-Di-(1-methylpropyl)aminomethyl, N,N-Di(2-methylpropyl)aminomethyl, N,N-Di[C(CH₃)₃]aminomethyl, N-Ethyl-N-methylaminomethyl, N-Methyl-N-propylaminomethyl, N-Methyl-N-[CH(CH₃)₂]aminomethyl, N-Butyl-N-methylaminomethyl, N-Methyl-N-(1-methylpropyl)aminomethyl, N-Methyl-N-(2-methylpropyl)aminomethyl, N-[C(CH₃)₃]-N-methylaminomethyl, N-Ethyl-N-propylaminomethyl, N-Ethyl-N-[CH(CH₃)₂]aminomethyl, N-Butyl-N-ethylaminomethyl, N-Ethyl-N-(1-methylpropyl)aminomethyl, N-Ethyl-N-(2-methylpropyl)aminomethyl, N-Ethyl-N-[C(CH₃)₃]aminomethyl, N-[CH(CH₃)₂]-N-propylaminomethyl, N-Butyl-N-propylaminomethyl, N-(1-Methylpropyl)-N-propylaminomethyl, N-(2-Methylpropyl)-N-propylaminomethyl, N-[C(CH₃)₃]-N-propylaminomethyl, N-Butyl-N-(1-methylethyl)-aminomethyl, N-[CH(CH₃)₂]-N- (1-methylpropyl) aminomethyl, N- [CH(CH₃)₂]-N-(2-methylpropyl)aminomethyl, N- [C (CH₃)₃]-N-[CH(CH₃)₂]aminomethyl, N-Butyl-N-(1-methylpropyl)aminomethyl, N-Butyl-N-(2-methylpropyl)aminomethyl, N-Butyl-N-[C(CH₃)₃]-aminomethyl, N-(1-Methylpropyl)-N-(2-methylpropyl)aminomethyl, N-[C(CH₃)₃]-N-(1-methylpropyl)aminomethyl, N-[C(CH₃)₃]-N-(2-methylpropyl)aminomethyl, N,N-Dimethylaminoethyl, N,N-Diethylaminoethyl, N,N-Di(n-propyl)aminoethyl, N,N-Di-[CH(CH₃)₂]-aminoethyl, N,N-Dibutylaminoethyl, N,N-Di(1-methylpropyl)aminoethyl, N,N-Di(2-methylpropyl)aminoethyl, N,N-Di-[C(CH₃)₃]-aminoethyl, N-Ethyl-N-methylaminoethyl, N-Methyl-N-propylaminoethyl, N-Methyl-N-[CH(CH₃)₂]aminoethyl, N-Butyl-N-methylaminoethyl, N-Methyl-N-(1-methylpropyl)aminoethyl, N-Methyl-N-(2-methylpropyl) aminoethyl, N-[C(CH₃)₃]-N-methylaminoethyl, N-Ethyl-N-propylaminoethyl, N-Ethyl-N-[CH(CH₃)₂]aminoethyl, N-Butyl-N-ethylaminoethyl, N-Ethyl-N-(1-methylpropyl)aminoethyl, N-Ethyl-N-(2-methylpropyl)aminoethyl, N-Ethyl-N-[C(CH₃)₃]aminoethyl, N-[CH(CH₃)₂]-N-propylaminoethyl, N-Butyl-N-propylaminoethyl, N-(1-Methylpropyl)-N-propylaminoethyl, N-(2-Methylpropyl)-N-propylaminoethyl, N- [C(CH₃)₃]-N-propylaminoethyl, N-Butyl-N-[CH(CH₃)₂]aminoethyl, N-[CH(CH₃)₂]-N-(1-methylpropyl) aminoethyl, N-[CH(CH₃)₂]-N-(2-methylpropyl)-aminoethyl, N-[C(CH₃)₃]-N-[CH(CH₃)₂]aminoethyl, N-Butyl-N-(1-methylpropyl)aminoethyl, N-Butyl-N-(2-methylpropyl)aminoethyl, N-Butyl-N-[C(CH₃)₃]aminoethyl, N-(1-Methylpropyl)-N-(2-methylpropyl)aminoethyl, N-[C(CH₃)₃]-N-(1-methylpropyl)-aminoethyl oder N-[C(CH₃)₃]-N-(2-methylpropyl)aminoethyl, insbesondere für N,N-Dimethylaminoethyl oder N,N-Diethylaminoethyl;
- Di(C₁-C₄-alkyl)aminocarbonyl für: CO-N(CH₃)₂, CO-N(C₂H₅), CO-N(CH₂-C₂H₅)₂, CO-N[CH(CH₃)₂]₂, CO-N(n-C₄H₉)₂, CO-N[CH(CH₃)-C₂H₅]₂, CO-N[CH₂-CH(CH₃)₂]₂, CO-N[C(CH₃)₃]₂, N-Ethyl-N-methylaminocarbonyl, N-Methyl-N-propylaminocarbonyl, N-Methyl-N-[CH(CH₃)₂]aminocarbonyl, N-Butyl-N-methylaminocarbonyl, N-Methyl-N-(1-methylpropyl)aminocarbonyl, N-Methyl-N- (2-methylpropyl) aminocarbonyl, N- [C(CH₃)₃]-N-methylaminocarbonyl, N-Ethyl-N-propylaminocarbonyl, N-Ethyl-N-[CH(CH₃)₂]-aminocarbonyl, N-Butyl-N-ethylaminocarbonyl, N-Ethyl-N-(l-methylpropyl)aminocarbonyl, N-Ethyl-N-(2-methylpropyl)aminocarbonyl, N-Ethyl-N-[C(CH₃)₃]aminocarbonyl, N-[CH(CH₃)₂]-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-[C(CH₃)₃]-N-propylaminocarbonyl, N-Butyl-N-[CH(CH₃)₂]aminocarbonyl, N-[CH(CH₃)₂-N-(1-methylpropyl)aminocarbonyl, N- [CH(CH₃)₂]-N-(2-methylpropyl)aminocarbonyl, N-[C(CH₃)₃]-N-[CH(CH₃)₂]aminocarbonyl, N-Butyl-N-(1-methylpropyl)aminocarbonyl, N-Butyl-N-(2-methylpropyl)-aminocarbonyl, N-Butyl-N-[C(CH₃)₃]aminocarbonyl, N-(1-Methylpropyl) -N-(2-methylpropyl aminocarbonyl, N- [C(CH₃)₃]-N-(1-methylpropyl) aminocarbonyl oder N-[C(CH₃)₃]-N-(2-methylpropyl)aminocarbonyl, vorzugsweise für CO-N(CH₃)₂ oder CO-N(C₂H₅)₂;
- Di(C₁-C₄-alkyl)aminocarbonyl-C₁-C₄-alkyl für: durch Di(C₁-C₄alkyl)aminocarbonyl wie vorstehend genannt, vorzugsweise CO-N(CH₃)₂ oder CO-N(C₂H₅), substituiertes C₁-C₄-Alkyl, also z.B. für CH₂-CO-N(CH₃)₂, CH₂-CO-N(C₂H₅)₂, CH(CH₃)-CO-N(CH₃)₂ oder CH(CH₃)-CO-N(C₂H₅)₂, vorzugsweise für CH₂-CO-N(CH₃)₂ oder CH(CH₃)-CO-N(CH₃)₂;
- Di(C₁-C₄-alkyl)phosphonyl für: -PO(OCH₃)₂, -PO(OC₂H₅)₂, N,N-Dipropylphosphonyl, N,N-Di-(1-methylethyl)phosphonyl, N,N-Dibutylphosphonyl, N,N-Di-(1-methylpropyl)phosphonyl, N,N-Di-(2-methylpropyl)phosphonyl, N,N-Di-(1,1-dimethylethyl)phosphonyl, N-Ethyl-N-methylphosphonyl, N-Methyl-N-propylphosphonyl, N-Methyl-N-(1-methylethyl)phosphonyl, N-Butyl-N-methylphosphonyl, N-Methyl-N-(1-methylpropyl)-phosphonyl, N-Methyl-N-(2-methylpropyl)phosphonyl, N-(1,1-Dimethylethyl) -N-methylphosphonyl, N-Ethyl-N-propylphosphonyl, N-Ethyl-N-(1-methylethyl)phosphonyl, N-Butyl-N-ethylphosphonyl, N-Ethyl-N-(1-methylpropyl)phosphonyl, N-Ethyl-N-(2-methylpropyl)phosphonyl, N-Ethyl-N-(1,1-dimethylethyl)phosphonyl, N-(1-Methylethyl)-N-propylphosphonyl, N-Butyl-N-propylphosphonyl, N-(1-Methylpropyl)-N-propylphosphonyl, N-(2-Methylpropyl)-N-propylphosphonyl, N-(1,1-Dimethylethyl)-N-propylphosphonyl, N-Butyl-N-(1-methylethyl)phosphonyl, N-(1-Methylethyl)-N-(1-methylpropyl)phosphonyl, N-(1-Methylethyl)-N-(2-methylpropyl)phosphonyl, N-(1,1-Dimethylethyl)-N-(1-methylethyl)phosphonyl, N-Butyl-N-(1-methylpropyl)phosphonyl, N-Butyl-N- (2-methylpropyl)phosphonyl, N-Butyl-N-(1,1-dimethylethyl)phosphonyl, N-(1-Methylpropyl)-N-(2-methylpropyl)-phosphonyl, N-(1,1-Dimethylethyl)-N-(1-methylpropyl)phosphonyl oder N-(1,1-Dimethylethyl)-N-(2-methylpropyl)phosphonyl, vorzugsweise für -PO(OCH₃)₂ oder -PO(OC₂H₅)₂;
- Di(C₁-C₄-alkyl)phosphonyl-C₁-C₄-alkyl für: durch Di(C₁-C₄-alkyl)phosphonyl wie vorstehend genannt, vorzugsweise -PO(OCH₃)₂ oder -PO(OC₂H₅)₂, substituiertes C₁-C₄-Alkyl, also z.B. für CH₂-PO(OCH₃)₂, CH₂-PO(OC₂H₅)₂, CH(CH₃)-PO(OCH₃)₂ oder CH(CH₃)-PO(OC₂H₅)₂;
- C₃-C₆-Alkenyl für: Prop-1-en-1-yl, Allyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl, 1-Methylprop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methylbut-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methylpent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methylpent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethylbut-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₃-C₆-Halogenalkenyl für: C₃-C₆-Alkenyl wie vorstehend genannt, das partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- Cyano-C₃-C₆-alkenyl für: z.B. 2-Cyanoallyl, 3-Cyanoallyl, 4-Cyanobut-2-enyl, 4-Cyanobut-3-enyl oder 5-Cyanopent-4-enyl;
- C₃-C₆-Alkinyl für: Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methylpent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für Prop-2-in-1-yl;
- C₃-C₆-Halogenalkinyl für: C₃-C₆-Alkinyl wie vorstehend genannt, das partiell oder vollständig durch Fluor, Chlor, Brom und/oder Iod substituiert ist, also z.B. 1,1-Difluorprop-2-in-1-yl, 4-Fluorbut-2-in-1-yl, 4-Chlorbut-2-in-1-yl, 1,1-Difluorbut-2-in-1-yl, 5-Fluorpent-3-in-1-yl oder 6-Fluorhex-4-in-1-yl;
- Cyano-C₃-C₆-alkinyl für: z.B. 3-Cyanopropargyl, 4-Cyanobut-2-in-1-yl, 5-Cyanopent-3-in-1-yl und 6-Cyanohex-4-in-1-yl;
- C₃-C₄-Alkenyloxy-C₁-C₄-alkyl für: durch C₃-C₄-Alkenyloxy wie Allyloxy, But-1-en-3-yloxy, But-1-en-4-yloxy, But-2-en-1-yloxy, 1-Methylprop-2-enyloxy oder 2-Methylprop-2-enyloxy substituiertes C₁-C₄-Alkyl, also beispielsweise für Allyloxymethyl, 2-Allyloxyethyl oder But-1-en-4-yloxymethyl, insbesondere für 2-Allyloxyethyl;
- C₃-C₄-Alkinyloxy-C₁-C₄-alkyl für: durch C₃-C₄-Alkinyloxy wie Propargyloxy, But-1-in-3-yloxy, But-1-in-4-yloxy, But-2-in-1-yloxy, 1-Methylprop-2-inyloxy oder 2-Methylprop-2-inyloxy, vorzugsweise Propargyloxy, substituiertes C₁-C₄-Alkyl, also beispielsweise für Propargyloxymethyl oder 2-Propargyloxyethyl, insbesondere für 2-Propargyloxyethyl;
- (C₃-C₄-Alkenyloxy)imino-C₁-C₄-alkyl für: durch (C₃-C₄-Alkenyloxy)imino wie Allyloxyimino, But-1-en-3-yloxyimino, But-1-en-4-yloxyimino, But-2-en-1-yloxyimino, 1-Methylprop-2-enyloxyimino oder 2-Methylprop-2-enyloxyimino substituiertes C₁-C₄-Alkyl, also beispielsweise für Allyloxy-N=CH-CH₂ oder But-1-en-4-yloxy-N=CH, insbesondere für Allyloxy-N=CH-CH₂;
- C₃-C₄-Alkenylthio-C₁-C₄-alkyl für: durch C₃-C₄-Alkenylthio wie Allylthio, But-1-en-3-ylthio, But-1-en-4-ylthio, But-2-en-1-ylthio, 1-Methylprop-2-enylthio oder 2-Methylprop-2-enylthio substituiertes C₁-C₄-Alkyl, also beispielsweise für Allylthiomethyl, 2-Allylthioethyl oder But-1-en-4-ylthiomethyl, insbesondere für 2-(Allylthio)ethyl;
- C₃-C₄-Alkinylthio-C₁-C₄-alkyl für: durch C₃-C₄-Alkinylthio wie Propargylthio, But-1-in-3-ylthio, But-1-in-4-ylthio, But-2-in-1-ylthio, 1-Methylprop-2-inylthio oder 2-Methylprop-2-inylthio, vorzugsweise Propargylthio, substituiertes C₁-C₄-Alkyl, also beispielsweise für Propargylthiomethyl oder 2-Propargylthioethyl, insbesondere für 2-(Propargylthio)-ethyl;
- C₃-C₄-Alkenylsulfinyl-C₁-C₄-alkyl für: durch C₃-C₄-Alkenylsulfinyl wie Allylsulfinyl, But-1-en-3-ylsulfinyl, But-1-en-4-ylsulfinyl, But-2-en-1-ylsulfinyl, 1-Methylprop-2-enylsulfinyl oder 2-Methylprop-2-enylsulfinyl substituiertes C₁-C₄-Alkyl, also beispielsweise für Allylsulfinylmethyl, 2-Allylsulfinylethyl oder But-1-en-4-ylsulfinylmethyl, insbesondere für 2-(Allylsulfinyl)ethyl;
- C₃-C₄-Alkinylsulfinyl-C₁-C₄-alkyl für: durch C₃-C₄-Alkinylsulfinyl wie Propargylsulfinyl, But-1-in-3-ylsulfinyl, But-1-in-4-ylsulfinyl, But-2-in-1-ylsulfinyl, 1-Methylprop-2-inylsulfinyl oder 2-Methylprop-2-inylsulfinyl, vorzugsweise Propargylsulfinyl, substituiertes C₁-C₄-Alkyl, also beispielsweise für Propargylsulfinylmethyl oder 2-Propargylsulfinylethyl, insbesondere für 2-(Propargylsulfinyl)ethyl;
- C₃-C₄-Alkenylsulfonyl-C₁-C₄-alkyl für: durch C₃-C₄-Alkenylsulfonyl wie Allylsulfonyl, But-1-en-3-ylsulfonyl, But-1-en-4-ylsulfonyl, But-2-en-1-ylsulfonyl, l-Mechylprop-2-enylsulfonyl oder 2-Methylprop-2-enylsulfonyl substituiertes C₁-C₄-Alkyl, also beispielsweise für Allylsulfonylmethyl, 2-Allylsulfonylethyl oder But-1-en-4-ylsulfonylmethyl, insbesondere für 2-(Allylsulfonyl)ethyl;
- C₃-C₄-Alkinylsulfonyl-C₁-C₄-alkyl für: durch C₃-C₄-Alkinylsulfonyl wie Propargylsulfonyl, But-1-in-3-ylsulfonyl, But-1-in-4-ylsulfonyl, But-2-in-1-ylsulfonyl, 1-Methylprop-2-inylsulfonyl oder 2-Methylprop-2-inylsulfonyl, vorzugsweise Propargylsulfonyl, substituiertes C₁-C₄-Alkyl, also beispielsweise für Propargylsulfonylmethyl oder 2-Propargylsulfonylethyl, insbesondere für 2-(Propargylsulfonyl)ethyl;
- C₃-C₈-Cycloalkyl für: Cyclopropyl, Cyclobutyl, Cyclopentyl, Cyclohexyl, Cycloheptyl oder Cyclooctyl;
- C₃-C₈-Cycloalkyl-C₁-C₆-alkyl für: z.B. Cyclopropylmethyl, Cyclobutylmethyl, Cyclopentylmethyl, Cyclohexylmethyl, Cycloheptylmethyl, Cyclooctylmethyl, 2-(Cyclopropyl)ethyl, 2-(Cyclobutyl)-ethyl, 2-(Cyclopentyl)ethyl, 2-(Cyclohexyl)ethyl, 2-(Cycloheptyl)ethyl, 2-(Cyclooctyl)ethyl, 3-(Cyclopropyl)propyl, 3-(Cyclobutyl)propyl, 3-(Cyclopentyl)propyl, 3-(Cyclohexyl)-propyl, 3-(Cycloheptyl)propyl, 3-(Cyclooctyl)propyl, 4-(Cyclopropyl)butyl, 4-(Cyclobutyl)butyl, 4-(Cyclopentyl)butyl, 4-(Cyclohexyl)butyl, 4-(Cycloheptyl)butyl, 4-(Cyclooctyl)butyl, 5-(Cyclopropyl)pentyl, 5-(Oyclobutyl)pentyl, 5-(Cyclopentyl)-pentyl, 5-(Cyclohexyl)pentyl, 5-(Cycloheptyl)pentyl, 5-(Cyclooctyl)pentyl, 6-(Cyclopropyl)hexyl, 6-(Cyclobutyl)hexyl, 6-(Cyclopentyl)hexyl, 6-(Cyclohexyl)hexyl, 6-(Cycloheptyl)hexyl oder 6-(Cyclooctyl)hexyl;
- C₃-C₈-Cycloalkyl, das ein Carbonyl- oder Thiocarbonyl-Ringglied enthält, z.B. für Cyclobutanon-2-yl, Cyclobutanon-3-yl, Cyclopentanon-2-yl, Cyclopentanon-3-yl, Cyclohexanon-2-yl, Cyclohexanon-4-yl, Cycloheptanon-2-yl, Cyclooctanon-2-yl, Cyclobutanthion-2-yl, Cyclobutanthion-3-yl, Cyclopentanthion-2-yl, Cyclopentanthion-3-yl, Cyclohexanthion-2-yl, Cyclohexanthion-4-yl, Cycloheptanthion-2-yl oder Cyclooctanthion-2-yl, vorzugsweise für Cyclopentanon-2-yl oder Cyclohexanon-2-yl;
- C₃-C₈-Cycloalkyl-C₁-C₄-alkyl, das ein Carbonyl- oder Thiocarbonyl-Ringglied enthält, für: z.B. Cyclobutanon-2-ylmethyl, Cyclobutanon-3-ylmethyl, Cyclopentanon-2-ylmethyl, Cyclopentanon-3-ylmethyl, Cyclohexanon-2-ylmethyl, Cyclohexanon-4-ylmethyl, Cycloheptanon-2-ylmethyl, Cyclooctanon-2-ylmethyl, Cyclobutanthion-2-ylmethyl, Cyclobutanthion-3-ylmethyl, Cyclopentanthion-2-ylmethyl, Cyclopencanthion-3-ylmethyl, Cyclohexanthion-2-ylmethyl, Cyclohexanthion-4-ylmethyl, Cycloheptanthion-2-ylmethyl, Cyclooctanthion-2-ylmethyl, 1-(Cyclobutanon-2-yl)ethyl, 1-(Cyclobutanon-3-yl)ethyl, 1-(Cyclopentanon-2-yl)-ethyl, 1-(Cyclopentanon-3-yl)ethyl, 1-(Cyclohexanon-2-yl)ethyl, 1-(Cyclohexanon-4-yl)ethyl, 1-(Cycloheptanon-2-yl)ethyl, 1-(Cyclooctanon-2-yl)ethyl, 1-(Cyclobutanthion-2-yl)ethyl, 1-(Cyclobutanthion-3-yl)ethyl, 1-(Cyclopentanthion-2-yl)ethyl, 1-(Cyclopentanthion-3-yl)ethyl, 1-(Cyclohexanthion-2-yl)ethyl, 1-(Cyclohexanthion-4-yl)ethyl, 1-(Cycloheptanthion-2-yl)ethyl, 1-(Cyclooctanthion-2-yl)ethyl, 2-(Cyclobutanon-2-yl)ethyl, 2-(Cyclobutanon-3-yl)ethyl, 2-(Cyclopentanon-2-yl)ethyl, 2-(Cyclopentanon-3-yl)ethyl, 2-(Cyclohexanon-2-yl)ethyl, 2-(Cyclohexanon-4-yl)ethyl, 2-(Cycloheptanon-2-yl)ethyl, 2-(Cyclooctanon-2-yl)ethyl, 2-(Cyclobutanthion-2-yl)ethyl, 2-(Cyclobutanthion-3-yl)ethyl, 2-(Cyclopentanthion-2-yl)ethyl, 2-(Cyclopentanthion-3-yl)ethyl, 2-(Cyclohexanthion-2-yl)ethyl, 2-(Cyclohexanthion-4-yl)ethyl, 2-(Cycloheptanthion-2-yl)ethyl, 2-(Cyclooctanthion-2-yl)ethyl, 3-(Cyclobutanon-2-yl)propyl, 3-(Cyclobutanon-3-yl)propyl, 3-(Cyclopentanon-2-yl)propyl, 3-(Cyclopentanon-3-yl)propyl, 3-(Cyclohexanon-2-yl)propyl, 3-(Cyclohexanon-4-yl)propyl, 3-(Cycloheptanon-2-yl)propyl, 3-(Cyclooctanon-2-yl)propyl, 3-(Cyclobutanthion-2-yl)propyl, 3-(Cyclobutanthion-3-yl)propyl, 3-(Cyclopentanthion-2-yl)-propyl, 3-(Cyclopentanthion-3-yl)propyl, 3-(Cyclohexanthion-2-yl)propyl, 3-(Cyclohexanthion-4-yl)propyl, 3-(Cycloheptanthion-2-yl)propyl, 3-(Cyclooctanthion-2-yl)propyl, 4-(Cyclobutanon-2-yl)butyl, 4-(Cyclobutanon-3-yl)butyl, 4-(Cyclopentanon-2-yl)butyl, 4-(Cyclopentanon-3-yl)butyl, 4-(Cyclohexanon-2-yl)butyl, 4-(Cyclohexanon-4-yl)butyl, 4-(Cycloheptanon-2-yl)butyl, 4-(Cyclooctanon-2-yl)butyl, 4-(Cyclobutanthion-2-yl)butyl, 4-(Cyclobutanthion-3-yl)butyl, 4-(Cyclopentanthion-2-yl)butyl, 4-(Cyclopentanthion-3-yl)butyl, 4-(Cyclohexanthion-2-yl)butyl, 4-(Cyclohexanthion-4-yl)butyl, 4-(Cycloheptanthion-2-yl)butyl oder 4-(Cyclooctanthion-2-yl)butyl;
- C₃-C₈-Cycloalkyloxy-C₁-C₄-alkyl für: Cyclopropyloxymethyl, 1-Cyclopropyloxy-ethyl, 2-Cyclopropyloxy-ethyl, 1-Cyclopropyloxy-prop-1-yl, 2-Cyclopropyloxy-prop-1-yl, 3-Cyclopropyloxyprop-1-yl, 1-Cyclopropyloxy-but-1-yl, 2-Cyclopropyloxy-but-1-yl, 3-Cyclopropyloxy-but-1-yl, 4-Cyclopropyloxy-but-1-yl, 1-Cyclopropyloxy-but-2-yl, 2-Cyclopropyloxy-but-2-yl, 3-Cyclopropyloxy-but-2-yl, 3-Cyclopropyloxy-but-2-yl, 4-Cyclopropyloxy-but-2-yl, 1-(Cyclopropyloxymethyl)-eth-1-yl, 1-(Cyclopropyloxymethyl)-1-(CH₃)-eth-1-yl, 1-(Cyclopropylmethyloxy)-prop-1-yl, Cyclobutyloxymethyl, 1-Cyclobutyloxy-ethyl, 2-Cyclobutyloxy-ethyl, 1-Cyclobutyloxy-prop-1-yl, 2-Cyclobutyloxyprop-1-yl, 3-Cyclobutyloxy-prop-1-yl, 1-Cyclobutyloxy-but-1-yl, 2-Cyclobutyloxy-but-1-yl, 3-Cyclobutyloxy-but-1-yl, 4-Cyclobutyloxy-but-1-yl, 1-Cyclobutyloxy-but-2-yl, 2-Cyclobutyloxybut-2-yl, 3-Cyclobutyloxy-but-2-yl, 3-Cyclobutyloxy-but-2-yl, 4-Cyclobutyloxy-but-2-yl, 1-(Cyclobutyloxymethyl)eth-1-yl, 1-(Cyclobutyloxymethyl)-1-(CH₃)-eth-1-yl, 1-(Cyclobutyloxymethyl)prop-1-yl, Cyclopentyloxymethyl, 1-Cyclopentyloxy-ethyl, 2-Cyclopentyloxy-ethyl, 1-Cyclopentyloxy-prop-1-yl, 2-Cyclopentyloxy-prop-1-yl, 3-Cyclopentyloxy-prop-1-yl, 1-Cyclopentyloxy-but-1-yl, 2-Cyclopentyloxy-but-1-yl, 3-Cyclopentyloxy-but-1-yl, 4-Cyclopentyloxy-but-1-yl, 1-Cyclopentyloxy-but-2-yl, 2-Cyclopentyloxy-but-2-yl, 3-Cyclopentyloxy-but-2-yl, 3-Cyclopentyloxy-but-2-yl, 4-Cyclopentyloxy-but-2-yl, 1-(Cyclopentyloxymethyl)eth-1-yl, 1-(Cyclopentyloxymethyl)-1-(CH₃)-eth-1-yl, 1-(Cyclopentyloxymethyl)prop-1-yl, Cyclohexyloxymethyl, 1-Cyclohexyloxy-ethyl, 2-Cyclohexyloxy-ethyl, 1-Cyclohexyloxyprop-1-yl, 2-Cyclohexyloxy-prop-1-yl, 3-Cyclohexyloxy-prop-1-yl, 1-Cyclohexyloxy-but-1-yl, 2-Cyclohexyloxy-but-1-yl, 3-Cyclohexyloxy-but-1-yl, 4-Cyclohexyloxy-but-1-yl, 1-Cyclohexyloxy-but-2-yl, 2-Cyclohexyloxy-but-2-yl, 3-Cyclohexyloxybut-2-yl, 3-Cyclohexyloxy-but-2-yl, 4-Cyclohexyloxy-but-2-yl, 1-(Cyclohexyloxymethyl)eth-1-yl, 1-(Cyclohexyloxymethyl)-1-(CH₃)-eth-1-yl, 1-(Cyclohexyloxymethyl)-prop-1-yl, Cycloheptyloxymethyl, 1-Cycloheptyloxy-ethyl, 2-Cycloheptyloxy-ethyl, 1-Cycloheptyloxy-prop-1-yl, 2-Cycloheptyloxy-prop-1-yl, 3-Cycloheptyloxy-prop-1-yl, 1-Cycloheptyloxy-but-1-yl, 2-Cycloheptyloxy-but-1-yl, 3-Cycloheptyloxy-but-1-yl, 4-Cycloheptyloxy-but-1-yl, 1-Cycloheptyloxy-but-2-yl, 2-Cycloheptyloxy-but-2-yl, 3-Cycloheptyloxy-but-2-yl, 3-Cycloheptyloxy-but-2-yl, 4-Cycloheptyloxy-but-2-yl, 1-(Cycloheptyloxymethyl)eth-1-yl, 1-(Cycloheptyloxymethyl)-1-(CH₃)-eth-1-yl, 1-(Cycloheptyloxymethyl)prop-1-yl, Cyclooctyloxymethyl, 1-Cyclooctyloxy-ethyl, 2-Cyclooctyloxy-ethyl, 1-Cyclooctyloxy-prop-1-yl, 2-Cyclooctyloxy-prop-1-yl, 3-Cyclooctyloxy-prop-l-yl, 1-Cyclooctyloxy-but-1-yl, 2-Cyclooctyloxy-but-1-yl, 3-Cyclooctyloxy-but-1-yl, 4-Cyclooctyloxy-but-1-yl, 1-Cyclooctyloxy-but-2-yl, 2-Cyclooctyloxy-but-2-yl, 3-Cyclooctyloxy-but-2-yl, 3-Cyclooctyloxybut-2-yl, 4-Cyclooctyloxy-but-2-yl, 1-(Cyclooctyloxymethyl)-eth-1-yl, 1-(Cyclooctyloxymethyl)-1-(CH₃)-eth-1-yl oder 1-(Cyclooctyloxymethyl)prop-1-yl, insbesondere für C₃-C₆-Cycloalkoxymethyl oder 2-(C₃-C₆-Cycloalkoxy)ethyl.

Unter 3- bis 7gliedrigem Heterocyclyl sind sowohl gesättigte, partiell oder vollständig ungesättigte als auch aromatische Heterocyclen mit ein bis drei Heteroatomen, ausgewählt aus einer Gruppe bestehend aus
- ein bis drei Stickstoffatomen,
- einem oder zwei Sauerstoff- und
- einem oder zwei Schwefelatomen,
zu verstehen.

Beispiele für gesättigte Heterocyclen, die ein Carbonyl- oder Thiocarbonyl-Ringglied enthalten können, sind:
Oxiranyl, Thiiranyl, Aziridin-1-yl, Aziridin-2-yl, Diaziridin-1-yl, Diaziridin-3-yl, Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl, 1,3-Dioxepan-6-yl, 1,3-Dithiepan-2-yl, 1,3-Dithiepan-2-yl, 1,3-Dithiepan-2-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl und Hexahydro-1,4-diazepin-2-yl.

Beispiele für ungesättigte Heterocyclen, die ein Carbonyloder Thiocarbonyl-Ringglied enthalten können, sind:
Dihydrofuran-2-yl, 1,2-Oxazolin-3-yl, 1,2-Oxazolin-5-yl, 1,3-Oxazolin-2-yl;

Unter den Heteroaromaten sind die 5- und 6-gliedrigen bevorzugt, also z.B.
Furyl wie 2-Furyl und 3-Furyl, Thienyl wie 2-Thienyl und
3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Pyrimidyl, Furanyl und Thienyl.

Alle Phenyl-, carbocyclischen und heterocyclischen Ringe sind vorzugsweise unsubstituiert oder tragen einen Substituenten.

Im Hinblick auf die Verwendung der substituierten (4-Brompyrazol-3-yl)benzazole I als Herbizide oder Desikkantien/Defoliantien sind diejenigen Verbindungen I bevorzugt, bei denen die Variablen folgende Bedeutungen haben, und zwar jeweils für sich allein oder in Kombination:
- R¹: C₁-C₄-Alkyl, insbesondere Methyl;
- R²: C₁-C₄-Halogenalkyl, C₁-C₄-Halogenalkoxy oder C₁-C₄-Alkylsulfonyl, insbesondere C₁-C₄-Halogenalkoxy oder C₁-C₄-Alkylsulfonyl, besonders bevorzugt Difluormethoxy;
- R⁴: Wasserstoff, Fluor oder Chlor, insbesondere Fluor oder Chlor;
- R⁵: Cyano, Halogen oder Trifluormethyl, insbesondere Halogen, besonders bevorzugt Chlor;
- A: eine über Sauerstoff oder Schwefel an α gebundene Gruppe -N=C(XR⁶)-O- oder -N=C(XR⁶)-S-, insbesondere über den Sauerstoff an α gebundenes -N=C(XR⁶)-O-;
- X: eine chemische Bindung, Sauerstoff, Schwefel, -NH- oder -N(R⁷)-;
- R⁶, R⁷: unabhängig voneinander
C₁-C₆-Alkyl, C₁-C₆-Halogenalkyl, Cyano-C₁-C₄-alkyl, Hydroxy-C₁-C₄-alkyl, C₃-C₆-Alkenyl, C₃-C₆-Alkinyl, C₁-C₄-Alkoxy-C₁-C₄-alkyl, C₁-C₄-Alkylthio-C₁-C₄-alkyl, C₁-C₄-Alkylsulfonyl-C₁-C₄-alkyl, (C₁-C₄-Alkoxy)carbonyl-C₁-C₄-alkyl, Di(C₁-C₄-alkyl)aminocarbonyl-C₁-C₄-alkyl, C₃-C₈-Cycloalkyl, C₃-C₈-Cycloalkyl-C₁-C₄-alkyl, Phenyl oder Phenyl-C₁-C₄-alkyl;
sofern X eine chemische Bindung, Sauerstoff, Schwefel, -NH- oder -N(R⁷)- ist, kann R⁶ auch (C₁-C₄-Alkyl)carbonyl oder C₁-C₄-Alkylsulfonyl bedeuten;
sofern X eine chemische Bindung ist, kann R⁶ außerdem Wasserstoff, Cyano, Amino, Halogen oder -CH=CH-R⁸ bedeuten;
- R⁶, R⁷: jeweils insbesondere C₁-C₆-Alkyl, Hydroxy-C₁-C₄-alkyl, C₃-C₆-Alkenyl, C₃-C₆-Alkinyl oder (C₁-C₄-Alkoxy)carbonyl-C₁-C₄-alkyl;
sofern X eine chemische Bindung ist, kann R⁶ außerdem insbesondere Wasserstoff oder -CH=CH-R⁸ bedeuten;
- R⁸: (C₁-C₄-Alkoxy)carbonyl.

Ganz besonders bevorzugt sind die substituierten (4-Brompyrazol-3-yl)benzazole der Formel Ia (≙ I mit R¹ = Methyl, R² = Difluormethoxy, R⁴ = Wasserstoff, R⁵ = Chlor, Z = über den Schwefel an α gebundenes -N=C(XR⁶)-S-), insbesondere die in der folgenden Tabelle 1 aufgeführten Verbindungen:

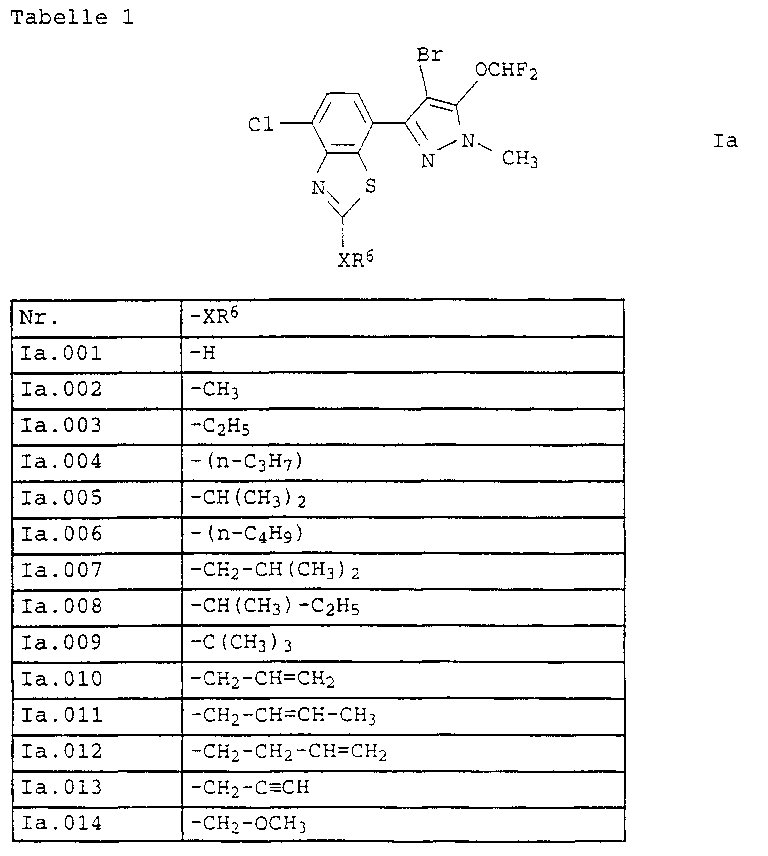

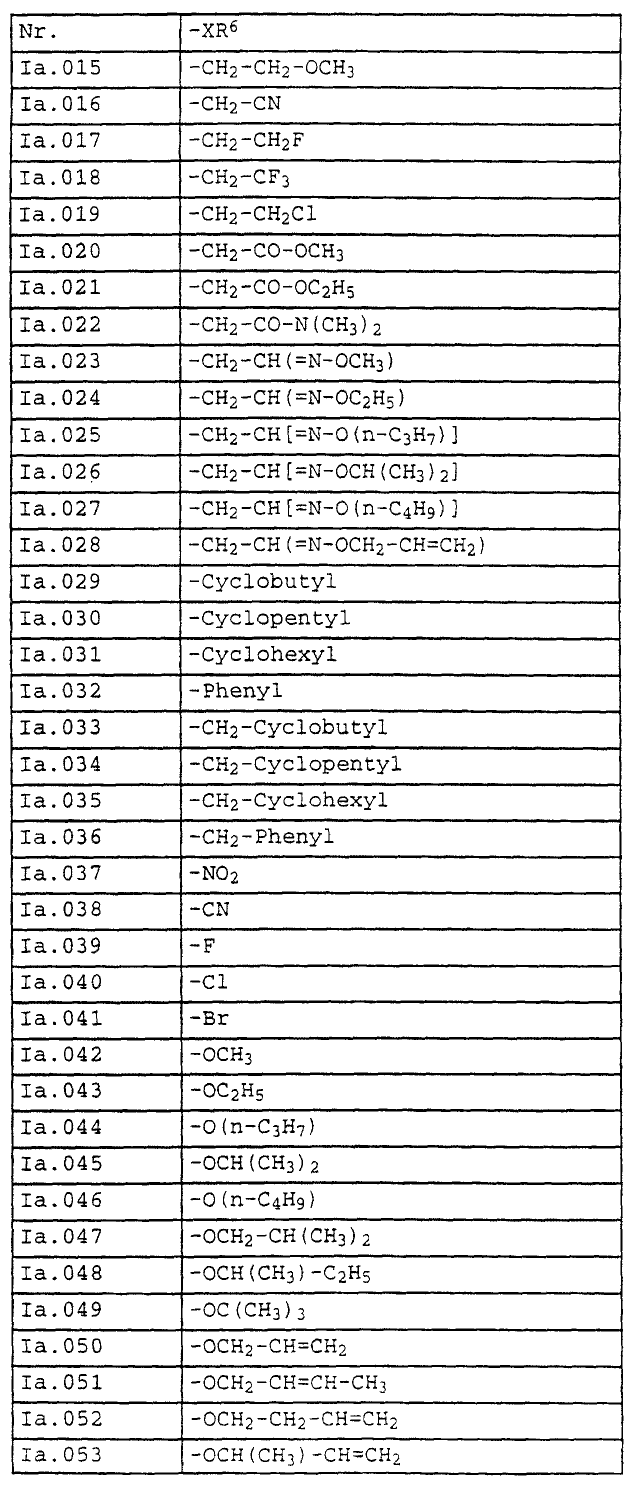

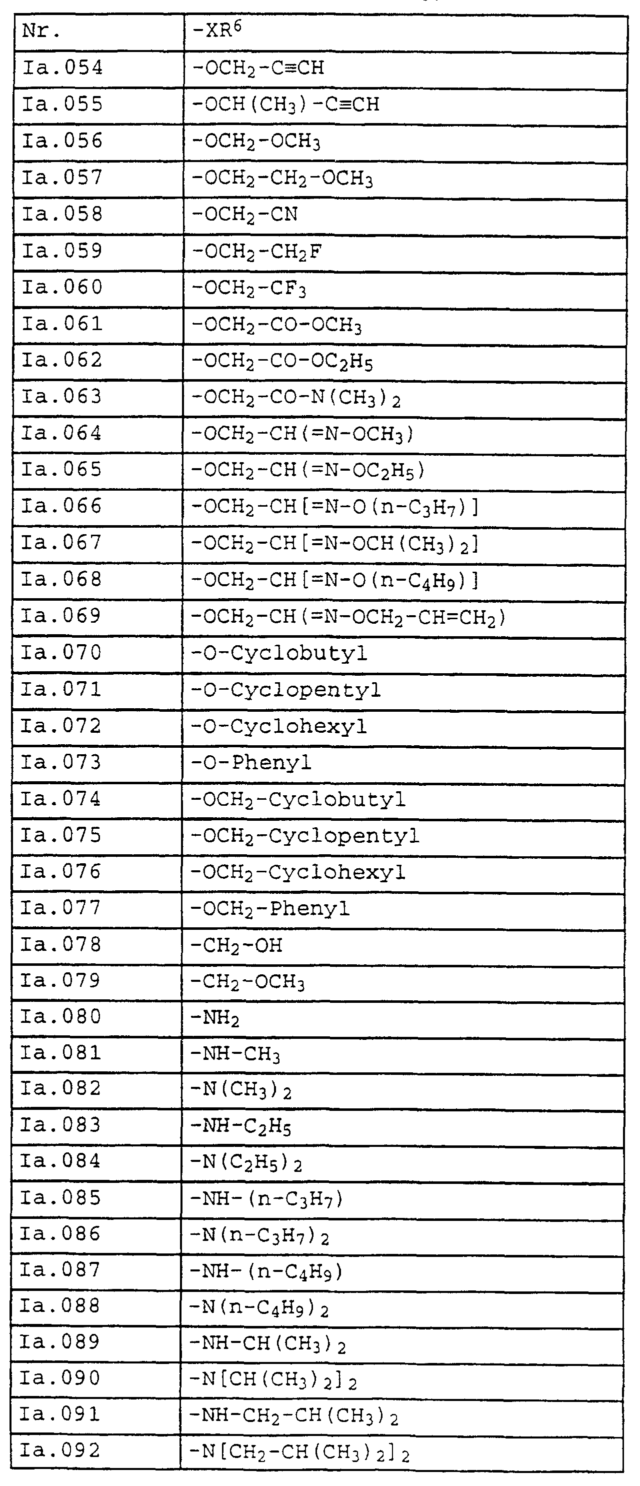

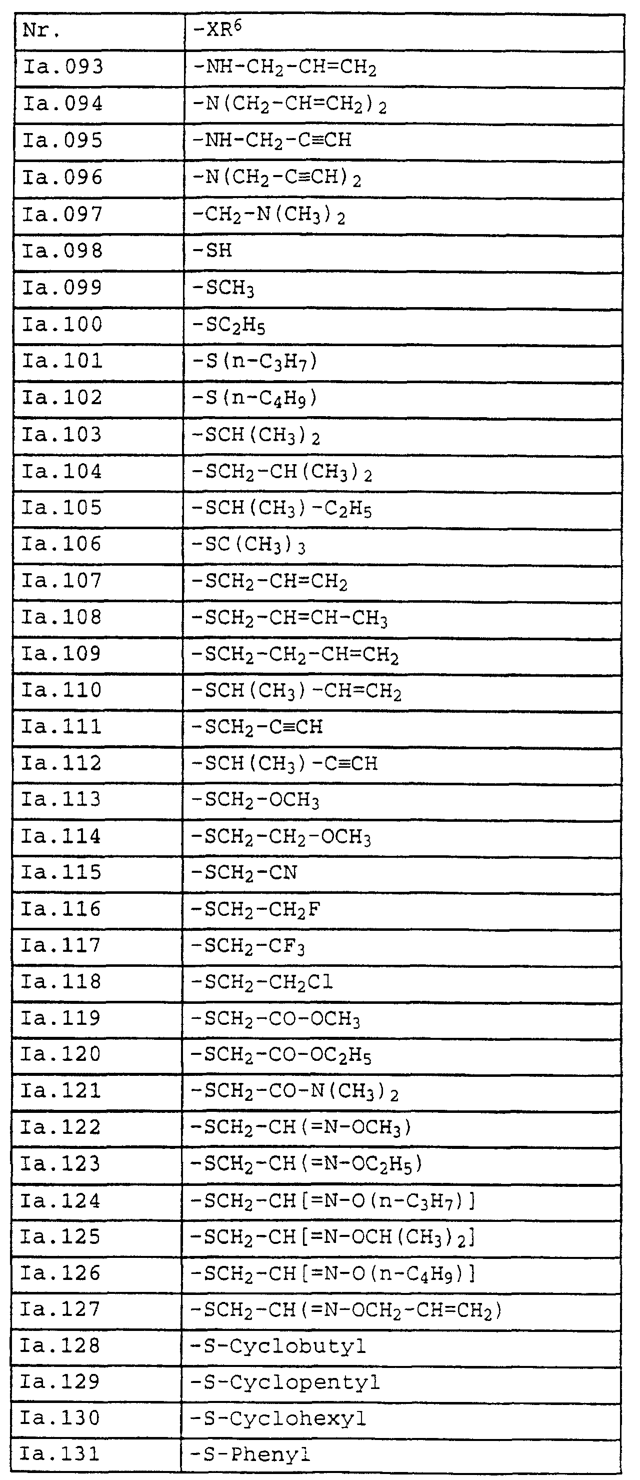

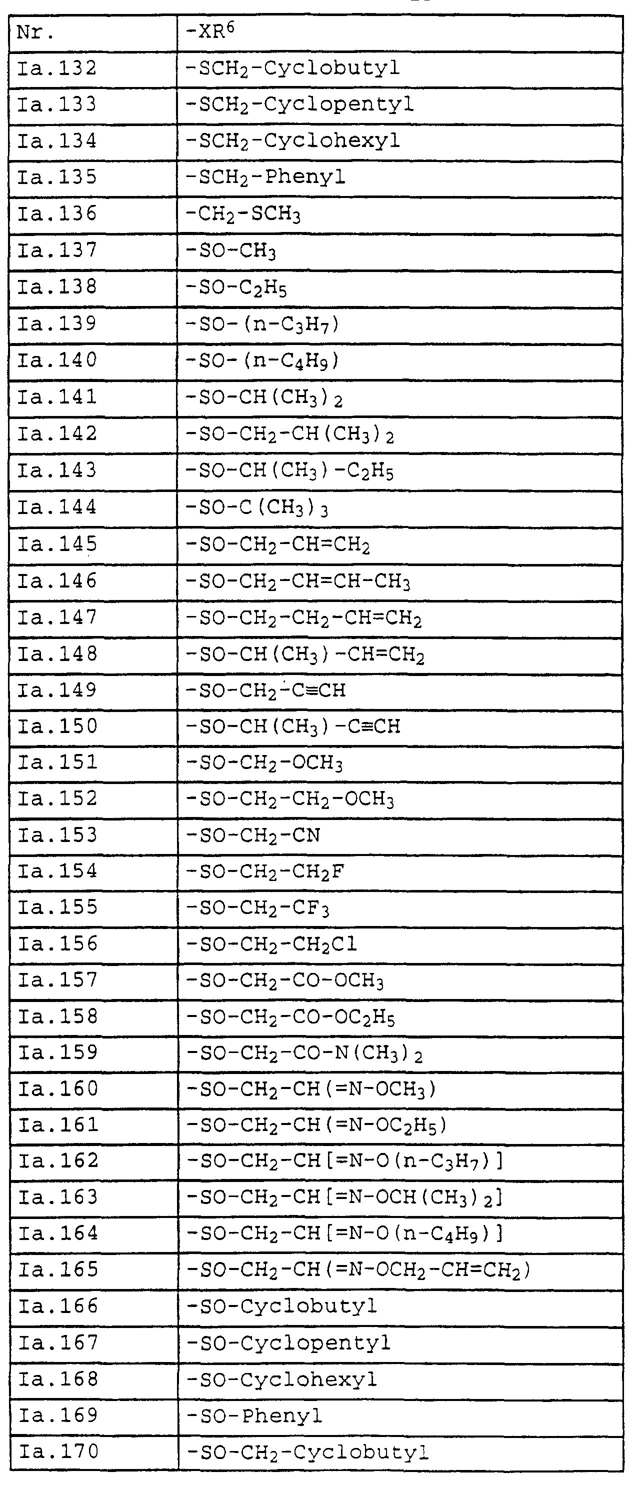

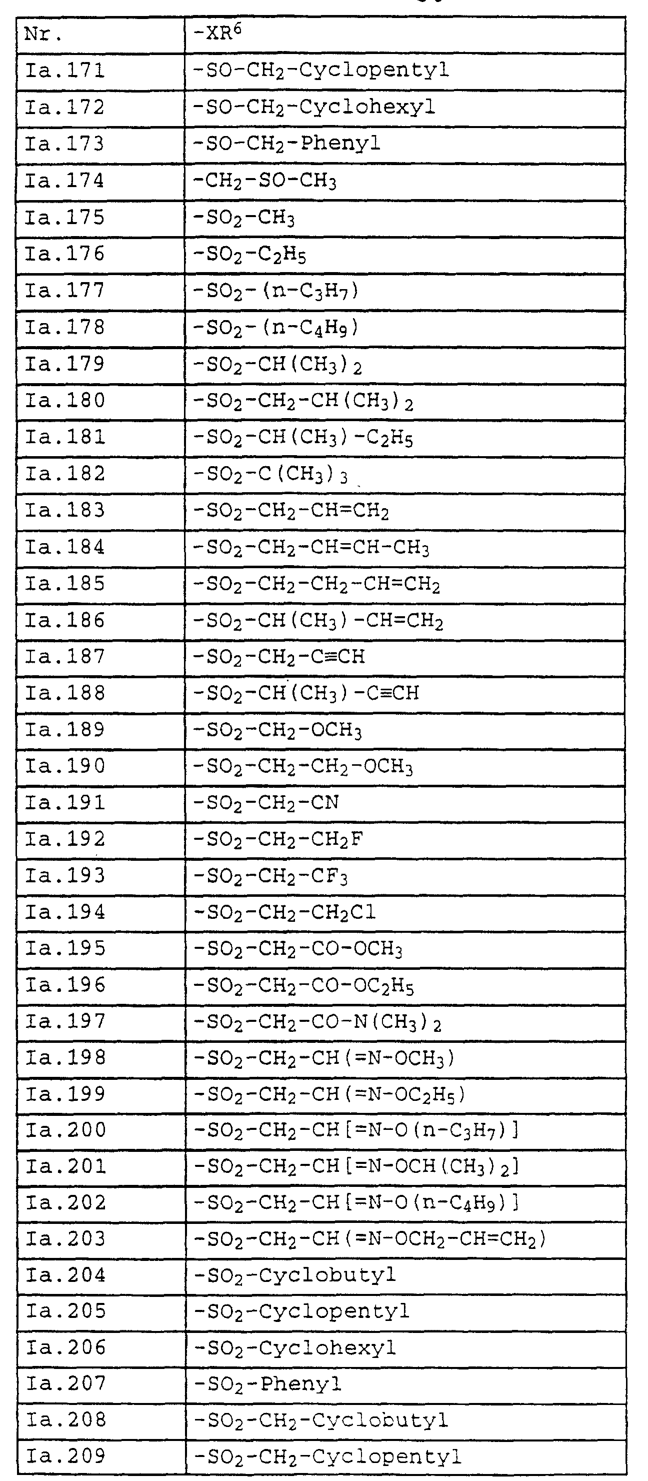

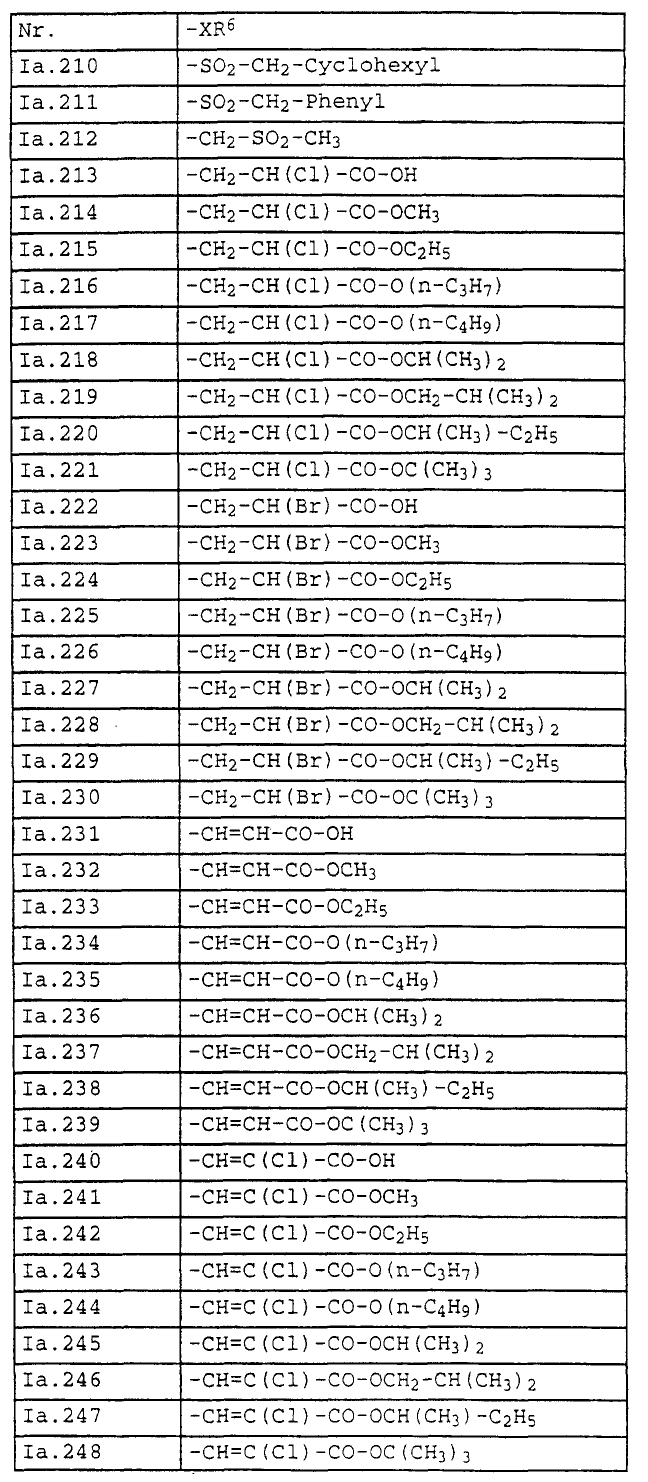

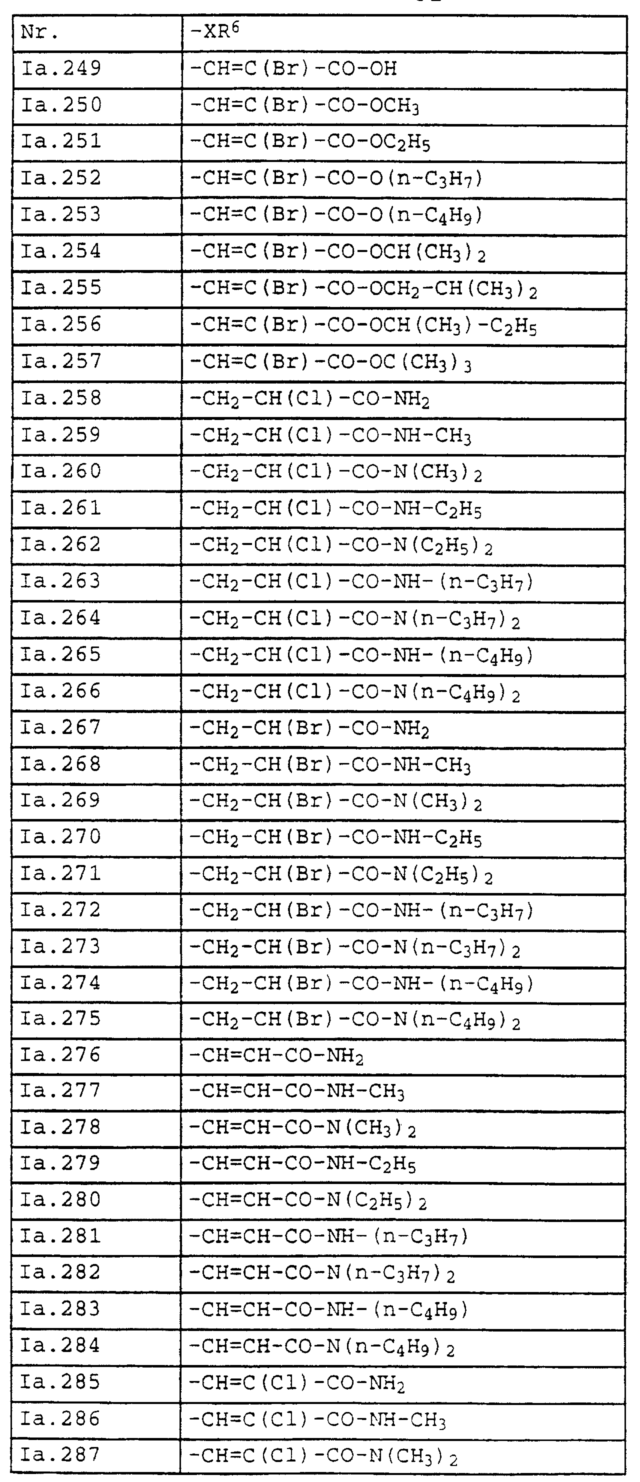

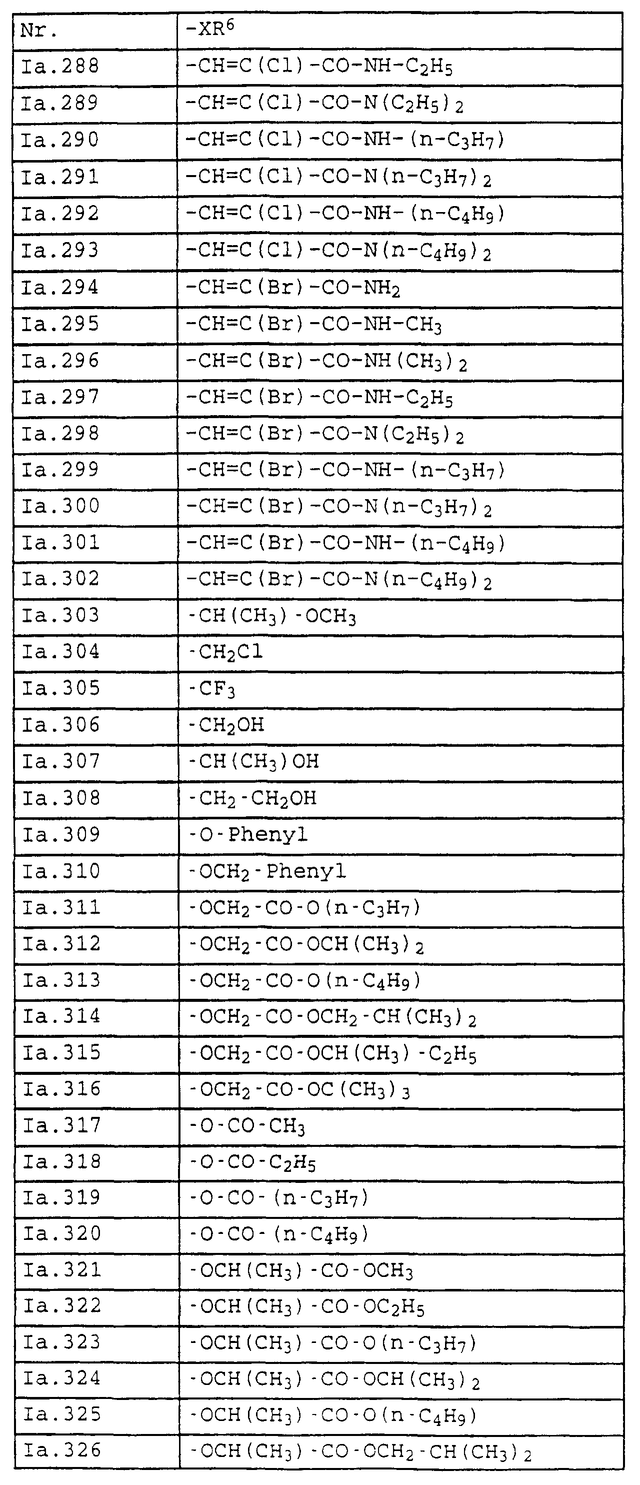

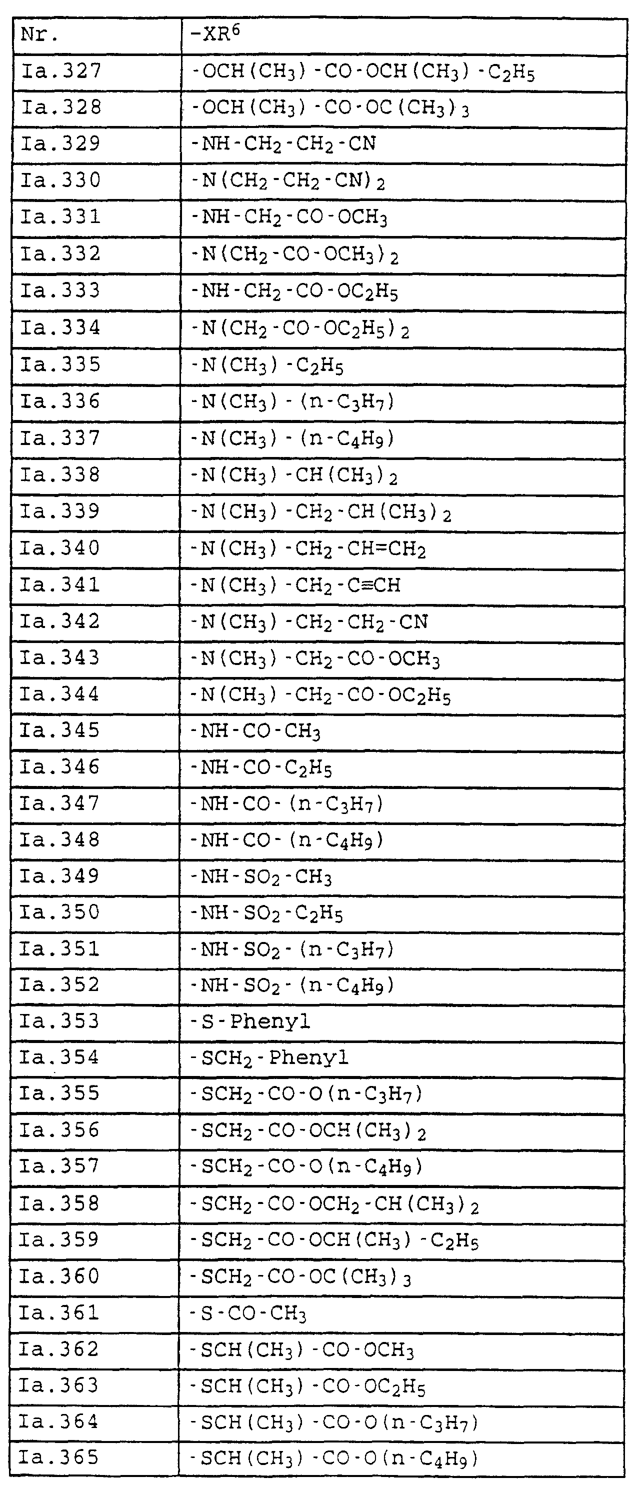

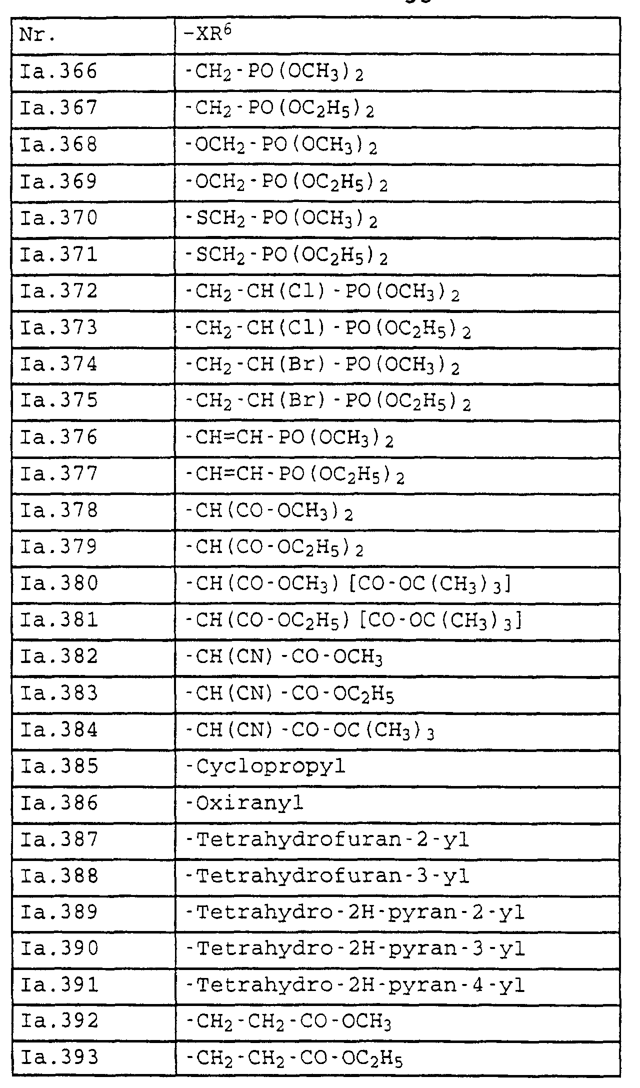

Des weiteren sind die substituierten (4-Brompyrazol-3-yl)benzazole der Formeln Ib bis It und IA bis IT besonders bevorzugt, insbesondere
- die Verbindungen Ib.001 bis Ib.393, die sich von den entsprechenden Verbindungen Ia.001 bis Ia.393 lediglich dadurch unterscheiden, daß R⁴ für Chlor steht: 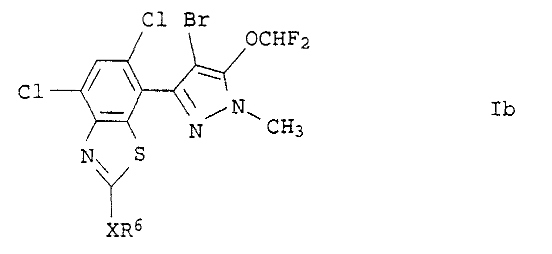
- die Verbindungen Ic.001 bis Ic.393, die sich von den entsprechenden Verbindungen Ia.001 bis Ia.393 lediglich dadurch unterscheiden, daß R⁴ für Fluor steht: 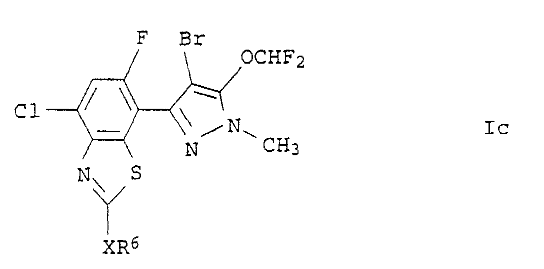
- die Verbindungen Id.001 bis Id.393, die sich von den entsprechenden Verbindungen Ia.001 bis Ia.393 lediglich dadurch unterscheiden, daß R² für Trifluormethyl steht: 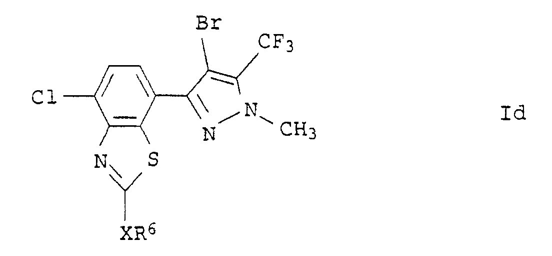
- die Verbindungen Ie.001 bis Ie.393, die sich von den entsprechenden Verbindungen Ia.001 bis Ia.393 lediglich dadurch unterscheiden, daß R² für Trifluormethyl und R⁴ für Chlor stehen: 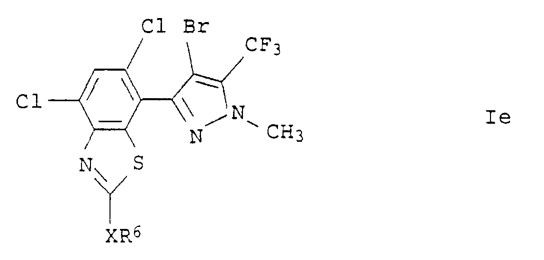
- die Verbindungen If.001 bis If.393, die sich von den entsprechenden Verbindungen Ia.001 bis Ia.393 lediglich dadurch unterscheiden, daß R² für Trifluormethyl und R⁴ für Fluor stehen: 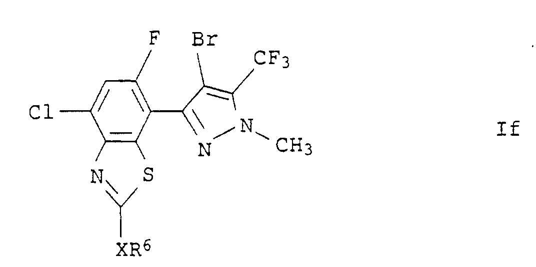
- die Verbindungen Ig.001 bis Ig.393, die sich von den entsprechenden Verbindungen Ia.001 bis Ia.393 lediglich dadurch unterscheiden, daß R² für Methylsulfonyl steht: 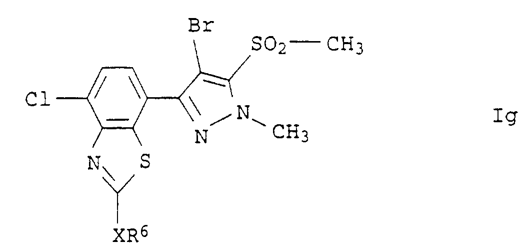
- die Verbindungen Ih.001 bis Ih.393, die sich von den entsprechenden Verbindungen Ia.001 bis Ia.393 lediglich dadurch unterscheiden, daß R² für Methylsulfonyl und R⁴ für Chlor stehen: 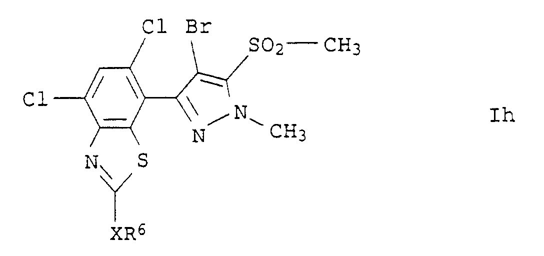
- die Verbindungen Ij.001 bis Ij.393, die sich von den entsprechenden Verbindungen Ia.001 bis Ia.393 lediglich dadurch unterscheiden, daß R² für Methylsulfonyl und R⁴ für Fluor stehen: 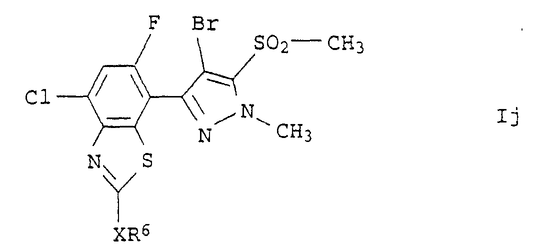
- die Verbindungen Ik.001 bis Ik.393, die sich von den entsprechenden Verbindungen Ia.001 bis Ia.393 lediglich dadurch unterscheiden, daß Z eine Gruppe -N=C(XR⁶)-O- bedeutet, die über den Sauerstoff an α gebunden ist: 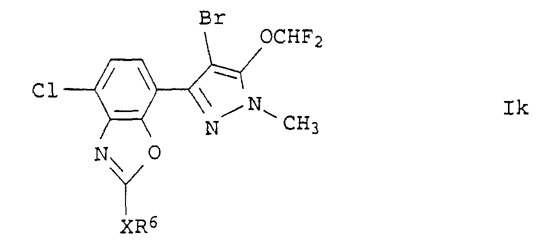
- die Verbindungen Im.001 bis Im.393, die sich von den entsprechenden Verbindungen Ia.001 bis Ia.393 dadurch unterscheiden, daß R⁴ für Chlor steht und Z eine Gruppe -N=C(XR⁶)-Obedeutet, die über den Sauerstoff an α gebunden ist: 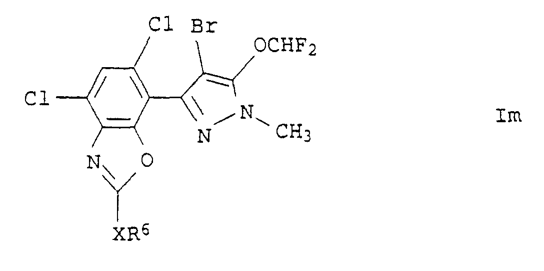
- die Verbindungen In.001 bis In.393, die sich von den entsprechenden Verbindungen Ia.001 bis Ia.393 dadurch unterscheiden, daß R⁴ für Fluor steht und Z eine Gruppe -N=C(XR⁶)-Obedeutet, die über den Sauerstoff an α gebunden ist: 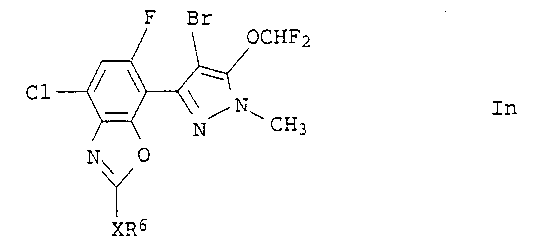
- die Verbindungen Io.001 bis Io.393, die sich von den entsprechenden Verbindungen Ia.001 bis Ia.393 dadurch unterscheiden, daß R² für Trifluormethyl steht und Z eine Gruppe -N=C(XR⁶)-O- bedeutet, die über den Sauerstoff an α gebunden ist: 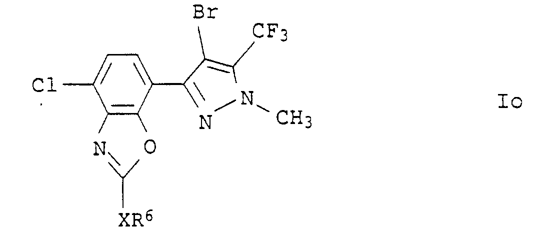
- die Verbindungen Ip.001 bis Ip.393, die sich von den entsprechenden Verbindungen Ia.001 bis Ia.393 dadurch unterscheiden, daß R² für Trifluormethyl und R⁴ für Chlor stehen und Z eine Gruppe -N=C(XR⁶)-O- bedeutet, die über den Sauerstoff an α gebunden ist: 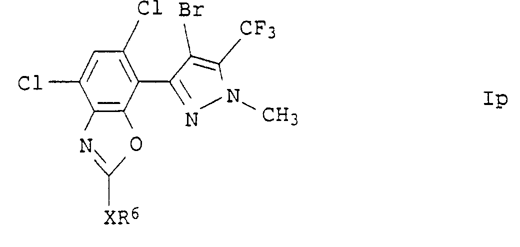
- die Verbindungen Iq.001 bis Iq.393, die sich von den entsprechenden Verbindungen Ia.001 bis Ia.393 dadurch unterscheiden, daß R² für Trifluormethyl und R⁴ für Fluor stehen und Z eine Gruppe -N=C(XR⁶)-O- bedeutet, die über den Sauerstoff an α gebunden ist: 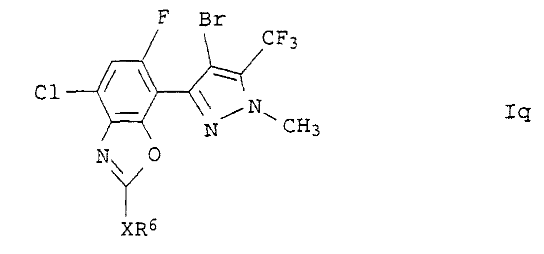
- die Verbindungen Ir.001 bis Ir.393, die sich von den entsprechenden Verbindungen Ia.001 bis Ia.393 dadurch unterscheiden, daß R² für Methylsulfonyl steht und Z eine Gruppe -N=C(XR⁶)-O- bedeutet, die über den Sauerstoff an α gebunden ist: 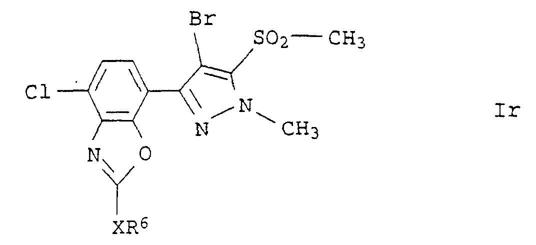
- die Verbindungen Is.001 bis Is.393, die sich von den entsprechenden Verbindungen Ia.001 bis Ia.393 dadurch unterscheiden, daß R² für Methylsulfonyl und R⁴ für Chlor stehen und Z eine Gruppe -N=C(XR⁶)-O- bedeutet, die über den Sauerstoff an α gebunden ist: 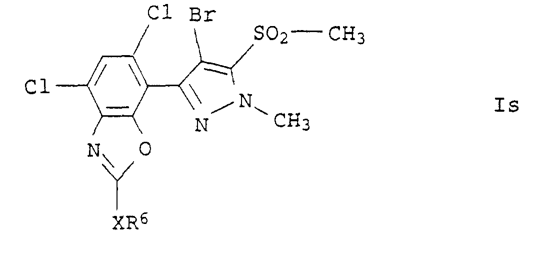
- die Verbindungen It.001 bis It.393, die sich von den entsprechenden Verbindungen Ia.001 bis Ia.393 dadurch unterscheiden, daß R² für Methylsulfonyl und R⁴ für Fluor stehen und Z eine Gruppe -N=C(XR⁶)-O- bedeutet, die über den Sauerstoff an α gebunden ist: 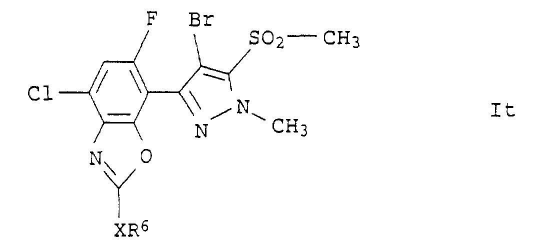
- die Verbindungen IA.001 bis IA.393, die sich von den entsprechenden Verbindungen Ia.001 bis Ia.393 lediglich dadurch unterscheiden, daß die Gruppe -N=C(XR⁶)-S- über den Stickstoff an α gebunden ist: 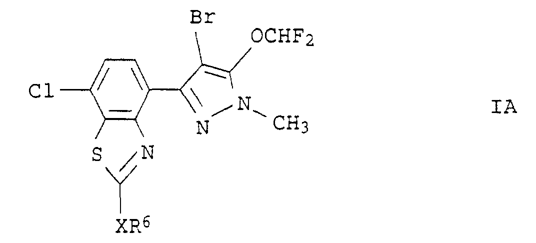
- die Verbindungen IB.001 bis IB.393, die sich von den entsprechenden Verbindungen Ia.001 bis Ia.393 lediglich dadurch unterscheiden, daß R⁴ für Chlor steht und die Gruppe -N=C(XR⁶)-S- über den Stickstoff an α gebunden ist: 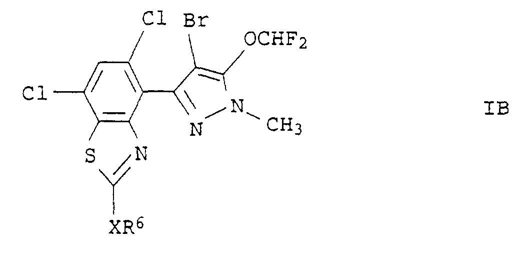
- die Verbindungen IC.001 bis IC.393, die sich von den entsprechenden Verbindungen Ia.001 bis Ia.393 lediglich dadurch unterscheiden, daß R⁴ für Fluor steht und die Gruppe -N=C(XR₆)-S- über den Stickstoff an α gebunden ist: 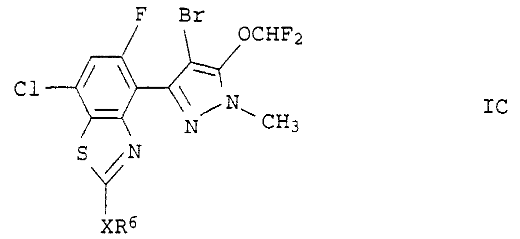
- die Verbindungen ID.001 bis ID.393, die sich von den entsprechenden Verbindungen Ia.001 bis Ia.393 lediglich dadurch unterscheiden, daß R² für Trifluormethyl steht und die Gruppe -N=C(XR⁶)-S- über den Stickstoff an α gebunden ist: 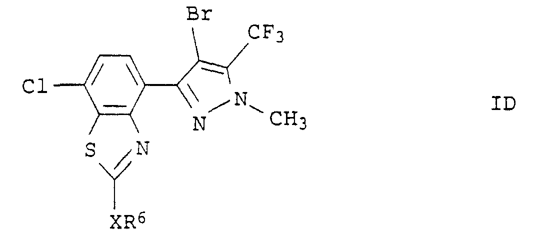
- die Verbindungen IE.001 bis IE.393, die sich von den entsprechenden Verbindungen Ia.001 bis Ia.393 lediglich dadurch unterscheiden, daß R² für Trifluormethyl und R⁴ für Chlor stehen und die Gruppe -N=C(XR⁶)-S- über den Stickstoff an α gebunden ist: 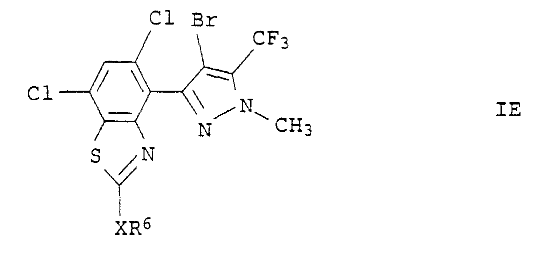
- die Verbindungen IF.001 bis IF.393, die sich von den entsprechenden Verbindungen Ia.001 bis Ia.393 lediglich dadurch unterscheiden, daß R² für Trifluormethyl und R⁴ für Fluor stehen und die Gruppe -N=C(XR⁶)-S- über den Stickstoff an α gebunden ist: 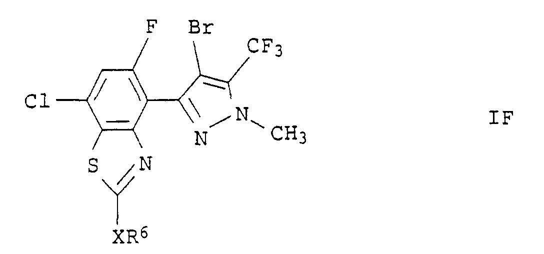
- die Verbindungen IG.001 bis IG.393, die sich von den entsprechenden Verbindungen Ia.001 bis Ia.393 lediglich dadurch unterscheiden, daß R² für Methylsulfonyl steht und die Gruppe -N=C(XR⁶)-S- über den Stickstoff an α gebunden ist: 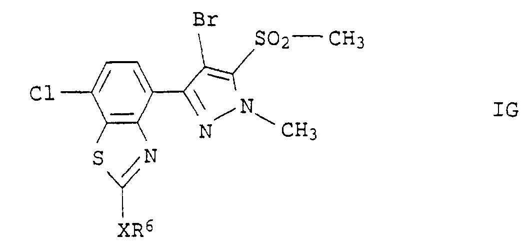
- die Verbindungen IH.001 bis IH.393, die sich von den entsprechenden Verbindungen Ia.001 bis Ia.393 lediglich dadurch unterscheiden, daß R² für Methylsulfonyl und R⁴ für Chlor stehen und die Gruppe -N=C(XR⁶)-S- über den Stickstoff an α gebunden ist: 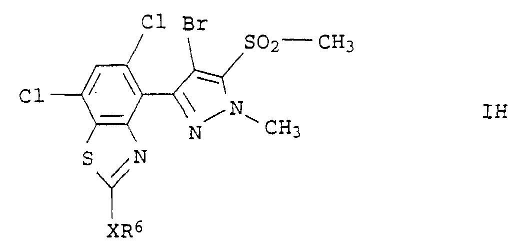
- die Verbindungen IJ.001 bis IJ.393, die sich von den entsprechenden Verbindungen Ia.001 bis Ia.393 lediglich dadurch unterscheiden, daß R² für Methylsulfonyl und R⁴ für Fluor stehen und die Gruppe -N=C(XR⁶)-S- über den Stickstoff an α gebunden ist: 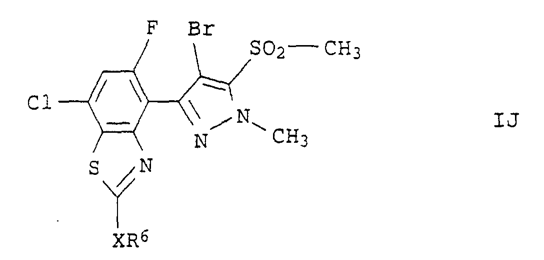
- die Verbindungen IK.001 bis IK.393, die sich von den entsprechenden Verbindungen Ia.001 bis Ia.393 lediglich dadurch unterscheiden, daß Z eine Gruppe -N=C(XR⁶)-O- bedeutet, die über den Stickstoff an α gebunden ist: 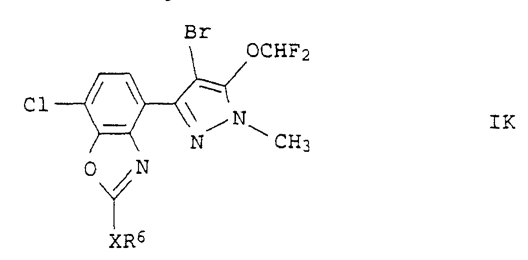
- die Verbindungen IM.001 bis IM.393, die sich von den entsprechenden Verbindungen Ia.001 bis Ia.393 dadurch unterscheiden, daß R⁴ für Chlor steht und Z eine Gruppe -N=C(XR⁶)-Obedeutet, die über den Stickstoff an α gebunden ist: 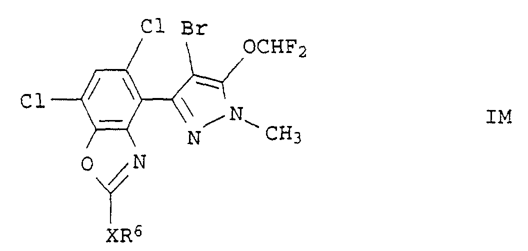
- die Verbindungen IN.001 bis IN.393, die sich von den entsprechenden Verbindungen Ia.001 bis Ia.393 dadurch unterscheiden, daß R⁴ für Fluor steht und Z eine Gruppe -N=C(XR⁶)-Obedeutet, die über den Stickstoff an α gebunden ist: 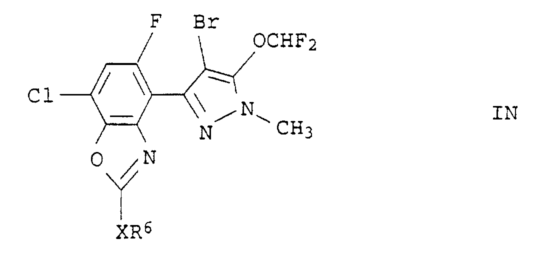
- die Verbindungen IO.001 bis IO.393, die sich von den entsprechenden Verbindungen Ia.001 bis Ia.393 dadurch unterscheiden, daß R² für Trifluormethyl steht und Z eine Gruppe -N=C(XR⁶)-O- bedeutet, die über den Stickstoff an α gebunden ist: 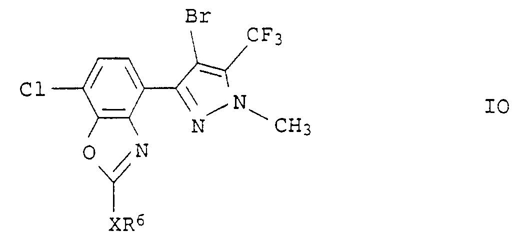
- die Verbindungen IP.001 bis IP.393, die sich von den entsprechenden Verbindungen Ia.001 bis Ia.393 dadurch unterscheiden, daß R² für Trifluormethyl und R⁴ für Chlor stehen und Z eine Gruppe -N=C(XR⁶)-O- bedeutet, die über den Stickstoff an α gebunden ist: 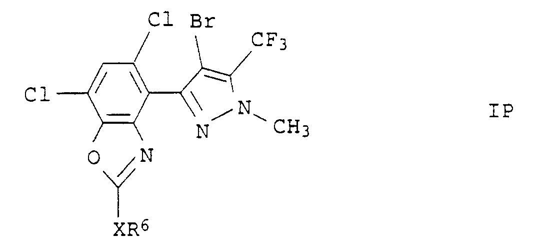
- die Verbindungen IQ.001 bis IQ.393, die sich von den entsprechenden Verbindungen Ia.001 bis Ia.393 dadurch unterscheiden, daß R² für Trifluormethyl und R⁴ für Fluor stehen und Z eine Gruppe -N=C(XR⁶)-O- bedeutet, die über den Stickstoff an α gebunden ist: 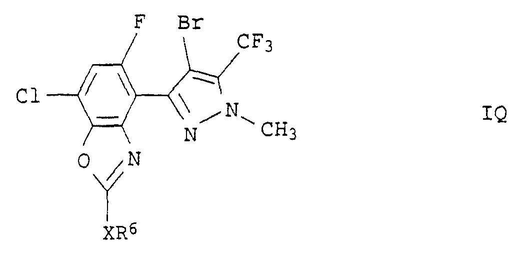
- die Verbindungen IR.001 bis IR.393, die sich von den entsprechenden Verbindungen Ia.001 bis Ia.393 dadurch unterscheiden, daß R² für Methylsulfonyl steht und Z eine Gruppe -N=C(XR⁶)-O- bedeutet, die über den Stickstoff an α gebunden ist: 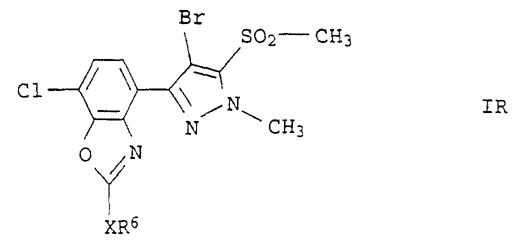
- die Verbindungen IS.001 bis IS.393, die sich von den entsprechenden Verbindungen Ia.001 bis Ia.393 dadurch unterscheiden, daß R² für Methylsulfonyl und R⁴ für Chlor stehen und Z eine Gruppe -N=C(XR⁶)-O- bedeutet, die über den Stickstoff an α gebunden ist: 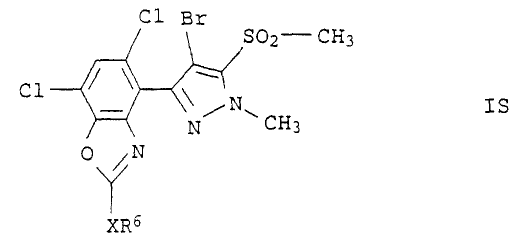
- die Verbindungen IT.001 bis IT.393, die sich von den entsprechenden Verbindungen Ia.001 bis Ia.393 dadurch unterscheiden, daß R² für Methylsulfonyl und R⁴ für Fluor stehen und Z eine Gruppe -N=C(XR⁶)-O- bedeutet, die über den Stickstoff an α gebunden ist:

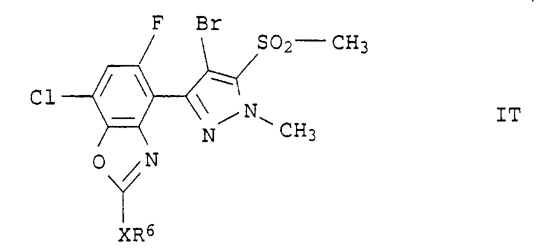

Die substituierten (4-Brompyrazol-3-yl)benzazole der Formel I sind auf verschiedene Weise erhältlich, insbesondere nach einem der folgenden Verfahren:
A) Umsetzung eines Aminophenylpyrazols der Formel IIIa oder IIIb mit einem Halogen und Ammoniumthiocyanat oder mit einem Alkalioder Erdalkalimetallthiocyanat: 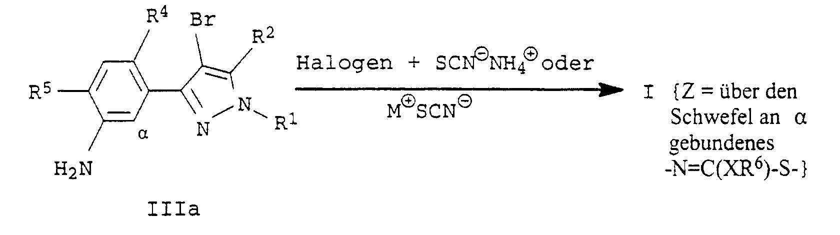 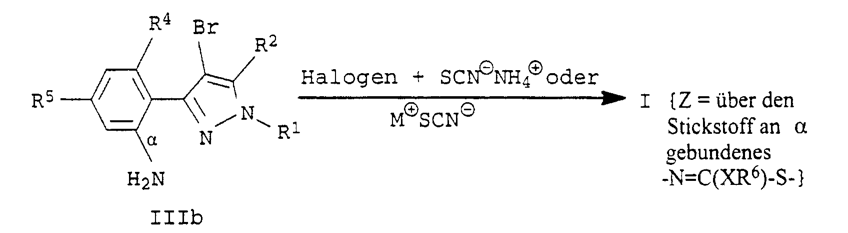 M^{⊕} = Alkali- oder 1/2 Erdalkalimetallion
Bevorzugtes Halogen ist Chlor oder Brom; unter den Alkali-/Erdalkalimetallthiocyanaten ist Natriumthiocyanat bevorzugt.
In der Regel arbeitet man in einem inerten Lösungs-/Verdünnungsmittel, z.B. in einem Kohlenwasserstoff wie Toluol und Hexan, in einem halogenierten Kohlenwasserstoff wie Dichlormethan, in einem Ether wie Tetrahydrofuran, in einem Alkohol wie Ethanol, in einer Carbonsäure wie Essigsäure, oder in einem aprotischen Solvens wie Dimethylformamid, Acetonitril und Dimethylsulfoxid.
Die Reaktionstemperatur liegt normalerweise zwischen Schmelzund Siedepunkt des Reaktionsgemisches, vorzugsweise bei 0 bis 150°C.
Um eine möglichst hohe Ausbeute an Wertprodukt zu erzielen verwendet man Halogen und Ammoniumthiocyanat bzw. Alkali-/Erdalkalimetallthiocyanat in etwa äquimolarer Menge oder im Überschuß, bis etwa zur 5fachen molaren Menge, bezogen auf die Menge an IIIa oder IIIb.
Eine Variante des Verfahrens besteht darin, das Aminophenylpyrazol IIIa oder IIIb zunächst mit Ammoniumthiocyanat oder einem Alkali- oder Erdalkalimetallthiocyanat zu einem Thioharnstoff IVa oder IVb 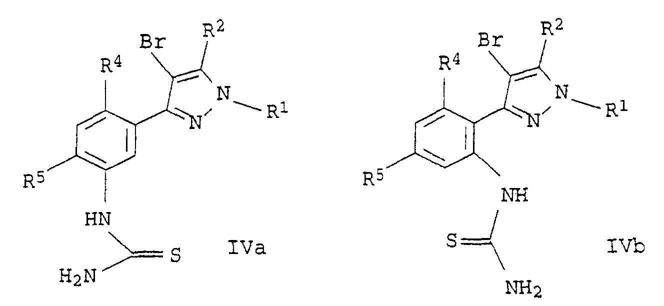 umzusetzen, und IVa oder IVb durch Behandlung mit Halogen anschließend in I mit Z = -N=C(XR⁶)-S- zu überführen.
B) Diazotierung eines Aminophenylpyrazols der Formel IIIa oder IIIb, Überführung des jeweiligen Diazoniumsalzes in ein Azidophenylpyrazol der Formel Va oder Vb und dessen Umsetzung entweder
B.1) mit einer Carbonsäure oder
B.2) zunächst mit einer Sulfonsäure (zu VIa oder VIb), Hydrolyse des gebildeten Sulfonats zum Aminophenol VIIa oder VIIb, und deren Überführung in I: 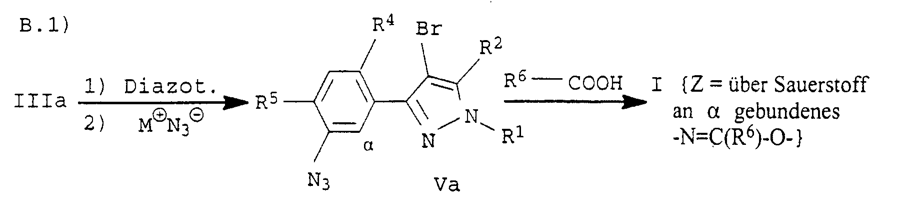 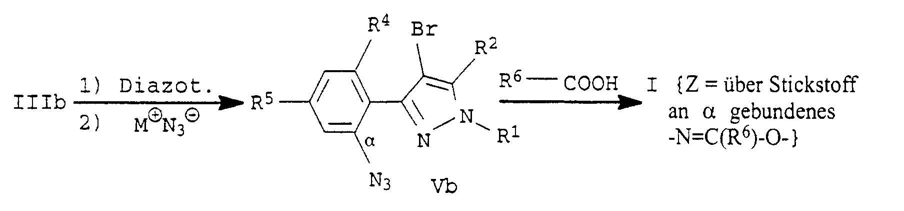 M^{⊕} steht für eine Alkalimetallion oder 1/2 Erdalkalimetallion. 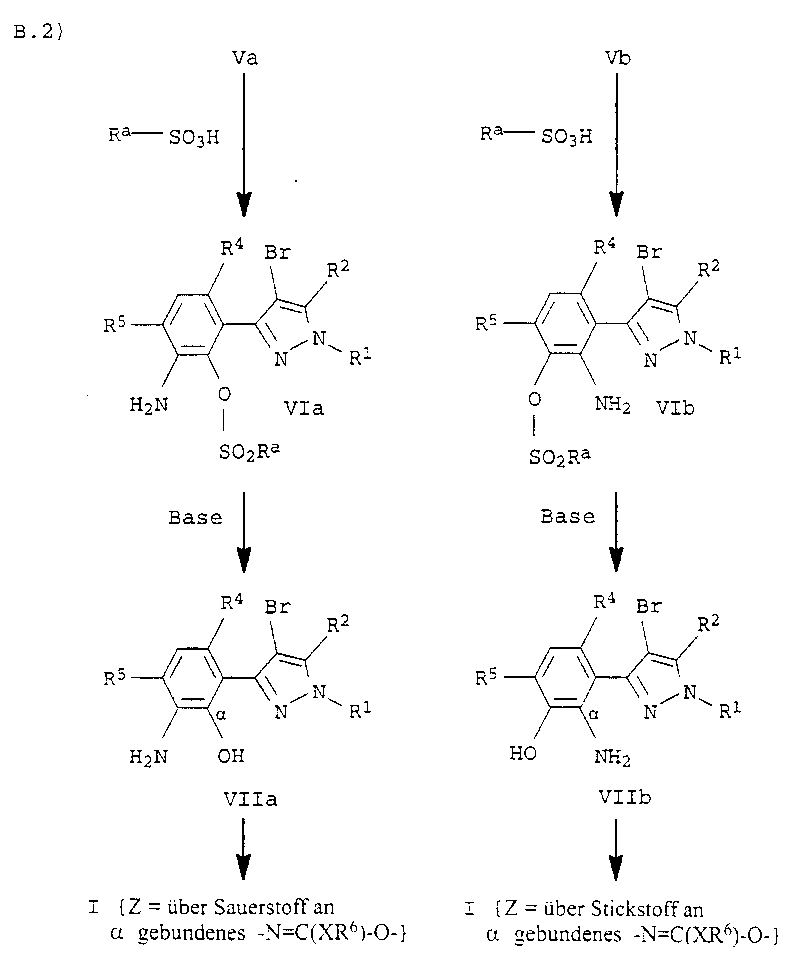
Für die Durchführung der Diazotierung gelten die bei Verfahren C) gemachten Angaben. Die Überführung in die Arylazide Va/Vb erfolgt vorzugsweise durch Umsetzung von IIIa/IIIb mit einem Alkali- oder Erdalkalimetallazid wie Natriumazid oder durch Umsetzung mit Trimethylsilylazid.
Bei der unter B.1) genannten Umsetzung mit einer Carbonsäure arbeitet man entweder in einem inerten Lösungsmittel, z.B. einem Ether wie Tetrahydrofuran und Dioxan, einem aprotischen Solvens wie Dimethylformamid und Acetonitril, einem Kohlenwasserstoff wie Toluol und Hexan, einem halogenierten Kohlenwasserstoff wie Dichlormethan, oder lösungsmittelfrei in einem Überschuß an Carbonsäure R⁶-COOH. Im letzteren Fall kann der Zusatz einer Mineralsäure wie Phosphorsäure hilfreich sein.
Die Umsetzung wird vorzugsweise bei erhöhter Temperatur, beispielsweise bei der Siedetemperatur des Reaktionsgemisches, vorgenommen.
Für die unter B.2) zunächst genannte Umsetzung von Va/Vb mit einer Sulfonsäure R^{a}-SO₃H (wobei R^{a} für C₁-C₄-Alkyl- oder C₁-C₄-Halogenalkyl, vorzugsweise Methyl oder Trifluormethyl, steht) gelten die vorstehend für die Umsetzung von Va/Vb mit R⁶-COOH gemachten Angaben.
Die anschließende Hydrolyse der Sulfonate VIa/VIb erfolgt vorzugsweise durch Umsetzung mit einer wäßrigen Base wie Natron- und Kalilauge, wobei gewünschtenfalls ein Lösungsmittel, z.B. ein Ether wie Dioxan und Tetrahydrofuran, oder ein Alkohol wie Methanol und Ethanol, zugesetzt werden kann.
Die abschließende Umsetzung zu I ist an sich bekannt und kann auf außerordentlich vielfältige Weise erfolgen. Hierzu sei auf die Angaben in Houben-Weyl, Methoden der Organischen Chemie, Georg Thieme Verlag Stuttgart, Bd E8a 1993, S. 1032 ff., verwiesen.
Die Azidophenylpyrazole der Formeln Va und Vb sind neu. Besonders bevorzugte Verbindungen der Formel Va sind die in der folgenden Tabelle 2 genannten Verbindungen Va.1 - Va.9:

| Nr. | R¹ | R² | R⁴ | R⁵ |
|---|---|---|---|---|
| Va.1 | CH₃ | OCHF₂ | H | Cl |
| Va.2 | CH₃ | OCHF₂ | Cl | Cl |
| Va.3 | CH₃ | OCHF₂ | F | Cl |
| Va.4 | CH₃ | CF₃ | H | Cl |
| Va.5 | CH₃ | CF₃ | Cl | Cl |
| Va.6 | CH₃ | CF₃ | F | Cl |
| Va.7 | CH₃ | SO₂-CH₃ | H | Cl |
| Va.8 | CH₃ | SO₂-CH₃ | Cl | Cl |
| Va.9 | CH₃ | SO₂-CH₃ | F | Cl |

C) Diazotierung von substituierten (4-Brompyrazol-3-yl)benzazolen der Formel I, bei denen XR⁶ für Amino steht, und anschließender Überführung des Diazoniumsalzes in Verbindungen I mit
- -XR⁶ = Cyano oder Halogen {zur Sandmeyer-Reaktion vgl. beispielsweise Houben-Weyl, Methoden der Organischen Chemie, Georg Thieme Verlag Stuttgart, Bd. 5/4, 4. Auflage 1960, S. 438ff.},
- -X- = Schwefel {vgl. hierzu beispielsweise Houben-weyl, Methoden der Organischen Chemie, Georg Thieme Verlag Stuttgart, Bd. E11 1984, S. 43 und 176},
- -XR⁶ = z.B. -CH₂-CH(Halogen)-R⁸, -CH=CH-R⁸, -CH=C(Halogen)-R⁸ {allgemein handelt es sich hierbei um Produkte einer Meerwein-Arylierung; vgl. hierzu beispielsweise C.S. Rondestredt, Org. React. 11, 189 (1960) und H.P. Doyle et al., J. Org. Chem. 42, 2431 (1977)}: 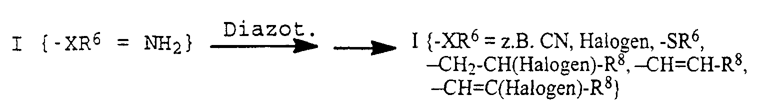
   Im allgemeinen erhält man das Diazoniumsalz auf an sich bekannte Weise durch Umsetzung von I mit -XR⁶ = Amino in einer wäßrigen Säurelösung, z.B. in Salzsäure, Bromwasserstoffsäure oder Schwefelsäure, mit einem Nitrit wie Natriumnitrit und Kaliumnitrit.
   Es besteht aber auch die Möglichkeit, wasserfrei, z.B. in Chlorwasserstoff haltigem Eisessig, in absolutem Alkohol, in Dioxan oder Tetrahydrofuran, in Acetonitril oder in Aceton zu arbeiten und hierbei die Ausgangsverbindung (I mit -XR⁶ = NH₂) mit einem Salpetrigsäureester wie tert.-Butylnitrit und Isopentylnitrit zu behandeln.
   Die Überführung des so erhaltenen Diazoniumsalzes in die entsprechende Verbindung I mit -XR⁶ = Cyano, Chlor, Brom oder Iod erfolgt besonders bevorzugt durch Behandeln mit einer Lösung oder Suspension eines Kupfer(I)salzes wie Kupfer(I)cyanid, -chlorid, -bromid und iodid, oder mit einer Alkalimetallsalz-Lösung.
   Verbindungen I mit -X- = Schwefel erhält man normalerweise durch Umsetzung des Diazoniumsalzes mit einem Dialkyldisulfid wie Dimethyldisulfid und Diethyldisulfid, oder mit z.B. Diallyldisulfid oder Dibenzyldisulfid.
   Bei der Meerwein-Arylierung handelt es sich üblicherweise um die Umsetzung der Diazoniumsalze mit Alkenen (hier H₂C=CH-R⁸) oder Alkinen (hier HC≡C-R⁸). Das Alken oder Alkin wird dabei vorzugsweise im Überschuß, bis etwa 3000 mol-%, bezogen auf die Menge des Diazoniumsalzes, eingesetzt.
   Die vorstehend beschriebenen Umsetzungen des Diazoniumsalzes können z.B. in Wasser, in wässriger Salzsäure oder Bromwasserstoffsäure, in einem Keton wie Aceton, Diethylketon und Methylethylketon, in einem Nitril wie Acetonitril, in einem Ether wie Dioxan und Tetrahydrofuran oder in einem Alkohol wie Methanol und Ethanol erfolgen.
   Sofern nicht bei den einzelnen Umsetzungen anders angegeben liegen die Reaktionstemperaturen normalerweise bei (-30) bis +50°C.
   Bevorzugt werden alle Reaktionspartner in etwa stöchiometrischen Mengen eingesetzt, jedoch kann auch ein Überschuß der einen oder anderen Komponente, bis etwa 3000 mol-%, von Vorteil sein.

D) Oxidation eines substituierten (4-Brompyrazol-3-yl)benzazols I, bei dem X für Schwefel steht, zu I mit X = -SO- auf an sich bekannte Weise (vgl. z.B. Houben-Weyl, Methoden der Organischen Chemie, Georg Thieme Verlag Stuttgart, Bd. E 11/1, 1985, S. 702 ff., Bd. IX, 4. Auflage, 1955, S. 211): 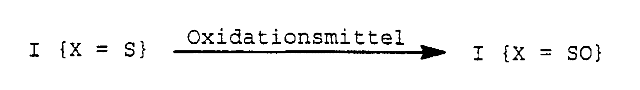
Geeignete Oxidationsmittel sind z.B. Wasserstoffperoxid, organische Peroxide wie Essigsäureperoxid, Trifluoressigsäureperoxid, m-Chlorperbenzoesäure, tert.-Butylhydroperoxid und tert.-Butylhypochlorit, sowie anorganische Verbindungen wie Natriummetaiodat, Chromsäure und Salpetersäure.
Üblicherweise arbeitet man - je nach Oxidationsmittel - in einer organischen Säure wie Essigsäure und Trichloressigsäure, in einem chlorierten Kohlenwasserstoff wie Methylenchlorid, Chloroform und 1,2-Dichlorethan, in einem aromatischen Kohlenwasserstoff wie Benzol, Chlorbenzol und Toluol oder in einem protischen Lösungsmittel wie Methanol und Ethanol. Auch Gemische der genannten Solventien kommen in Betracht.
Die Reaktionstemperatur liegt im allgemeinen bei (-30)°C bis zur Siedetemperatur des jeweiligen Reaktionsgemisches, wobei normalerweise der untere Temperaturbereich bevorzugt ist.
Zweckmäßigerweise setzt man Ausgangsverbindung und Oxidationsmittel in etwa stöchiometrischem Verhältnis ein, jedoch kann auch die eine oder andere Komponente im Überschuß eingesetzt werden.
E) Oxidation eines substituierten (4-Brompyrazol-3-yl)benzazols I, bei dem X für Schwefel oder -SO- steht, zu I mit X = -SO₂- auf an sich bekannte Weise (vgl. z.B. Houben-Weyl, Methoden der Organischen Chemie, Georg Thieme Verlag Stuttgart, Bd. E 11/2, 1985, S. 1132 ff. und Bd. IX, 4. Auflage, 1955, S. 222 ff.): 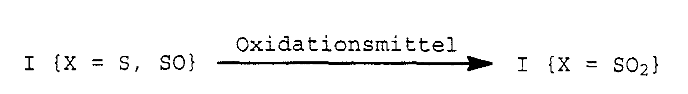
Als Oxidationsmittel eignen sich beispielsweise Wasserstoffperoxid, organische Peroxide wie Essigsäureperoxid, Trifluoressigsäureperoxid und m-Chlorperbenzoesäure, ferner anorganische Oxidationsmittel wie Kaliumpermanganat. Die Anwesenheit eines Katalysators, z.B. Wolframat, kann sich förderlich auf den Reaktionsverlauf auswirken.
In der Regel nimmt man die Umsetzung in einem inerten Lösungsmittel vor, wobei je nach Oxidationsmittel z.B. organische Säuren wie Essigsäure und Propionsäure, chlorierte Kohlenwasserstoffe wie Methylenchlorid, Chloroform und 1,2-Dichlorethan, aromatische Kohlenwasserstoffe oder Halogenkohlenwasserstoffe wie Benzol, Chlorbenzol und Toluol, oder Wasser brauchbar sind. Auch Mischungen der genannten Lösungsmittel kommen in Betracht.
Normalerweise arbeitet man bei (-30)°C bis zur Siedetemperatur des jeweiligen Reaktionsgemisches, vorzugsweise bei 10°C bis zur Siedetemperatur.
Zweckmäßigerweise werden die Ausgangsverbindung I mit X = Schwefel oder SO und das Oxidationsmittel in etwa stöchiometrischen Mengen eingesetzt. Zur Optimierung des Umsatzes an Ausgangsverbindung kann aber ein Überschuß an Oxidationsmittel empfehlenswert sein.
F) Umsetzung eines substituierten (4-Brompyrazol-3-yl)benzazols I, bei dem die Gruppierung -XR⁶ für Chlor, Brom, Alkylsulfonyl oder Halogenalkylsulfonyl steht, in Gegenwart einer Base mit einem Alkohol, Mercaptan, Amin oder einer CH-aciden Verbindung (VIII): 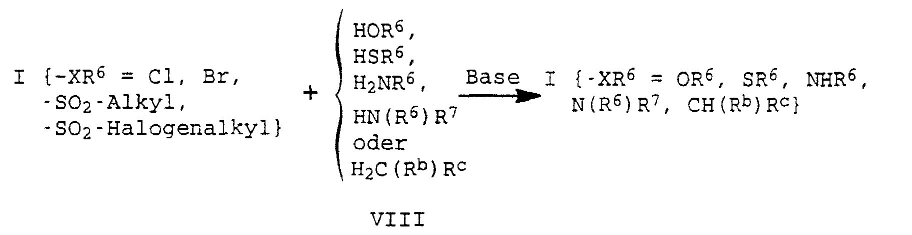 R^{b} und R^{c} stehen unabhängig voneinander für Cyano oder (C₁-C₄-Alkoxy)carbonyl.
Zweckmäßigerweise nimmt man die Umsetzung in einem inerten Lösungsmittel vor, beispielsweise in einem Ether wie Diethylether, Methyl-tert.-butylether, Dimethoxyethan, Diethylenglycoldimethylether, Tetrahydrofuran und Dioxan, einem Keton wie Aceton, Diethylketon, Ethylmethylketon und Cyclohexanon, einem dipolaren aprotischen Lösungsmittel wie Acetonitril, Dimethylformamid, N-Methylpyrrolidon und Dimethylsulfoxid, einem protischen Lösungsmittel wie Methanol und Ethanol, einem aromatischen, gewünschtenfalls halogenierten Kohlenwasserstoff wie Benzol, Chlorbenzol und 1,2-Dichlorbenzol, einem heteroaromatischen Lösungsmittel wie Pyridin und Chinolin oder in einem Gemisch solcher Lösungsmittel. Tetrahydrofuran, Aceton, Diethylketon und Dimethylformamid sind bevorzugt.
Als Basen können hierbei z.B. die Hydroxide, Hydride, Alkoxide, Carbonate oder Hydrogencarbonate von Alkalimetall- und Erdalkalimetallkationen, tertiäre aliphatische Amine wie Triethylamin, N-Methylmorpholin und N-Ethyl-N,N-diisopropyl-amin, bi- und tricyclische Amine wie Diazabicycloundecan (DBU) und Diazabicyclooctan (DABCO), oder aromatische Stickstoffbasen wie Pyridin, 4-Dimethylaminopyridin und Chinolin, dienen. Auch Kombinationen verschiedener Basen kommen in Betracht. Bevorzugte Basen sind Natriumhydrid, Natriumhydroxid, Natriumcarbonat, Kaliumcarbonat, Natriummethylat, Natriumethylat und Kaliumtert.-butylat.
Die Amine H₂NR⁶ oder HN(R⁶)R⁷ können als Reaktionspartner und gleichzeitig als Base dienen, wobei dann das Amin in mindestens doppeltem Überschuß, bezogen auf die Menge an Ausgangsverbindung I, vorliegen sollte. Selbstverständlich ist auch ein größerer Überschuß an Amin möglich, bis etwa zur 10fachen molaren Menge, bezogen auf die Menge an I mit -XR⁶ = Cl, Br, -SO₂-Alkyl oder -SO₂-Halogenalkyl.
Üblicherweise setzt man die Ausgangsstoffe in etwa stöchiometrischen Mengen ein, jedoch kann auch ein Überschuß der einen oder anderen Komponente im Hinblick auf die Verfahrensführung oder eine möglichst vollständige Umsetzung der Ausgangsverbindung I {-XR⁶ = Cl, Br, -SO₂-Alkyl, -SO₂-Halogenalkyl} vorteilhaft sein.
Das molare Verhältnis von Alkohol, Mercaptan, Amin oder CH-acider Verbindung (VIII) zu Base beträgt im allgemeinen 1:1 bis 1:3.
Die Konzentration der Ausgangsstoffe im Lösungsmittel liegt normalerweise bei 0,1 bis 5,0 mol/l.
Die Umsetzung kann bei Temperaturen von 0°C bis zur Rückflußtemperatur des jeweiligen Reaktionsgemisches durchgeführt werden.
G) Umsetzung eines substituierten (4-Brompyrazol-3-yl)benzazols I, bei dem -XR⁶ für Halogen steht, mit einer (C₁-C₆-Alkyl)-Grignard-Verbindung: 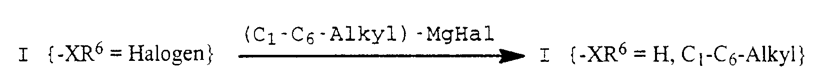 Hal steht dabei für Chlorid oder Bromid.
In der Regel arbeitet man in einem inerten Lösungs-/Verdünnungsmittel, beispielsweise einem Kohlenwasserstoff wie Hexan und Toluol, oder einem Ether wie Diethylether, Tetrahydrofuran und Dioxan.
Gewünschtenfalls kann dabei ein Übergangsmetallkatalysator, in Mengen von 0,0001 bis 10 mol-%, zugesetzt werden. Hierfür kommen beispielsweise Nickel- und Palladiumkatalysatoren wie Nickeldichlorid, Bis(triphenylphosphin)nickeldichlorid, [Bis-(1,2-diphenylphosphino)ethan]nickeldichlorid, [Bis-(1,3-diphenylphosphino)propan]nickeldichlorid, Palladiumdichlorid, Tetrakis(triphenylphosphin)palladium, Bis(triphenylphosphin)palladiumdichlorid, [Bis-(1,2-diphenylphosphino)-ethan]palladiumdichlorid, [Bis-(1,3-diphenylphosphino)propan]-palladiumdichlorid und [Bis(diphenylphospino)ferrocen]-palladiumdichlorid, aber auch Mischungen von Palladiumoder Nickeldichlorid und Phosphinen wie Triphenylphosphin, Bis-1,2-(Diphenylphosphino)ethan und Bis-1,3-(Diphenylphosphino)propan in Betracht.
Je nach Reaktionsführung entstehen dabei Verbindungen I mit -XR⁶ = Wasserstoff oder C₁-C₆-Alkyl oder entsprechende Mischungen aus alkylierter und nicht-alkylierter Verbindung I, die jedoch auf übliche Weise getrennt werden können.
In der Regel arbeitet man bei (-100)°C bis zur Siedetemperatur des Reaktionsgemisches.
Die Menge an Grignard-Reagenz ist nicht kritisch; normalerweise verwendet man (C₁-C₆-Alkyl)-MgHal in ca. äquimolarer Menge oder im Überschuß, bis etwa zur 10fachen molaren Menge, bezogen auf die Menge an I mit -XR⁶ = Halogen.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel auf an sich bekannte Weise. Sofern nicht bei den vorstehend beschriebenen Verfahren etwas anderes angegeben ist, erhält man die Wertprodukte z.B. nach Verdünnen der Reaktionslösung mit Wasser durch Filtration, Kristallisation oder Lösungsmittelextraktion, oder durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten (4-Brompyrazol-3-yl)benzazole I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die weitgehend reinen Isomeren getrennt werden können. Reine optisch aktive Isomere lassen sich vorteilhaft aus entsprechenden optisch aktiven Ausgangsprodukten herstellen.

Landwirtschaftlich brauchbare Salze der Verbindungen I können durch Reaktion mit einer Base des entsprechenden Kations, vorzugsweise einem Alkalimetallhydroxid oder -hydrid, oder durch Reaktion mit einer Säure des entsprechenden Anions, vorzugsweise der Chlorwasserstoffsäure, Bromwasserstoffsäure, Schwefelsäure, Phosphorsäure oder Salpetersäure, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwendet werden.

Des weiteren eignen sich die substituierten (4-Brompyrazol-3-yl)benzazole I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen z.B. die Alkali-, Erdalkali-, Ammoniumsalze von aromatischen Sulfonsäuren wie Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.
Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit und Diatomeenerde, Calcium- und Magnesiumsulfat, Magnesiumoxid, gemahlene Kunststoffe, Düngemittel wie Ammoniumsulfat, Ammoniumphosphat und Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen etwa 0,001 bis 98 Gew.-%, vorzugsweise 0,01 bis 95 Gew.-%, mindestens eines Wirkstoffs. Die Wirkstoffe I werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. In.003 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. Im.003 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. Ik.003 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. It.003 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
V. 3 Gewichtsteile des Wirkstoffs Nr. In.003 werden mit 97 Gewichtsteilen feinteiligem Kaolin vermischt. Man erhält auf diese Weise ein Stäubemittel, das 3 Gew. -% des Wirkstoffs enthält.
VI. 20 Gewichtsteile des Wirkstoffs Nr. Im.003 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. Ik.003 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Anschließend kann die Mischung mit Wasser auf die gewünschte Wirkstoffkonzentration verdünnt werden. Man erhält ein stabiles Emulsionskonzentrat.
VIII. 1 Gewichtsteil der Verbindung Nr. It.003 wird in einer Mischung gelöst, die aus 80 Gewichtsteilen Cyclohexan und 20 Gewichtsteilen Wettol® EM 31 (= nichtionischer Emulgator auf der Basis von ethoxyliertem Rizinusöl; BASF AG) besteht. Danach kann mit Wasser auf die gewünschte Wirkstoffkonzentration verdünnt werden. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha, aktive Substanz (a.S.).

Zur Verbreiterung des Wirkungsspektrums und zur Erzielung synergistischer Effekte können die substituierten (4-Brompyrazol-3-yl)benzazole I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### 7-(4-Brom-5-difluormethoxy-1-methyl-1H-pyrazol-3-yl)-4-chlor-2-ethyl-6-fluorbenzoxazol (Verbindung Nr. In.003)

Eine Lösung von 12,9 g (33 mmol) 5-(4-Brom-5-difluormethoxy-1-methyl-lH-pyrazol-3-yl)-2-chlor-4-fluorphenylazid in 300 ml Propionsäure wurde 4 Std. auf Rückflußtemperatur erhitzt. Anschließend engte man ein. Nach Lösen des Rückstandes in Ethylacetat wurde die organische Phase mit verdünnter wässriger Natriumhydrogencarbonat-Lösung gewaschen, dann über Magnesiumsulfat getrocknet und schließlich eingeengt. Die Reinigung des Rohproduktes erfolgte mittels Kieselgelchromatographie (Eluens: Hexan/Ethylacetat = 9:1). Ausbeute: 3,3 g. MS [m/z]: 423 [M⁺]. ¹H-NMR (400 MHz, in CDCl₃) : δ [ppm] = 1,45 (t, 3H), 2,98 (q, 2H), 3,92 (s, 3H), 6,74 (t, 1H), 7,24 (d, 1H).

### Vorstufe 1.1

### 4-Brom-3-(4-chlor-2-fluor-5-nitrophenyl)-5-difluormethoxy-1-methyl-1H-pyrazol

Zu 16,6 g (0,26 mol) konz. Salpetersäure wurden bei (-30)°C 15,5 g (0,16 mol) konz. Schwefelsäure gegeben. Nach Kühlen auf (-40)°C versetzte man die Mischung tropfenweise mit einer Lösung von 18,7 g (53 mmol) 4-Brom-3-(4-chlor-2-fluorphenyl)-5-difluormethoxy-1-methyl-1H-pyrazol in 100 ml Dichlormethan. Anschließend wurde das Reaktionsgemisch 4 Std. gerührt, wonach man auf Eiswasser goß. Die organische Phase wurde abgetrennt, mit verdünnter wässriger Natriumhydrogencarbonat-Lösung und Wasser gewaschen, über Magnesiumsulfat getrocknet und schließlich eingeengt. Ausbeute: 15,6 g. ¹H-NMR (270 MHz, in CDCl₃): δ [ppm] = 3,88 (s, 3H), 6,72 (t, 1H), 7,41 (d, 1H), 8,24 (d, 1H).

### Vorstufe 1.2

### 5-(4-Brom-5-difluormethoxy-1-methyl-1H-pyrazol-3-yl)-2-chlor-4-fluoranilin

Zu einer Lösung von 15,6 g (39 mmol) 4-Brom-3-(4-chlor-2-fluor-5-nitrophenyl)-5-difluormethoxy-1-methyl-1H-pyrazol in 150 ml Tetrahydrofuran wurden 5 g Raney-Nickel gegeben, wonach man unter leichtem Wasserstoffüberdruck bis zur Aufnahme der berechneten Menge Wasserstoff hydrierte. Anschließend wurde der Katalysator durch ein Kieselgurbett abfiltriert. Das Filtrat engte man ein. Ausbeute: quantitativ. ¹H-NMR (400 MHz, in CDCl₃): δ [ppm] = 3,85 (s, 3H), 3,97 (s, 2H), 6,70 (t, 1H), 6,89 (d, 1H), 7,12 (d, 1H).

### Vorstufe 1.3

### 5-(4-Brom-5-difluormethoxy-1-methyl-lH-pyrazol-3-yl)-2-chlor-4-fluorphenylazid (Nr. Va.3)

Zu einer Lösung von 14,4 g (39 mmol) 5-(4-Brom-5-difluormethoxy-1-methyl-1H-pyrazol-3-yl)-2-chlor-4-fluoranilin in 60 ml Trifluoressigsäure wurden bei 10-15°C zunächst 6,0 g (59 mmol) t-Butylnitrit und dann portionsweise 3,8 g (59 mmol) Natriumazid gegeben. Anschließend rührte man noch eine Stunde bei ca. 20°C, bevor die Reaktionslösung auf Eis gegossen wurde. Aus der wäßrigen Phase extrahierte man das Produkt mit Methyl-t-butylether.

Danach wurde die organische Phase mit 10 %iger wässriger Natronlauge gewaschen, über Magnesiumsulfat getrocknet und schließlich eingeengt, wobei darauf geachtet wurde, daß konzentrierte Lösungen oder die reine Substanz nicht über 40°C erhitzt wurden. Ausbeute: 12,9 g. MS [m/z]: 395 [M⁺]. ¹H-NMR (270 MHz, in CDCl₃) : δ [ppm] = 3,88 (s, 3H), 6,71 (t, 1H), 7,25 (d, 1H), 7,33 (d, 1H).

### Beispiel 2

### 7-(4-Brom-5-difluormethoxy-1-methyl-1H-pyrazol-3-yl)-4,6-dichlor-2-ethylbenzoxazol (Verbindung Nr. Im.003)

Diese Verbindung wurde analog Beispiel 1 hergestellt. ¹H-NMR (270 MHz, in CDCl₃): δ [ppm] = 1,43 (t, 3H), 2,97 (q, 2H), 3,92 (s, 3H), 6,75 (t, 1H), 7,49 (s, 1H).

### Vorstufe 2.1

### 4-Brom-3-(2,4-dichlor-5-nitrophenyl)-5-difluormethoxy-1-methyl-1H-pyrazol

Diese Verbindung wurde analog Vorstufe 1.1 hergestellt. ¹H-NMR (270 MHz, in CDCl₃) : δ [ppm] = 3,88 (s, 3H), 6,72 (t, 1H), 7,71 (s, 1H), 8,04 (s, 1H).

### Vorstufe 2.2

### 5-(4-Brom-5-difluormethoxy-1-methyl-1H-pyrazol-3-yl)-2,4-dichloranilin

Diese Verbindung wurde analog Vorstufe 1.2 hergestellt. ¹H-NMR (270 MHz, in CDCl₃) : δ [ppm] = 3,84 (s, 3H), 4,12 (s, 2H), 6,72 (t, 1H), 6,79 (s, 1H), 7,38 (s, 1H).

### Vorstufe 2.3

### 5-(4-Brom-5-difluormethoxy-1-methyl-1H-pyrazol-3-yl)-2,4-dichlorphenylazid (Nr. Va.2)

Diese Verbindung wurde analog Vorstufe 1.3 hergestellt. MS [m/z]: 411 [M⁺]. ¹H-NMR (270 MHz, in CDCl₃) : δ [ppm] = 3,87 (s, 3H), 6,72 (t, 1H), 7,21 (s, 1H), 7,52 (s, 1H).

### Beispiel 3

### 7-(4-Brom-5-difluormethoxy-1-methyl-lH-pyrazol-3-yl)-4-chlor-2-ethylbenzoxazol (Verbindung Nr. Ik.003)

Diese Verbindung wurde analog Beispiel 1 hergestellt. ¹H-NMR (270 MHz, in CDCl₃) : δ [ppm] = 1,48 (t, 3H), 3,03 (q, 2H), 3,92 (s, 3H), 6,74 (t, 1H), 7,40 (d, 1H), 7,55 (d, 1H).

### Vorstufe 3.1

### 4-Brom-3-(4-chlorphenyl)-5-difluormethoxy-1-methyl-1H-pyrazol

Zu einer Lösung von 10,1 g (39 mmol) 3-(4-Chlorphenyl)-5-difluormethoxy-1-methyl-1H-pyrazol in 100 ml Tetrachlormethan wurden 7,9 g (43 mmol) Brom gegeben, wonach man 16 Std. rührte. Danach wurde die Lösung mit ges. wässriger Natriumhydrogencarbonat-Lösung gewaschen, über Magnesiumsulfat getrocknet und schließlich eingeengt. Ausbeute: 12,9 g. ¹H-NMR (270 MHz, in CDCl₃) : δ [ppm] = 3,84 (s, 3H), 6,69 (t, 1H), 7,40 (d, 2H), 7,81 (d, 2H).

### Vorstufe 3.2

### 4-Brom-3-(4-chlor-3-nitrophenyl)-5-difluormethoxy-1-methyl-1H-pyrazol

Diese Verbindung wurde analog Vorstufe 1.1 hergestellt. ¹H-NMR (270 MHz, in CDCl₃) : δ [ppm] = 3,86 (s, 3H), 6,70 (t, 1H), 7,40 (d, 1H), 8,08 (dd, 1H), 8,46 (d, 1H).

### Vorstufe 3.3

### 5-(4-Brom-5-difluormethoxy-1-methyl-1H-pyrazol-3-yl)-2-chloranilin

Zu einer auf 70-75°C erwärmten Suspension von 10,2 g (0,18 mol) Eisenpulver in 30 ml Essigsäure und 50 ml Ethanol wurden portionsweise 11,7 g (35 mmol) 4-Brom-3-(4-chlor-3-nitrophenyl)-5-difluormethoxy-1-methyl-1H-pyrazol gegeben. Anschließend erhitzte man 2 Std. auf Rückflußtemperatur. Nach dem Abkühlen wurde Ethylacetat zugesetzt, bevor man die Lösung über ein Kieselgurbett filtrierte. Das Filtrat wurde noch mit gesättigter wässriger Natriumhydrogencarbonat-Lösung und Wasser gewaschen, dann über Magnesiumsulfat getrocknet und schließlich eingeengt. Ausbeute: quantitativ. ¹H-NMR (400 MHz, in CDCl₃) : δ [ppm] = 3,81 (s, 3H), 4,12 (s, 2H), 6,68 (t, 1H), 7,19 (dd, 1H), 7,26 (d, 1H), 7,29 (d, 1H).

### Vorstufe 3.4

### 5-(4-Brom-5-difluormethoxy-1-methyl-1H-pyrazol-3-yl)-2-chlorphenylazid (Nr. Va.1)

Diese Verbindung wurde analog Vorstufe 1.3 hergestellt.

### Beispiel 4

### 7-(4-Brom-1-methyl-5-methylsulfonyl-1H-pyrazol-3-yl)-4-chlor-2-ethyl-6-fluorbenzoxazol (Nr. It.003)

Diese Verbindung wurde analog Beispiel 1 hergestellt. ¹H-NMR (400 MHz, in CDCl₃): δ [ppm] = 1,45 (t, 3H), 2,98 (q, 2H), 3,36 (s, 3H), 4,32 (s, 3H), 7,27 (d, 1H).

### Vorstufe 4.1

### 4-Brom-3-(4-chlor-2-fluorphenyl)-1-methyl-5-methylthio-1H-pyrazol

Zu einer Lösung von 20 g (78 mmol) 3-(4-Chlor-2-fluorphenyl)-1-methyl-5-methylthio-1H-pyrazol in 400 ml Tetrachlormethan wurden tropfenweise 13,7 g (86 mmol) Brom gegeben, wonach man 3 Tage rührte. Anschließend wurde die Reaktionslösung mit gesättigter wässriger Natriumhydrogencarbonat-Lösung und Wasser gewaschen, noch über Magnesiumsulfat getrocknet und schließlich eingeengt. Die Reinigung des Rohproduktes erfolgte mittels Kieselgelchromatographie (Eluens: Hexan/Ethylacetat = 9:1). Ausbeute: 22,4 g. ¹H-NMR (270 MHz, in CDCl₃): δ [ppm] = 2,40 (s, 3H), 4,06 (s, 3H), 7,15-7,25 (m, 2H), 7,48 (t, 1H).

### Vorstufe 4.2

### 4-Brom-3-(4-chlor-2-fluorphenyl)-1-methyl-5-methylsulfonyl-1H-pyrazol

Zu einer Lösung von 22,4 g (67 mmol) 4-Brom-3-(4-chlor-2-fluorphenyl)-1-methyl-5-methylthio-1H-pyrazol in 300 ml Dichlormethan wurden unter Eiskühlung portionsweise 31,1 g (0,18 mol) m-Chlorperbenzoesäure gegeben, wonach man 16 Std. bei ca. 20°C rührte. Anschließend wurde die Reaktionslösung mit gesättigter wässriger Natriumhydrogencarbonat-Lösung, wässriger Na₂S₂O₃-Lösung und Wasser gewaschen, noch über Magnesiumsulfat getrocknet und schließlich eingeengt. Ausbeute: 17,7 g. ¹H-NMR (270 MHz, in CDCl₃): δ [ppm] = 3,32 (s, 3H), 4,27 (s, 3H), 7,20-7,28 (m, 2H), 7,41 (t, 1H).

### Vorstufe 4.3

### 4-Brom-3-(4-chlor-2-fluor-5-nitrophenyl)-1-methyl-5-methylsulfonyl-1H-pyrazol

Diese Verbindung wurde analog Vorstufe 1.1 hergestellt. ¹H-NMR (270 MHz, in CDCl₃) : δ [ppm] = 3,33 (s, 3H), 4,29 (s, 3H), 7,44 (d, 1H), 8,20 (d, 1H).

### Vorstufe 4.4

### 5-(4-Brom-1-methyl-5-methylsulfonyl-1H-pyrazol-3-yl)-2-chlor-4-fluoranilin

Diese Verbindung wurde analog Vorstufe 1.2 hergestellt. ¹H-NMR {270 MHz, in (CD₃)₂SO} : δ [ppm] = 3,47 (s, 3H), 4,17 (s, 3H), 5,43 (s, 2H), 6,88 (d, 1H), 7,34 (d, 1H).

### Vorstufe 4.5

### 5-(4-Brom-1-methyl-5-methylsulfonyl-1H-pyrazol-3-yl)-2-chlor-4-fluorphenylazid (Nr. Va.9)

Diese Verbindung wurde analog Vorstufe 1.3 hergestellt. MS [m/z]: 407 [M⁺]. ¹H-NMR (400 MHz, in CDCl₃): 8 [ppm] = 3,32 (s, 3H), 4,28 (s, 3H), 7,28 (m, 2H).

### Beispiel 5

### 2-Amino-7-(4-brom-5-difluormethoxy-1-methyl-1H-pyrazol-3-yl)-4-chlor-6-fluorbenzthiazol (Verbindung Nr. Ic.080)

Zu einer Lösung von 4,7 g (13 mmol) 5-(4-Brom-5-difluormethoxy-1-methyl-1H-pyrazol-3-yl)-2-chlor-4-fluoranilin in 100 ml Essigsäure wurden 4,1 g (50 mmol) Natriumthiocyanat gegeben. Nach 10 Minuten Rühren tropfte man 4 g (25 mmol) Brom in die Mischung. Anschließend wurde 16 Std. gerührt. Dann goß man das Reaktionsgemisch auf Wasser. Schließlich wurde das entstandene feste Wertprodukt noch abfiltriert und getrocknet. Ausbeute: quantitativ. ¹H-NMR (270 MHz, in CDCl₃): δ [ppm] = 3,87 (s, 3H), 6,20 (s, 2H), 6,74 (t, 1H), 7,24 (d, 1H).

### Beispiel 6

### 7-(4-Brom-5-difluormethoxy-1-methyl-1H-pyrazol-3-yl)-4-chlor-6-fluor-2-(methylthio)benzthiazol (Verbindung Nr. Ic.099)

Eine Lösung von 1,5 g (3,5 mmol) 2-Amino-7-(4-brom-5-difluormethoxy-1-methyl-1H-pyrazol-3-yl)-4-chlor-6-fluorbenzthiazol in 50 ml Dichlormethan wurde tropfenweise mit 0,98 g (10,5 mmol) Dimethyldisulfid und 1,08 g (7 mmol) t-Butylnitrit versetzt. Nach 16 Std. wurde die Lösung eingeengt und der Rückstand durch Kieselgelchromatographie gereinigt. Ausbeute 0,4 g. ¹H-NMR (270 MHz, in CDCl₃): δ [ppm] = 2,80 (s, 3H), 3,90 (s, 3H), 6,74 (t, 1H), 7,34 (d, 1H).

### Beispiel 7

### 2-Brom-7-(4-brom-5-difluormethoxy-1-methyl-1H-pyrazol-3-yl)-4-chlor-6-fluorbenzthiazol (Verbindung Nr. Ic.041)

Eine Lösung von 1,5 g (3,5 mmol) 2-Amino-7-(4-brom-5-difluormethoxy-1-methyl-1H-pyrazol-3-yl)-4-chlor-6-fluorbenzthiazol in 50 ml Acetonitril wurde mit 1,8 g (17,5 mmol) Natriumbromid, 1 g (7 mmol) Kupfer(I)bromid und 0,47 g (4,6 mmol) t-Butylnitrit versetzt. Nach 16 Std. wurde mit 50 ml verd. Salzsäure versetzt, vom Niederschlag abfiltriert und mit Ethylacetat nachgewaschen. Die Phasen des Filtrats wurden getrennt, die wäßrige noch zweimal mit Ethylacetat extrahiert, die vereinigten organischen Phasen über MgSO₄ getrocknet, filtriert und eingeengt. Das Rohprodukt wurde durch Kieselgelchromatographie gereinigt. Ausbeute 0,4 g. MS [m/z] : 489 [M⁺].

### Beispiel 8

### 7-(4-Brom-5-difluormethoxy-1-methyl-1H-pyrazol-3-yl)-4-chlor-6-fluor-2-methoxybenzthiazol (Verbindung Nr. Ic.042)

60 mg (2,7 mmol) Natriumhydrid wurden in 20 ml Methanol gelöst und danach mit 0,4 g (1 mmol) 2-Brom-7-(4-brom-5-difluormethoxy-1-methyl-1H-pyrazol-3-yl)-4-chlor-6-fluorbenzthiazol versetzt. Nach 1 Std. wurde eingeengt und das Rohprodukt durch Kieselgelchromatographie (Eluens Cyclohexan/Ethylacetat 4:1) gereinigt. Ausbeute 0,4 g. ¹H-NMR (270 MHz, in CDCl₃): δ [ppm] = 3,88 (s, 3 H), 4,13 (s, 3 H), 6,74 (t, 1 H), 7,29 (d, 1 H).

### Anwendungsbeispiele (herbizide Wirksamkeit)

Die herbizide Wirkung der substituierten Pyrazol-3-ylbenzazole I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

### Anwendungsbeispiele (desikkant/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der Wirkstoffe (unter Zusatz von 0,15 Gew.-% des Fettalkoholalkoxylats Plurafac® LF 700¹⁾, bezogen auf die Spritzbrühe) blattbehandelt. Die ausgebrachte Wassermenge betrug umgerechnet 1000 1/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. Substituierte (4-Brompyrazol-3-yl)benzazole der Formel I 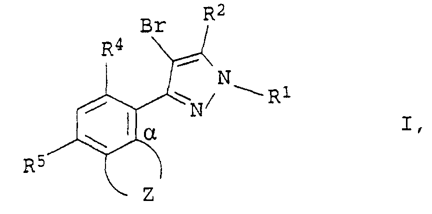 in der die Variablen folgende Bedeutungen haben:
R¹ Wasserstoff, C₁-C₄-Alkyl oder C₁-C₄-Halogenalkyl;
R² Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
R⁴ Wasserstoff oder Halogen;
R⁵ Wasserstoff, Halogen, Cyano, C₁-C₄-Alkyl, C₁-C₄-Halogenalkyl, C₁-C₄-Alkoxy oder C₁-C₄-Halogenalkoxy;
Z eine Gruppe -N=C(XR⁶)-O- oder -N=C(XR⁶)-S-, die über den Stickstoff, Sauerstoff oder Schwefel an α gebunden sein kann;
X eine chemische Bindung, Sauerstoff, Schwefel, -S(O)-, -SO₂-, -NH- oder -N(R⁷)-;
R⁶, R⁷ unabhängig voneinander
C₁-C₆-Alkyl, C₁-C₆-Halogenalkyl, Cyano-C₁-C₄-alkyl, Hydroxy-C₁-C₄-alkyl, C₃-C₆-Alkenyl, Cyano-C₃-C₆-alkenyl, C₃-C₆-Halogenalkenyl, C₃-C₆-Alkinyl, Cyano-C₃-C₆-alkinyl, C₃-C₆-Halogenalkinyl, C₁-C₄-Alkoxy-C₁-C₄-alkyl, C₁-C₄-Halogenalkoxy-C₁-C₄-alkyl, C₃-C₄-Alkenyloxy-C₁-C₄-alkyl, C₃-C₄-Alkinyloxy-C₁-C₄-alkyl, C₃-C₈-Cycloalkyloxy-C₁-C₄-alkyl, Amino-C₁-C₄-alkyl, C₁-C₄-Alkylamino-C₁-C₄-alkyl, Di(C₁-C₄-alkyl)amino-C₁-C₄-alkyl, C₁-C₄-Alkylthio-C₁-C₄-alkyl, C₁-C₄-Halogenalkylthio-C₁-C₄-alkyl, C₃-C₄-Alkenylthio-C₁-C₄-alkyl, C₃-C₄-Alkinylthio-C₁-C₄-alkyl, C₁-C₄-Alkylsulfinyl-C₁-C₄-alkyl, C₁-C₄-Halogenalkylsulfinyl-C₁-C₄-alkyl, C₃-C₄-Alkenylsulfinyl-C₁-C₄-alkyl, C₃-C₄-Alkinylsulfinyl-C₁-C₄-alkyl, C₁-C₄-Alkylsulfonyl-C₁-C₄-alkyl, C₁-C₄-Halogenalkylsulfonyl-C₁-C₄-alkyl, C₃-C₄-Alkenylsulfonyl-C₁-C₄-alkyl, C₃-C₄-Alkinylsulfonyl-C₁-C₄-alkyl, Hydroxycarbonyl-C₁-C₄-alkyl, (C₁-C₄-Alkoxy)-carbonyl-C₁-C₄-alkyl, das eine Cyano- oder (C₁-C₄-Alkoxy)carbonylgruppe tragen kann,
(C₁-C₄-Alkylthio)carbonyl-C₁-C₄-alkyl, Aminocarbonyl-C₁-C₄-alkyl, C₁-C₄-Alkylaminocarbonyl-C₁-C₄-alkyl, Di(C₁-C₄-alkyl)aminocarbonyl-C₁-C₄-alkyl, Di(C₁-C₄-alkyl)-phosphonyl-C₁-C₄-alkyl, (C₁-C₄-Alkoxy)imino-C₁-C₄-alkyl, (C₃-C₄-Alkenyloxy)imino-C₁-C₄-alkyl,
C₃-C₈-Cycloalkyl, C₃-C₈-Cycloalkyl-C₁-C₄-alkyl, Phenyl, Phenyl-C₁-C₄-alkyl, 3- bis 7-gliedriges Heterocyclyl oder Heterocyclyl-C₁-C₄-alkyl, wobei jeder Cycloalkyl- und jeder Heterocyclyl-Ring ein Carbonyl- oder Thiocarbonyl-Ringglied enthalten kann,
und wobei jeder Cycloalkyl-, Phenyl- und Heterocyclylring unsubstituiert sein oder ein bis vier Substituenten tragen kann, jeweils ausgewählt aus der Gruppe bestehend aus Cyano, Nitro, Amino, Hydroxy, Carboxy, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl, C₁-C₄-Halogenalkylsulfonyl, (C₁-C₄-Alkoxy)-carbonyl, (C₁-C₄-Alkyl)carbonyl, (C₁-C₄-Halogenalkyl)-carbonyl, (C₁-C₄-Alkyl)carbonyloxy, (C₁-C₄-Haloaenalkyl)-carbonyloxy und Di(C₁-C₄-alkyl)amino;
sofern X eine chemische Bindung, Sauerstoff, Schwefel, -NH- oder -N(R⁷)- ist, kann R⁶ auch (C₁-C₄-Alkyl)carbonyl, (C₁-C₄-Halogenalkyl)carbonyl, (C₁-C₄-Alkoxy)carbonyl, C₁-C₄-Alkylsulfonyl oder C₁-C₄-Halogenalkylsulfonyl bedeuten;
sofern X eine chemische Bindung ist, kann R⁶ außerdem Wasserstoff, Cyano, Mercapto, Amino, Halogen, -CH₂-CH(Halogen)-R⁸, -CH=CH-R⁸ oder -CH=C(Halogen)-R⁸ bedeuten, wobei R⁸ für Hydroxycarbonyl, (C₁-C₄-Alkoxy)carbonyl, (C₁-C₄-Alkylthio)carbonyl, Aminocarbonyl, C₁-C₄-Alkylaminocarbonyl, Di(C₁-C₄-alkyl) aminocarbonyl oder Di(C₁-C₄-alkyl)phosphonyl steht;
oder R⁶ und R⁷ stehen zusammen für eine 1,3-Propylen-, Tetramethylen-, Pentamethylen- oder Ethylenoxyethylen-Kette, die jeweils unsubstituiert sein oder ein bis vier C₁-C₄-Alkylgruppen oder ein oder zwei (C₁-C₄-Alkoxy)carbonylgruppen tragen kann,
sowie die landwirtschaftlich brauchbaren Salze dieser Verbindungen I.

2. Verwendung von substituierten (4-Brompyrazol-3-yl)benzazolen und deren landwirtschaftlich brauchbaren Salzen, gemäß Anspruch 1, als Herbizide oder zur Desikkation/Defoliation von Pflanzen.

3. Herbizides Mittel, enthaltend eine herbizid wirksame Menge mindestens eines substituierten (4-Brompyrazol-3-yl)benzazols der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

4. Mittel zur Desikkation und/oder Defoliation von Pflanzen, enthaltend eine desikkant und/oder defoliant wirksame Menge mindestens eines substituierten (4-Brompyrazol-3-yl)benzazols der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

5. Verfahren zur Herstellung von herbizid wirksamen Mitteln, **dadurch gekennzeichnet, daß** man eine herbizid wirksame Menge mindestens eines substituierten (4-Brompyrazol-3-yl)benzazols der Formel I oder eines landwirtschaftlich brauchbaren Salzes von I, gemäß Anspruch 1, mit mindestens einem inerten flüssigen und/oder festen Trägerstoff sowie gewünschtenfalls mindestens einem oberflächenaktiven Stoff mischt.

6. Verfahren zur Herstellung von desikkant und/oder defoliant wirksamen Mitteln, **dadurch gekennzeichnet, daß** man eine desikkant/defoliant wirksame Menge mindestens eines substituierten (4-Brompyrazol-3-yl)benzazols der Formel I oder eines landwirtschaftlich brauchbaren Salzes von I, gemäß Anspruch 1, mit mindestens einem inerten flüssigen und/oder festen Trägerstoff sowie gewünschtenfalls mindestens einem oberflächenaktiven Stoff mischt.

7. Verfahren zur Bekämpfung von unerwünschtem Pflanzenwuchs, **dadurch gekennzeichnet, daß** man eine herbizid wirksame Menge mindestens eines substituierten (4-Brompyrazol-3-yl)benzazols der Formel I oder eines landwirtschaftlich brauchbaren Salzes von I, gemäß Anspruch 1, auf Pflanzen, deren Lebensraum oder auf Saatgut einwirken läßt.

8. Verfahren zur Desikkation und/oder Defoliation von Pflanzen, **dadurch gekennzeichnet, daß** man eine desikkant und/oder defoliant wirksame Menge mindestens eines substituierten (4-Brompyrazol-3-yl)benzazols der Formel I oder eines landwirtschaftlich brauchbaren Salzes von I, gemäß Anspruch 1, auf Pflanzen einwirken läßt.

9. Verfahren nach Anspruch 8, **dadurch gekennzeichnet, daß** man Baumwolle behandelt.

10. Verfahren zur Herstellung von substituierten (4-Brompyrazol-3-yl)benzazolen der Formel I gemäß Anspruch 1, wobei Z für -N=C(R⁶)-O- steht, **dadurch gekennzeichnet, daß** man ein Aminophenylpyrazol der Formel IIIa oder IIIb 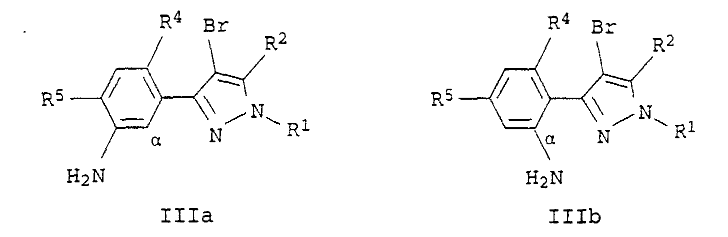 diazotiert, das gebildete Diazoniumsalz mit einem Alkalimetallazid zu einem Azidophenylpyrazol der Formel Va oder Vb 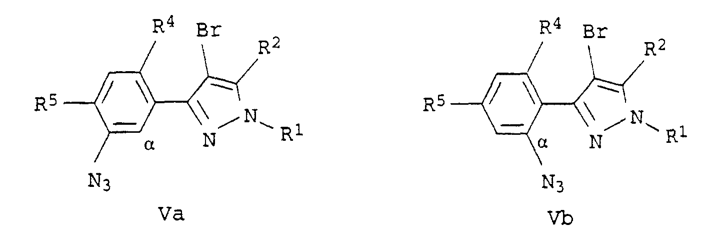 umsetzt und dieses schließlich mit einer Carbonsäure der Formel R⁶-COOH reagieren läßt.

11. Azidophenylpyrazole der Formel Va oder Vb 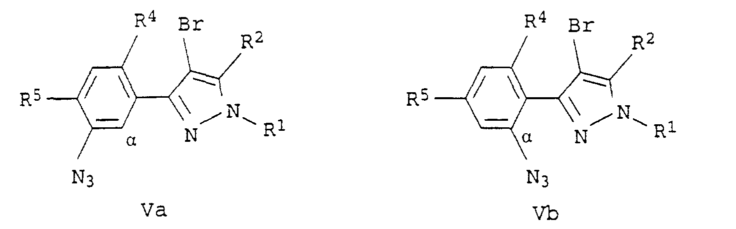 wobei die Substituenten R¹, R², R⁴ und R⁵ die in Anspruch 1 angegebenen Bedeutungen haben.

## Claims

1. A substituted (4-bromopyrazol-3-yl)benzazole of the formula I 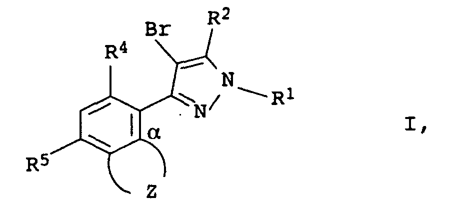 where:
R¹ is hydrogen, C₁-C₄-alkyl or C₁-C₄-haloalkyl;
R² is cyano, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylsulfinyl, C₁-C₄-haloalkylsulfinyl, C₁-C₄-alkylsulfonyl or C₁-C₄-haloalkylsulfonyl;
R⁴ is hydrogen or halogen;
R⁵ is hydrogen, halogen, cyano, C₁-C₄-alkyl, C₁-C₄-haloalkyl, C₁-C₄-alkoxy or C₁-C₄-haloalkoxy;
Z is a group -N=C(XR⁶)-O- or -N=C(XR⁶)-S- which may be attached to α via the nitrogen, oxygen or sulfur;
X is a chemical bond, oxygen, sulfur, -S(O)-, -SO₂-, -NH- or -N(R⁷)-;
R⁶, R⁷ independently of one another are each
C₁-C₆-alkyl, C₁-C₆-haloalkyl, cyano-C₁-C₄-alkyl, hydroxy-C₁-C₄-alkyl, C₃-C₆-alkenyl, cyano-C₃-C₆-alkenyl, C₃-C₆-haloalkenyl, C₃-C₆-alkynyl, cyano-C₃-C₆-alkynyl, C₃-C₆-haloalkynyl, C₁-C₄-alkoxy-C₁-C₄-alkyl, C₁-C₄-haloalkoxy-C₁-C₄-alkyl, C₃-C₄-alkenyloxy-C₁-C₄-alkyl, C₃-C₄-alkynyloxy-C₁-C₄-alkyl, C₃-C₈-cycloalkyloxy-C₁-C₄-alkyl, amino-C₁-C₄-alkyl, C₁-C₄-alkylamino-C₁-C₄-alkyl, di(C₁-C₄-alkyl)amino-C₁-C₄-alkyl, C₁-C₄-alkylthio-C₁-C₄-alkyl, C₁-C₄-haloalkylthio-C₁-C₄-alkyl, C₃-C₄-alkenylthio-C₁-C₄-alkyl, C₃-C₄-alkynylthio-C₁-C₄-alkyl, C₁-C₄-alkylsulfinyl-C₁-C₄-alkyl, C₁-C₄-haloalkylsulfinyl-C₁-C₄-alkyl, C₃-C₄-alkenylsulfinyl-C₁-C₄-alkyl, C₃-C₄-alkynylsulfinyl-C₁-C₄-alkyl, C₁-C₄-alkylsulfonyl-C₁-C₄-alkyl, C₁-C₄-haloalkylsulfonyl-C₁-C₄-alkyl, C₃-C₄-alkenylsulfonyl-C₁-C₄-alkyl, C₃-C₄-alkynylsulfonyl-C₁-C₄-alkyl, hydroxycarbonyl-C₁-C₄-alkyl, (C₁-C₄-alkoxy)carbonyl-C₁-C₄-alkyl, which may carry a cyano or (C₁-C₄-alkoxy)carbonyl group,
(C₁-C₄-alkylthio)carbonyl-C₁-C₄-alkyl, aminocarbonyl-C₁-C₄-alkyl, C₁-C₄-alkylaminocarbonyl-C₁-C₄-alkyl, di(C₁-C₄-alkyl)aminocarbonyl-C₁-C₄-alkyl, di(C₁-C₄-alkyl)-phosphonyl-C₁-C₄-alkyl, (C₁-C₄-alkoxy)imino-C₁-C₄-alkyl, (C₃-C₄-alkenyloxy)imino-C₁-C₄-alkyl,
C₃-C₈-cycloalkyl, C₃-C₈-cycloalkyl-C₁-C₄-alkyl, phenyl, phenyl-C₁-C₄-alkyl, 3- to 7-membered heterocyclyl or heterocyclyl-C₁-C₄-alkyl, where each cycloalkyl and each heterocyclyl ring may contain a carbonyl or thiocarbonyl ring member,
and where each cycloalkyl, phenyl and heterocyclyl ring may be unsubstituted or may carry from one to four substituents, in each case selected from the group consisting of cyano, nitro, amino, hydroxyl, carboxyl, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylsulfonyl, C₁-C₄-haloalkylsulfonyl, (C₁-C₄-alkoxy)carbonyl, (C₁-C₄-alkyl)carbonyl, (C₁-C₄-haloalkyl)carbonyl, (C₁-C₄-alkyl)carbonyloxy, (C₁-C₄-haloalkyl)carbonyloxy and di(C₁-C₄-alkyl)amino;
if X is a chemical bond, oxygen, sulfur, -NH- or -N(R⁷)-, R⁶ may also be (C₁-C₄-alkyl)carbonyl,
(C₁-C₄-haloalkyl)carbonyl, (C₁-C₄-alkoxy)carbonyl, C₁-C₄-alkylsulfonyl or C₁-C₄-haloalkylsulfonyl;
if X is a chemical bond, R⁶ may furthermore be hydrogen, cyano, mercapto, amino, halogen, -CH₂-CH(halogen)-R⁸, -CH=CH-R⁸ or -CH=C(halogen)-R⁸, where R⁸ is
hydroxycarbonyl, (C₁-C₄-alkoxy)carbonyl, (C₁-C₄-alkylthio)carbonyl, aminocarbonyl, C₁-C₄-alkylaminocarbonyl, di(C₁-C₄-alkyl)aminocarbonyl or di(C₁-C₄-alkyl)phosphonyl;
or R⁶ and R⁷ together are a 1,3-propylene, tetramethylene, pentamethylene or ethyleneoxyethylene chain which may in each case be unsubstituted or may carry from one to four C₁-C₄-alkyl groups or one or two (C₁-C₄-alkoxy)carbonyl groups,
and the agriculturally useful salts of these compounds I.

2. The use of substituted (4-bromopyrazol-3-yl)benzazoles and their agriculturally useful salts, as claimed in claim 1, as herbicides or for the desiccation/defoliation of plants.

3. A herbicidal composition, comprising a herbicidally effective amount of at least one substituted
(4-bromopyrazol-3-yl)benzazole of the formula I or an agriculturally useful salt of I, as claimed in claim 1, and at least one inert liquid and/or solid carrier and also, if desired, at least one surfactant.

4. A composition for the desiccation and/or defoliation of plants, comprising such an amount of at least one substituted (4-bromopyrazol-3-yl)benzazole of the formula I or an agriculturally useful salt of I, as claimed in claim 1, that it acts as a desiccant and/or defoliant, and at least one inert liquid and/or solid carrier and also, if desired, at least one surfactant.

5. A process for preparing herbicidally active compositions, which comprises mixing a herbicidally effective amount of at least one substituted (4-bromopyrazol-3-yl)benzazole of the formula I or an agriculturally utilizable salt of I, as claimed in claim 1, with at least one inert liquid and/or solid carrier and also, if desired, at least one surfactant.

6. A process for preparing compositions having desiccant and/or defoliant action, which comprises mixing such an amount of at least one substituted (4-bromopyrazol-3-yl)benzazole of the formula I or an agriculturally utilizable salt of I, as claimed in claim 1, that it acts as a desiccant/defoliant with at least one inert liquid and/or solid carrier and also, if desired, at least one surfactant.

7. A method for controlling undesirable vegetation, which comprises allowing a herbicidally effective amount of at least one substituted (4-bromopyrazol-3-yl)benzazole of the formula I or an agriculturally useful salt of I, as claimed in claim 1, to act on plants, their habitat or on seeds.

8. A method for the desiccation and/or defoliation of plants, which comprises allowing such an amount of at least one substituted (4-bromopyrazol-3-yl)benzazole of the formula I or an agriculturally utilizable salt of I, as claimed in claim 1, that it acts as a desiccant and/or defoliant to act on plants.

9. The method as claimed in claim 8, wherein cotton is treated.

10. A process for the preparation of substituted (4-bromopyrazol-3-yl)benzazoles of the formula I as claimed in claim 1 in which Z is -N=C(R⁶)-O-, which comprises diazotizing an aminophenylpyrazole of the formula IIIa or IIIb, 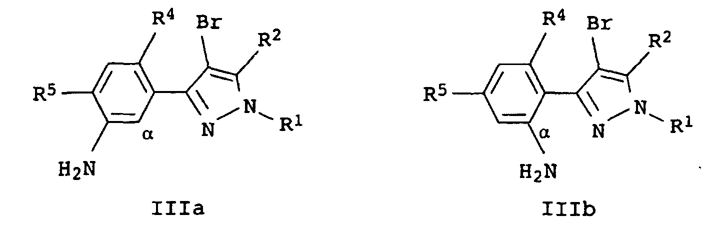 reacting the diazonium salt which is formed with an alkali metal azide to give an azidophenylpyrazole of the formula Va or Vb 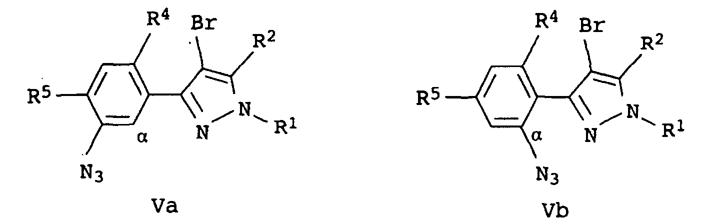 and finally reacting this with a carboxylic acid of the formula R⁶-COOH.

11. An azidophenylpyrazole of the formula Va or Vb 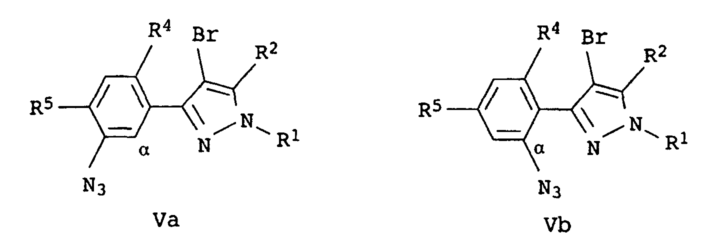 where the substituents R¹, R², R⁴ and R⁵ are each as defined in claim 1.

## Revendications

1. (4-bromopyrazol-3-yl)benzazoles substitués de formule I 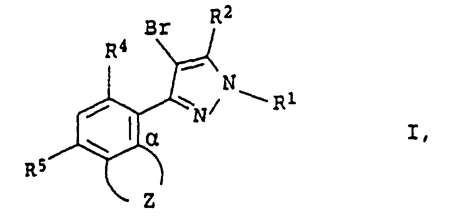 dans laquelle les symboles ont les significations suivantes :
R¹ : l'hydrogène, un groupe alkyle en C1-C4, halogénoalkyle en C1-C4 ;
R² : un groupe cyano, alkyle en C1-C4, halogénoalkyle en C1-C4, alcoxy en C1-C4, halogénoalcoxy en C1-C4, alkylthio en C1-C4, halogénoalkylthio en C1-C4, alkylsulfinyle en C1-C4, halogénoalkylsulfinyle en C1-C4, alkylsulfonyle en C1-C4 ou halogénoalkylsulfonyle en C1-C4 ;
R⁴ : l'hydrogène ou un halogène ;
R⁵ : l'hydrogène, un halogène, un groupe cyano, alkyle en C1-C4, halogénoalkyle en C1-C4, alcoxy en C1-C4 ou halogénoalcoxy en C1-C4 ;
Z : un groupe -N=C(XR⁶)-O- ou -N=C(XR⁶)-S-, qui peut être relié à α par l'azote, l'oxygène ou le soufre ;
X : une liaison chimique, l'oxygène, le soufre, -S(O)-, -SO₂-, -NH- ou -N(R⁷)- ;
R⁶, R⁷, indépendamment l'un de l'autre :
un groupe alkyle en C1-C6, halogénoalkyle en C1-C6, cyano-alkyle en C1-C4, hydroxyalkyle en C1-C4, alcényle en C3-C6, cyano-alcényle en C3-C6, halogénoalcényle en C3-C6, alcynyle en C3-C6, cyanoalcynyle en C3-C6, halogénoalcynyle en C3-C6, (alcoxy en C1-C4)alkyle en C1-C4, (halogénoalcoxy en C1-C4)alkyle en C1-C4, (alcényloxy en C3-C4)alkyle en C1-C4, (alcynyloxy en C3-C4)alkyle en C1-C4, (cycloalkyloxy en C3-C8)alkyle en C1-C4, aminoalkyle en C1-C4, (alkylamino en C1-C4)alkyle en C1-C4, di-(alkyle en C1-C4)aminoalkyle en C1-C4, (alkylthio en C1-C4)alkyle en C1-C4, (halogénoalkylthio en C1-C4)alkyle en C1-C4, (alcénylthio en C3-C4)alkyle en C1-C4, (alcynylthio en C3-C4)alkyle en C1-C4, (alkylsulfinyle en C1-C4)alkyle en C1-C4, (halogénoalkyle en C1-C4)sulfinyl-alkyle en C1-C4, (alcényle en C3-C4)sulfinyl-alkyle en C1-C4, (alcynyle en C3-C4)sulfinyl-alkyle en C1-C4, (alkyle en C1-C4)sulfonyl-alkyle en C1-C4, (halogénoalkyle en C1-C4)sulfonylalkyle en C1-C4, (alcényle en C3-C4)sulfonyl-alkyle en C1-C4, (alcynyle en C3-C4)sulfonyl-alkyle en C1-C4, hydroxycarbonyl-alkyle en C1-C4, (alcoxy en C1-C4)carbonyl-alkyle en C1-C4, qui peut-porter un groupe cyano ou un groupe (alcoxy en C1-C4)carbonyle,
(alkylthio en C1-C4)carbonyl-alkyle en C1-C4, aminocarbonyl-alkyle en C1-C4, (alkyle en C1-C4)aminocarbonyl-alkyle en C1-C4, di-(alkyle en C1-C4)aminocarbonyl-alkyle en C1-C4, di-(alkyle en C1-C4)phosphonyl-alkyle en C1-C4, (alcoxy en C1-C4)imino-alkyle en C1-C4, (alcényloxy en C3-C4)imino-alkyle en C1-C4, cycloalkyle en C3-C8, (cycloalkyle en C3-C8)alkyle en C1-C4, phényle, phényl-alkyle en C1-C4, hétérocyclyle ou hétérocyclyl-alkyle en C1-C4 de trois à sept chaînons dans lequel chaque noyau cycloalkyle et chaque noyau hétérocyclyle peut contenir un chaînon carbonyle ou thiocarbonyle,
chaque noyau cycloalkyle, phényle et hétérocyclyle pouvant être non substitué ou pouvant porter un à quatre substituants choisis parmi les groupes cyano, nitro, amino, hydroxy, carboxy, les halogènes, les groupes alkyle en C1-C4, halogénoalkyle en C1-C4, alcoxy en C1-C4, halogénoalcoxy en C1-C4, alkylthio en C1-C4, halogénoalkylthio en C1-C4, alkylsulfonyle en C1-C4, halogénoalkylsulfonyle- en C1-C4, (alcoxy en C1-C4)carbonyle, (alkyle en C1-C4)carbonyle, (halogénoalkyle en C1-C4)carbonyle, (alkyle en C1-C4)carbonyloxy, (halogénoalkyle en C1-C4)carbonyloxy et di-(alkyle en C1-C4)amino ;
sous réserve que lorsque X représente une liaison chimique, l'oxygène, le soufre, -NH- ou -N(R⁷)-, R⁶ peut également représenter un groupe (alkyle en C1-C4)carbonyle, (halogénoalkyle en C1-C4)carbonyle, (alcoxy en C1-C4)carbonyle, alkylsulfonyle en C1-C4 ou halogénoalkylsulfonyle en C1-C4 ;
sous réserve que lorsque X représente une liaison chimique, R⁶ peut en outre représenter l'hydrogène, un groupe cyano, mercapto, amino, un halogène, un groupe -CH₂-CH(halogène)-R⁸, -CH=CH-R8 ou -CH=C(halogène)-R⁸, R⁸ représentant un groupe hydroxycarbonyle, (alcoxy en C1-C4)carbonyle, (alkylthio en C1-C4)carbonyle, aminocarbonyle, (alkyle en Cl-C4)aminocarbonyle, di-(alkyle en C1-C4)aminocarbonyle ou di-(alkyle en C1-C4)phosphonyle ;
ou bien R⁶ et R⁷ représentent ensemble une chaîne 1,3-propylène, tétraméthylène, pentaméthylène ou éthylèneoxyéthylène qui peut être non substitué ou porter un à quatre groupes alkyle en C1-C4 ou un ou deux groupes (alcoxy en C1-C4)carbonyle,
et les sels de ces composés I aptes aux applications agricoles.

2. Utilisation des (4-bromopyrazol-3-yl)benzazoles substitués et de leurs sels aptes aux applications agricoles selon la revendication 1 en tant qu'herbicides ou pour la dessiccation/défoliation de végétaux.

3. Produit herbicide contenant une quantité herbicide efficace d'au moins un (4-bromopyrazol-3-yl)benzazole substitué de formule I ou de l'un de ses sels aptes aux applications agricoles selon la revendication 1 et au moins un véhicule inerte, liquide et/ou solide, et le cas échéant au moins une substance tensio-active.

4. Produit pour la dessiccation et/ou la défoliation de végétaux, contenant une quantité dessiccante et/ou défoliante efficace d'au moins un (4-bromopyrazol-3-yl)benzazole substitué de formule I ou de l'un de ses sels aptes aux applications agricoles selon la revendication 1 et au moins un véhicule inerte, liquide et/ou solide et le cas échéant au moins une substance tensio-active.

5. Procédé pour la préparation de produits à activité herbicide, **caractérisé par le fait que** l'on mélange une quantité herbicide efficace d'au moins un (4-bromopyrazol-3-yl)benzazole substitué de formule I ou de l'un de ses sels aptes aux applications agricoles selon la revendication 1 avec au moins un véhicule inerte, liquide et/ou solide et le cas échéant au moins une substance tensio-active.

6. Procédé pour la préparation de produits à activité dessiccante et/ou défoliante, **caractérisé par le fait que** l'on mélange une quantité dessiccante/défoliante efficace d'au moins un (4-bromopyrazol-3-yl)benzazole substitué de formule I ou de l'un de ses sels aptes aux applications agricoles selon la revendication 1 avec au moins un véhicule inerte, liquide et/ou solide et le cas échéant au moins une substance tensio-active.

7. Procédé pour combattre les croissances de végétaux indésirables, **caractérisé par le fait que** l'on fait agir une quantité herbicide efficace d'au moins un (4-bromopyrazol-3-yl)benzazole substitué de formule I ou de l'un de ses sels aptes aux applications agricoles selon la revendication 1 sur les végétaux, leur habitat ou les semences.

8. Procédé pour la dessiccation et ou la défoliation de végétaux, **caractérisé par le fait que** l'on fait agir une quantité dessiccante et/ou défoliante efficace d'au moins un (4-bromopyrazol-3-yl)benzazole substitué de formule I ou de l'un de ses sels aptes aux applications agricoles selon la revendication 1 sur les végétaux.

9. Procédé selon la revendication 8, **caractérisé par le fait que** l'on traite le coton.

10. Procédé pour la préparation des (4-bromopyrazol-3-yl)benzazoles substitués de formule I de la revendication 1 dans laquelle Z représente -N=C(R⁶)-O-, **caractérisé par le fait que** l'on diazote un aminophénylpyrazole de formule IIIa ou IIIb 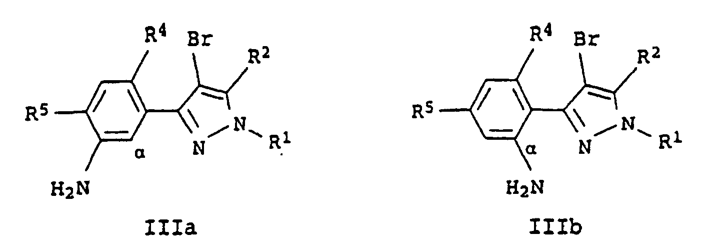 on fait réagir le sel de diazonium formé avec un azothydrate de métal alcalin, ce qui donne un azidophénylpyrazole de formule Va ou Vb 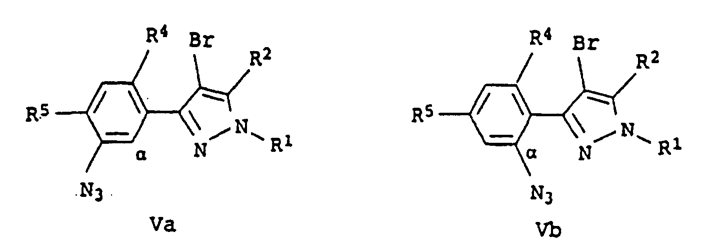 qu'on fait réagir finalement avec un acide carboxylique de formule R⁶-COOH.

11. Azidophénylpyrazoles de formule Va ou Vb 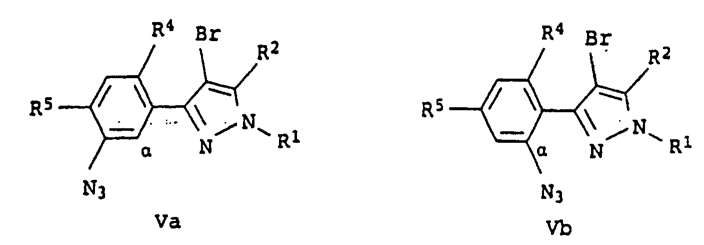 dans laquelle les symboles R¹, R², R⁴ et R⁵ ont les significations indiquées dans la revendication 1.
